# EUROPEAN PATENT SPECIFICATION

(11) **EP 3 936 514 B1**
(45) Date of publication and mention of the grant of the patent: **24.05.2023**
(21) Application number: 20185156.5
(22) Date of filing: 10.07.2020
(51) Int. Cl.: C07H 21/00, A61P 35/00, A61P 31/12

(54) **3'3' CYCLIC DINUCLEOTIDES WITH AN ALKENYLENE**
3'3'-CYCLISCHE DINUKLEOTIDE MIT EINEM ALKENYLEN
DINUCLÉOTIDES CYCLIQUES 3'3' AVEC UN ALCÉNYLÈNE

(43) Date of publication of application: 12.01.2022
(73) Proprietor: Institute of Organic Chemistry and Biochemistry ASCR, V.V.I., 166 10 Prague (CZ)
(72) Inventor: Birkus, Gabriel, Louth Lake Tahoe, CA 96150 (US); Dejmek, Milan, 16000 Praha 6 (CZ); Nencka, Radim, 25263 Roztoky (CZ); Otava, Tomas, 53901 Hlinsko (CZ); Sala, Michal, 27202 Kladno (CZ); Pav, Ondrej, 19700 Praha 9 (CZ)
(74) Representative: Hartvichova, Katerina

(56) References cited:
- EP-A1- 1 782 826
- WO-A1-2019/123339
- WO-A1-2019/123340
- XU ZHANG ET AL: "Cyclic GMP-AMP Containing Mixed Phosphodiester Linkages Is An Endogenous High-Affinity Ligand for STING", MOLECULAR CELL, vol. 51, no. 2, 6 June 2013 (2013-06-06), XP55375940, AMSTERDAM, NL ISSN: 1097-2765, DOI: 10.1016/j.molcel.2013.05.022

## Description

### FIELD OF INVENTION

The present disclosure relates to 3'3' cyclic dinucleotides and derivatives thereof that may be useful in the treatments of diseases in which modulation of STING adaptor protein (Stimulator of Interferon Genes) is beneficial, for example, inflammation, allergic and autoimmune diseases, cancer, and viral infections such as chronic hepatitis B and human immunodeficiency virus, and in the preparation of immunogenic compositions or vaccine adjuvants.

### BACKGROUND ART

The innate immune system recognizes the presence of pathogen or disruption of the homeostasis of the host by a battery of Pattern Recognition Receptors (PRRs) which detect a small set of ligands associated with pathogens or damage. These ligands are generally called Pathogen or Damage Associated Molecular Patterns (PAMPs and DAMPs) (Takeuchi O et al, Cell, 2010:140, 805). A number of PRRs have been identified over the past two decades including Toll-like receptors, retinoic acids inducible gene (RIG-I)-like receptors, nucleotide-binding oligomerization domain-like (NOD) receptors, C-type lectin receptors and cytosolic DNA sensors. Recognition of PAMPs and DAMPs by PRRs ultimately leads to the upregulation of cytokines and chemokines, including interferons, and recruitment of immune cells to the sites of infection. All these processes slow down pathogen replication and contribute to the development of adaptive immunity.

Cellular DNA is normally restricted to the nucleus and mitochondria of healthy cells. DNA present in cytosol, therefore, represents a signal indicating the presence of pathogen or disruption of the host homeostasis. The sensing of exogenous DNA is initiated by several DNA sensors such as DNA-dependent activator of IRFs (DAI) or DEAD box polypeptide 41 (DDX41). They signal via adaptor protein STING (Stimulator Of Interferon Genes, also called TMEM173, MITA, ERIS) (Unterholzner L, Immunology, 2013: 218, 1312) by recruiting protein kinase TBK1 that triggers activation of the transcription factors NFκ-B (nuclear factor kappa B) and IRF-3 (interferon regulatory factor 3). Activation of STING ultimately results in release of type I and III interferons and variety of cytokines and chemokines such as IL-6, TNF-α and INF-γ.

Alternatively, STING can be activated by the second messenger cyclic dinucleotides (CDNs)(Burdette et al. Nature 2011: 478,515). CDNs with affinity to STING contain two purine nucleotide monophosphates linked with either two 3'-5' (3'3'-CDNs), two 2'-5' (2'2'-CDNs) or 2'-5' and 3'-5' phosphodiester bonds (2'3'-CDNs). The prototype 2'3' cGAMP (c[G(2',5')pA(3',5')p]) is a product of the activation of host cGAS in the presence of pathogen or self dsDNA and it has the highest binding affinity to STING of all linkage isomers (Zhang et al, Molecular Cell 2013:51,226).

The type I interferons (IFNs) are immune-regulatory cytokines that play a pivotal role in viral immunity. They induce dendritic cell (DC) and macrophage maturation and activation (Galluci et al, Nat Med, 1999:5, 1249) and promote T- and B-cell survival, activation and differentiation. Furthermore, they activate numerous intracellular pathways that inhibit virus replication. The clinical utility of type I interferons has been demonstrated by their usefulness in treatment of chronic hepatitis B and C (Lin and Young, Cytokine Growth Factor Rev, 2014:25,369).

In addition, interferons have shown utility in treatment of human cancers (Cohen et al, N Engl J Med, 2005:353, 2477, Tsao et al, N Engl J Med, 2004:351, 998). They can directly inhibit proliferation of tumor cells and may be synergistic with many approved anticancer agents. Furthermore, type-I-IFNs can act on immune cells to induce antitumor response (Musella et al, Oncoimmunology 2017:6, e1314424). Type I IFN signaling was shown to be important in tumor-initiated T cell priming in mice and animals lacking the IFN-α/β receptor in dendritic cells were unable to reject immunogenic tumors, and were defective in antigen cross-presentation to CD8+ T cells (Fuertes et al, J Exp Med, 2011:208, 2005, Diamond et al, J Exp Med, 2011:208, 1989). Consistently with these observations, intratumoral injection of STING agonists has been recently shown to induce regression of established tumors in mice and generated substantial systemic immune responses capable of rejecting distant metastases and providing long-lived immunologic memory (Corrales et al, Cell Rep, 2015:11, 1018).

CDNs are believed to promote priming of both cellular and humoral immunity. For example, CDNs were shown to be an effective adjuvant in animal models (Dubensky et al, Ther Adv Vaccines, 2013:1, 131). Further also WO2019/12334 and WO2019/123339 disclose cyclic di-nucleotides for pharmaceutical use.

There continues to be a need for novel CDNs that activate STING.

### DESCRIPTION OF THE INVENTION

In an aspect, this disclosure describes novel 3'3' cyclic phosphonate di-nucleotides and derivatives thereof that bind to and activate protein STING and, consequently, stimulate the signal transduction pathway that induces interferons and other cytokines/chemokines. One advantage compared to previously disclosed CDNs arises from the replacement of a phosphoester bond with a phosphonate bond that is resistant toward hydrolysis by phosphodiesterases present in tissues and bodily fluids. Such compounds may find utility as an anti-viral and anti-cancer agent, act as adjuvants in vaccines or may be used in the treatment of allergic or other inflammatory diseases.

In one embodiment, the present disclosure provides a compound of formula (J): 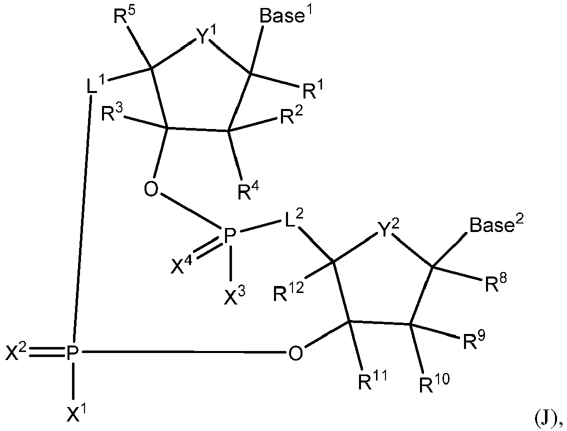
or an enantiomer, hydrate, solvate, or pharmaceutically acceptable salt thereof, wherein
L¹ is -C(R⁶R⁷)-O- and L² is -C(R¹³)=C(R¹⁴)-,
L¹ is -O-C(R⁶R⁷)- and L² is -C(R¹³)=C(R¹⁴)-,
L¹ is -C(R¹³)=C(R¹⁴)- and L² is -C(R¹³)=C(R¹⁴)-,
L¹ is -C(R¹³)=C(R¹⁴)- and L² is -C(R⁶R⁷)-O-, or
L¹ is -C(R¹³)=C(R¹⁴)- and L² is -O-C(R⁶R⁷)-;
Y¹ and Y² are each independently -O-, -S-, or -CH₂-;
X¹ and X³ are each independently OH, SH, OR¹⁵, SR¹⁵, or N(R¹⁵)₂;
X² and X⁴ are each independently O or S;
R¹, R⁵, R⁸ and R¹² are each independently H, CN, N₃, F, Cl, Br, I, COOR¹⁵, CON(R¹⁵)₂, C₁-C₆ alkyl, C₂-C₆ alkenyl, C₂-C₆ alkynyl, OR¹⁵, SR¹⁵, or N(R¹⁵)₂;
R², R³, R⁴, R⁹, R¹⁰ and R¹¹ are each independently H, F, Cl, Br, I, CN, N₃, C₁-C₆ alkyl, C₂-C₆ alkenyl, C₂-C₆ alkynyl, OR¹⁵, SR¹⁵, or N(R¹⁵)₂;
R⁶, R⁷, R¹³ and R¹⁴ are each independently H, CN, N₃, F, Cl, Br, I, COOR¹⁵, CON(R¹⁵)₂, OR¹⁵, SR¹⁵, N(R¹⁵)₂, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each R¹⁵ is independently H, -C(=Z)R¹⁶, -C(=Z)OR¹⁶, -C(=Z)SR¹⁶, -C(=Z)N(R¹⁶)₂, -CH₂-Z-C(=Z)R¹⁶, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each R¹⁶ is independently H, C₁-C₁₂ alkyl, C₂-C₁₂ alkenyl, C₂-C₁₂ alkynyl, C₃-C₇ cycloalkyl, C₂-C₁₀ heterocycloalkyl, C₆-C₁₀ aryl, or C₂-C₁₀ heteroaryl; preferably each R¹⁶ is independently H, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each Z is independently O or S;
Base¹ and Base² are each independently: 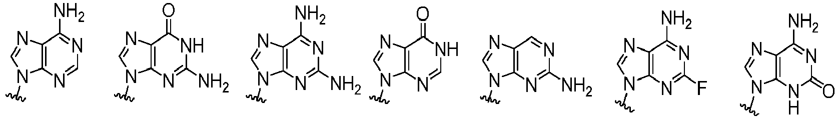 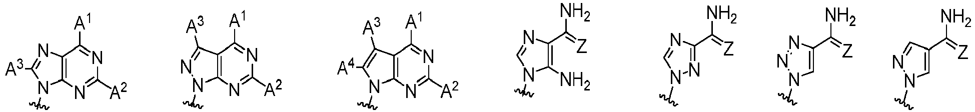 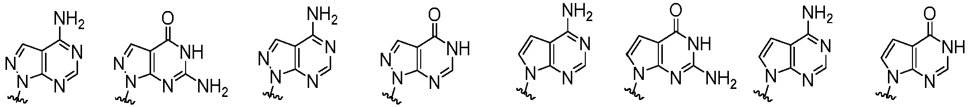 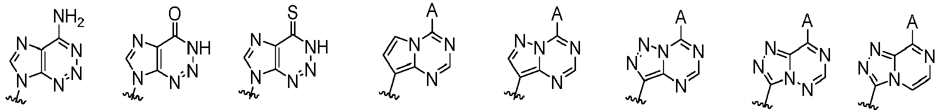 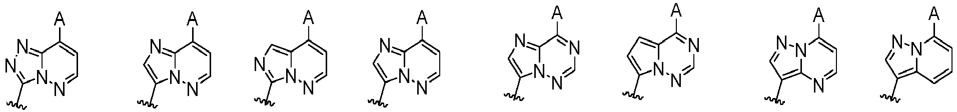 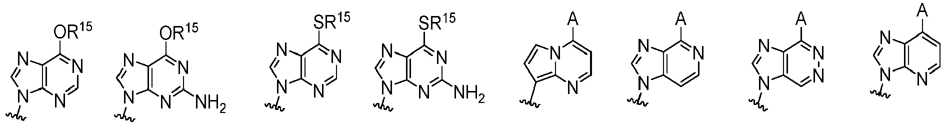 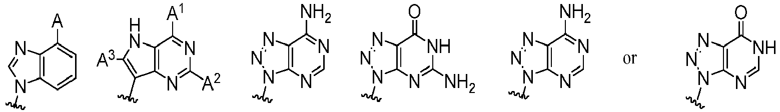 wherein
   A, A¹, A², A³ and A⁴ are each independently H, OH, SH, F, Cl, Br, I, NH₂, OR¹⁵, SR¹⁵, NHR¹⁵, N(R¹⁵)₂, or R¹⁶; and
   wherein the C₁-C₆ alkyl, C₂-C₆ alkenyl, C₂-C₆ alkynyl, C₂-C₁₀ heterocycloalkyl, C₆-C₁₀ aryl, or C₂-C₁₀ heteroaryl independently in each instance is optionally substituted with 1, 2, or 3 -OH; -SH; -NH₂; =O; =NH; =S; halogen; -N₃; C₆-C₁₀ aryl optionally substituted with 1, 2, or 3-OH, -CN, -O(C=O)OR^{B}, -O(C=O)R^{B}, or -COOR^{B}; unsubstituted C₁-C₆ alkyl; unsubstituted C₁-C₆ alkoxy; unsubstituted C₁-C₆ alkylthio; unsubstituted C₁-C₆ alkylamino; unsubstituted C₁-C₆ dialkylamino; -CN; -O(C=O)OR^{B}; -O(C=O)R^{B}; or -COOR^{B}; wherein R^{B} is H or unsubstituted C₁-C₆ alkyl.

In some embodiments, at least one substituent is selected from the following list, preferably the substituents are selected from the following list:
Y¹ and Y² are each independently -O- or -S-;
X¹ and X³ are each independently OH, SH, OR¹⁵ or SR¹⁵;
X² and X⁴ are each independently O or S;
R¹, R⁵, R⁸ and R¹² are H;
R², R³, R⁹, and R¹¹ are H;
R⁴ and R¹⁰ are each independently H, F, Cl, CN, N₃, OH, SH, NH₂, OR¹⁵, SR¹⁵, or N(R¹⁵)₂; preferably R⁴ and R¹⁰ are independently selected from H, F, Cl, OH, SH, NH₂, CN and N₃;
R⁶, R⁷, R¹³ and R¹⁴ are each independently H, C₁-C₆ alkyl, C₂-C₆ alkenyl, or C₆-C₁₀ aryl; preferably R⁶, R⁷, R¹³ and R¹⁴ are independently H or C₁-C₄ alkyl;
R¹⁵ is independently H, -C(=Z)R¹⁶, -CH₂-Z-C(=Z)R¹⁶, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each R¹⁶ is independently H, C₁-C₁₂ alkyl, C₂-C₁₂ alkenyl, C₂-C₁₂ alkynyl, C₃-C₇ cycloalkyl, C₂-C₁₀ heterocycloalkyl, C₆-C₁₀ aryl, or C₂-C₁₀ heteroaryl; preferably each R¹⁶ is independently H, C₁-C₆ alkyl, C₂-C₆ alkenyl, C₃-C₇ cycloalkyl, C₆-C₁₀ aryl, or C₂-C₁₀ heteroaryl;
each Z is independently O or S.

In some embodiments, the present disclosure provides a compound of formula (J) for use as a medicament.

Preferably, the present disclosure provides a compound of formula (J) for use in treatment of inflammation, allergic and autoimmune diseases, cancer, and viral infections such as chronic hepatitis B and human immunodeficiency virus.

In some embodiments, the present disclosure provides a compound of formula (J) for use as components of immunogenic compositions or for use as vaccine adjuvants.

In some embodiments, a pharmaceutical composition comprises the compound of Formula (J), or an enantiomer, hydrate, solvate, or pharmaceutically acceptable salt thereof, and a pharmaceutically acceptable carrier, excipient, and/or diluent. The references to methods of treatment in the subsequent paragraphs of this description are to be interpreted as references to the compounds, pharmaceutical compositions and medicaments of the present invention for use in a method for treatment of the human body by therapy (or for diagnosis where appropriate).

In some embodiments, a method of treating or preventing a disease or disorder, e.g., a method of treating or preventing a viral infection, hepatitis B virus infection, HIV infection, hyperproliferative disease or cancer, comprises administering to a human or animal in need thereof a therapeutically effective amount of a compound of Formula (J), or an enantiomer, hydrate, solvate, or pharmaceutically acceptable salt thereof, or a pharmaceutical composition of any of the foregoing.

In some embodiments, a method of enhancing the efficacy of a vaccine comprises administering a therapeutically effective amount of a compound of Formula (J), or an enantiomer, hydrate, solvate, or pharmaceutically acceptable salt thereof, or a pharmaceutical composition of any of the foregoing.

In some embodiments, a method of modulating the activity of STING adaptor protein to induce production of a type I interferon, cytokine and/or chemokine dependent on the STING adaptor protein, e.g., inducing a STING adaptor protein-dependent type I interferon, cytokine or chemokine in a human or animal, comprises administering a therapeutically effective amount of a compound of Formula (J), or an enantiomer, hydrate, solvate, or pharmaceutically acceptable salt thereof, or a pharmaceutical composition of any of the foregoing.

The disclosure provides novel 3'3' cyclic dinucleotides, comprising at least one phosphonate group, that bind to and modulate the activity of, e.g., activate, the STING protein. The dinucleotides have at least one 4' linkage that is an alkenylene variant of the naturally occurring methylene phosphate attachment to the sugar.

Unless defined otherwise, all technical and scientific terms used herein have the same meaning as commonly understood by one of ordinary skill in the art. A dash at the front or end of a chemical group is a matter of convenience to indicate the point of attachment to a parent moiety; chemical groups may be depicted with or without one or more dashes without losing their ordinary meaning. A prefix such as "Cᵤ₋ᵥ" or "Cᵤ-Cᵥ" indicates that the following group has from u to v carbon atoms, where u and v are integers. For example, "C₁₋₆ alkyl" or "C₁-C₆ alkyl" indicates that the alkyl group has from 1 to 6 carbon atoms.

"Alkyl" is a linear or branched saturated monovalent hydrocarbon. For example, an alkyl group can have 1 to 12 carbon atoms (i.e., C₁₋₁₂ alkyl) or 1 to 8 carbon atoms (i.e., C₁₋₈ alkyl) or 1 to 6 carbon atoms (i.e., C₁₋₆ alkyl) or 1 to 4 carbon atoms (i.e., C₁₋₄ alkyl). Examples of alkyl groups include, but are not limited to, methyl (Me, -CH₃), ethyl (Et, -CH₂CH₃), 1-propyl (*n*-Pr, *n*-propyl, -CH₂CH₂CH₃), 2-propyl (*i*-Pr, *i*-propyl, -CH(CH₃)₂), 1-butyl (*n*-Bu, *n*-butyl, -CH₂CH₂CH₂CH₃), 2-methyl-1-propyl (i-Bu, *i*-butyl, -CH₂CH(CH₃)₂), 2-butyl (*s*-Bu, *s*-butyl, -CH(CH₃)CH₂CH₃), 2-methyl-2-propyl (*t*-Bu, *t*-butyl, -C(CH₃)₃), 1-pentyl (*n*-pentyl, -CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), 1-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), and octyl (-(CH₂)₇CH₃).

"Alkoxy" refers to the group -O-alkyl, where alkyl is as defined above. For example, C₁₋₄ alkoxy refers to an -O-alkyl group having 1 to 4 carbons.

"Alkenyl" is a linear or branched monovalent hydrocarbon radical with at least one carbon-carbon double bond. For example, an alkenyl group can have 2 to 8 carbon atoms (i.e., C₂₋₈ alkenyl) or 2 to 6 carbon atoms (i.e., C₂₋₆ alkenyl) or 2 to 4 carbon atoms (i.e., C₂₋₄ alkenyl). Examples of alkenyl groups include, but are not limited to, ethenyl (-CH=CH₂), allyl (-CH₂CH=CH₂), and -CH₂-CH=CH-CH₃. Alkenyl groups can be unsubstituted or substituted.

"Alkynyl" is a linear or branched monovalent hydrocarbon radical with at least one carbon-carbon triple bond. For example, an alkynyl group can have 2 to 8 carbon atoms (i.e., C₂₋₈ alkynyl) or 2 to 6 carbon atoms (i.e., C₂₋₆ alkynyl) or 2 to 4 carbon atoms (i.e., C₂₋₄ alkynyl). Examples of alkynyl groups include, but are not limited to, acetylenyl (-C≡CH), propargyl (-CH₂C≡CH), and -CH₂-C≡C-CH₃. Alkynyl groups can be unsubstituted or substituted.

Alkylamino is -HNR_{b} group, where R_{b} is an alkyl.

Alkylthio is -SR_{b} group, where R_{b} is an alkyl.

"Halo" or "halogen" as used herein refers to fluoro (-F), chloro (-Cl), bromo (-Br) and iodo (-I).

"Haloalkyl" as used herein refers to an alkyl as defined herein, wherein one or more hydrogen atoms of the alkyl are independently replaced by a halo substituent, which may be the same or different. For example, C₁₋₄ haloalkyl is a C₁₋₄ alkyl wherein one or more of the hydrogen atoms of the C₁₋₄ alkyl have been replaced by a halo substituent. Examples of haloalkyl groups include but are not limited to fluoromethyl, fluorochloromethyl, difluoromethyl, difluorochloromethyl, trifluoromethyl, 1,1,1-trifluoroethyl and pentafluoroethyl.

"Aryl" as used herein refers to a single all carbon aromatic ring or a multiple condensed all carbon ring system wherein at least one of the rings is aromatic. For example, in certain embodiments, an aryl group has 6 to 20 carbon atoms, 6 to 14 carbon atoms, or 6 to 12 carbon atoms. Aryl includes a phenyl radical. Aryl also includes multiple condensed ring systems (e.g., ring systems comprising 2, 3 or 4 rings) having about 9 to 20 carbon atoms in which at least one ring is aromatic and wherein the other rings may be aromatic or not aromatic (i.e., carbocycle). Such multiple condensed ring systems are optionally substituted with one or more (e.g., 1, 2 or 3) oxo groups on any carbocycle portion of the multiple condensed ring system. The rings of the multiple condensed ring system can be connected to each other via fused, spiro and bridged bonds when allowed by valency requirements. Non-limiting examples of aryl groups include, but are not limited to, phenyl, indenyl, naphthyl, 1,2,3,4-tetrahydronaphthyl, anthracenyl, and the like. Aryl groups can be unsubstituted or substituted.

"Heteroaryl" as used herein refers to a single aromatic ring that has at least one atom other than carbon in the ring, wherein the atom is selected from the group consisting of oxygen, nitrogen and sulfur; "heteroaryl" also includes multiple condensed ring systems that have at least one such aromatic ring, which multiple condensed ring systems are further described below. Thus, "heteroaryl" includes single aromatic rings of from about 1 to 6 carbon atoms and about 1-4 heteroatoms selected from the group consisting of oxygen, nitrogen and sulfur. The sulfur and nitrogen atoms may also be present in an oxidized form provided the ring is aromatic. Exemplary heteroaryl ring systems include but are not limited to pyridyl, pyrimidinyl, oxazolyl or furyl. "Heteroaryl" also includes multiple condensed ring systems (e.g., ring systems comprising 2, 3 or 4 rings) wherein a heteroaryl group, as defined above, is condensed with one or more rings selected from heteroaryls (to form for example 1,8-naphthyridinyl), heterocycles, (to form for example 1,2,3,4-tetrahydro-1,8-naphthyridinyl), carbocycles (to form for example 5,6,7,8-tetrahydroquinolyl) and aryls (to form for example indazolyl) to form the multiple condensed ring system. Thus, a heteroaryl (a single aromatic ring or multiple condensed ring system) has about 1-20 carbon atoms and about 1-6 heteroatoms within the heteroaryl ring. Such multiple condensed ring systems may be optionally substituted with one or more (e.g., 1, 2, 3 or 4) oxo groups on the carbocycle or heterocycle portions of the condensed ring. The rings of the multiple condensed ring system can be connected to each other via fused, spiro and bridged bonds when allowed by valency requirements. It is to be understood that the individual rings of the multiple condensed ring system may be connected in any order relative to one another. It is to be understood that the point of attachment for a heteroaryl or heteroaryl multiple condensed ring system can be at any suitable atom of the heteroaryl or heteroaryl multiple condensed ring system including a carbon atom and a heteroatom (e.g., a nitrogen). It also to be understood that when a reference is made to a certain atom-range membered heteroaryl (e.g., a 5 to 10 membered heteroaryl), the atom range is for the total ring atoms of the heteroaryl and includes carbon atoms and heteroatoms. For example, a 5-membered heteroaryl would include a thiazolyl and a 10-membered heteroaryl would include a quinolinyl. Exemplary heteroaryls include but are not limited to pyridyl, pyrrolyl, pyrazinyl, pyrimidinyl, pyridazinyl, pyrazolyl, thienyl, indolyl, imidazolyl, oxazolyl, isoxazolyl, thiazolyl, furyl, oxadiazolyl, thiadiazolyl, quinolyl, isoquinolyl, benzothiazolyl, benzoxazolyl, indazolyl, quinoxalyl, quinazolyl, 5,6,7,8-tetrahydroisoquinolinyl benzofuranyl, benzimidazolyl, thianaphthenyl, pyrrolo[2,3-b]pyridinyl, quinazolinyl-4(3H)-one, and triazolyl. Heteroaryl groups can be unsubstituted or substituted.

"Cycloalkyl" refers to a single saturated all carbon ring having 3 to 20 annular carbon atoms (i.e., C₃₋₂₀ cycloalkyl), for example from 3 to 12 annular atoms, for example from 3 to 10 annular atoms, or 3 to 8 annular atoms, or 3 to 6 annular atoms, or 3 to 5 annular atoms, or 3 to 4 annular atoms. The term "cycloalkyl" also includes multiple condensed, saturated all carbon ring systems (e.g., ring systems comprising 2, 3 or 4 carbocyclic rings). Accordingly, cycloalkyl includes multicyclic carbocyles such as a bicyclic carbocycles (e.g., bicyclic carbocycles having about 6 to 12 annular carbon atoms such as bicyclo[3.1.0]hexane and bicyclo[2.1.1]hexane), and polycyclic carbocycles (e.g tricyclic and tetracyclic carbocycles with up to about 20 annular carbon atoms). The rings of a multiple condensed ring system can be connected to each other via fused, spiro and bridged bonds when allowed by valency requirements. Non-limiting examples of monocyclic cycloalkyl include cyclopropyl, cyclobutyl, cyclopentyl, , , cyclohexyl,. Cycloalkyl groups are unsubstituted.

"Heterocyclyl" or "heterocycle" or "heterocycloalkyl" as used herein refers to a single saturated or partially unsaturated non-aromatic ring or a non-aromatic multiple ring system that has at least one heteroatom in the ring (i.e., at least one annular heteroatom selected from oxygen, nitrogen, and sulfur). Unless otherwise specified, a heterocyclyl group has from 3 to about 20 annular atoms, for example from 3 to 12 annular atoms, for example from 3 to 10 annular atoms, or 3 to 8 annular atoms, or 3 to 6 annular atoms, or 3 to 5 annular atoms, or 4 to 6 annular atoms, or 4 to 5 annular atoms. Thus, the term includes single saturated or partially unsaturated rings (e.g., 3, 4, 5, 6 or 7-membered rings) having from about 1 to 6 annular carbon atoms and from about 1 to 3 annular heteroatoms selected from the group consisting of oxygen, nitrogen and sulfur in the ring. The rings of the multiple condensed ring (e.g. bicyclic heterocyclyl) system can be connected to each other via fused, spiro and bridged bonds when allowed by valency requirements. Heterocycles include, but are not limited to, azetidine, aziridine, imidazolidine, morpholine, oxirane (epoxide), oxetane, thietane, piperazine, piperidine, pyrazolidine, piperidine, pyrrolidine, pyrrolidinone, tetrahydrofuran, tetrahydrothiophene, dihydropyridine, tetrahydropyridine, quinuclidine,, 2-oxa-6-azaspiro[3.3]heptan-6-yl, 6-oxa-1-azaspiro[3.3]heptan-1-yl, 2-thia-6-azaspiro[3.3]heptan-6-yl, 2,6-diazaspiro[3.3]heptan-2-yl, 2-azabicyclo[3.1.0]hexan-2-yl, 3-azabicyclo[3.1.0]hexanyl, 2-azabicyclo[2.1.1]hexanyl, 2-azabicyclo[2.2.1]heptan-2-yl, 4-azaspiro[2.4]heptanyl, 5-azaspiro[2.4]heptanyl, and the like. Heterocyclyl groups can be unsubstituted or substituted.

"Oxo" as used herein refers to =O.

"Substituted" as used herein refers to substitution with one or more substituents (e.g., 1, 2, 3, or 4 or more) selected from the group consisting of -OH, -SH, -NH₂, =O, =NH, =S, =N, halogen, -N₃, C₁₋₆ alkyl, C₁₋₆ alkoxy, C₁₋₆ alkylthio, C₁₋₆ alkylamino, C₁₋₆ dialkylamino, -CN, -O(C=O)OR^{B}, -O(C=O)R^{B} and -COOR^{B}, where R^{B} is hydrogen or C₁ to C₆ alkyl.

A "compound of the present disclosure" includes compounds disclosed herein, for example a compound of the present disclosure includes compounds of Formula (J), (I), (Ia), (IIa), and/or (IIIb), including the compounds of the Examples.

"Treatment" or "treat" or "treating" as used herein includes one or more of the following: decreasing one or more symptoms resulting from the disease or condition, and/or diminishing the extent of the disease or condition; ameliorating the disease state, delaying the progression of the disease, increasing the quality of life, and/or prolonging survival.

"Delaying" as used herein means to defer, hinder, slow, retard, stabilize and/or postpone development of the disease or condition. As is evident to one skilled in the art, a sufficient or significant delay can, in effect, encompass prevention, in that the individual does not develop the disease or condition.

"Prevent" or "prevention" or "preventing" as used herein refers to a regimen that protects against the onset of the disease or disorder such that the clinical symptoms of the disease do not develop. In some instances, prevention may be understood as a reduction of the risk of cancer, but not a complete elimination of the occurrence of a cancer.

In certain embodiments, the term "preventing HBV infection" refers to administering to a subject who does not have a detectable HBV infection an anti-HBV therapeutic substance.

"Modulation" or "modulating" the activity of a protein, e.g., a STING adaptor protein, as used herein refers to alteration of the activity such that the activity increases or decreases. In some embodiments, the modulation increases the activity.

As used herein, the term "viral infection" describes a diseased state in which a virus invades healthy cells, uses the cell's reproductive machinery to multiply or replicate and ultimately lyse the cell resulting in cell death, release of viral particles and the infection of other cells by the newly produced progeny viruses. Latent infection by certain viruses is also a possible result of viral infection.

As used herein, the term "enhancing" refers to any form of increase in the immunogenic activity of an effective dosage of a vaccine as a result of administering to an animal or a human a therapeutically effective dose of a compound of the present disclosure, at any time prior to, simultaneous with, or just after administration to the same animal or human of the effective dosage of a vaccine.

"At risk individual" as used herein refers to an individual who is at risk of developing a condition to be treated. An individual "at risk" may or may not have detectable disease or condition, and may or may not have displayed detectable disease prior to the treatment of methods described herein. "At risk" denotes that an individual has one or more so-called risk factors, which are measurable parameters that correlate with development of a disease or condition and are known in the art

"Therapeutically effective amount" or "effective amount" as used herein refers to an amount that is effective to elicit the desired biological or medical response, including the amount of a compound that, when administered to a subject for treating a disease, is sufficient to effect such treatment for the disease. The effective amount will vary depending on the compound, the disease, and its severity and the age, weight, etc., of the subject to be treated. The effective amount can include a range of amounts. As is understood in the art, an effective amount may be in one or more doses, *i.e.,* a single dose or multiple doses may be required to achieve the desired treatment endpoint. An effective amount may be considered in the context of administering one or more therapeutic agents.

In some embodiments, a therapeutically effective amount of a compound provided herein or pharmaceutically acceptable salt thereof, may (i) reduce the number of diseased cells; (ii) reduce tumor size; (iii) inhibit, retard, slow to some extent, and preferably stop the diseased cell infiltration into peripheral organs; (iv) inhibit tumor metastasis; (v) inhibit tumor growth; (vi) prevent or delay occurrence and/or recurrence of a tumor; and/or (vii) relieve to some extent one or more of the symptoms associated with cancer or hyperproliferative disease. In some embodiments, a therapeutically effective amount is sufficient to ameliorate, palliate, lessen, and/or delay one or more of symptoms of cancer or hyperproliferative disease.

"Pharmaceutically acceptable excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved as being acceptable for use in humans or domestic animals.

Provided are also pharmaceutically acceptable salts, hydrates, solvates, tautomeric form; and polymorphs of the compounds described herein. "Pharmaceutically acceptable" or "physiologically acceptable" refer to compounds, salts, compositions, dosage forms and other materials which are useful in preparing a pharmaceutical composition that is suitable for veterinary or human pharmaceutical use. The compounds described herein may be prepared and/or formulated as pharmaceutically acceptable salts or when appropriate as a free base. Pharmaceutically acceptable salts are non-toxic salts of a free base form of a compound that possesses the desired pharmacological activity of the free base. These salts may be derived from inorganic or organic acids or bases. For example, a compound that contains a basic nitrogen may be prepared as a pharmaceutically acceptable salt by contacting the compound with an inorganic or organic acid. Non-limiting examples of pharmaceutically acceptable salts include sulfates, pyrosulfates, bisulfates, sulfites, bisulfites, phosphates, monohydrogen-phosphates, dihydrogenphosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, methylsulfonates, propylsulfonates, besylates, xylenesulfonates, naphthalene-1-sulfonates, naphthalene-2-sulfonates, phenylacetates, phenylpropionates, phenylbutyrates, citrates, lactates, γ-hydroxybutyrates, glycolates, tartrates, and mandelates. Lists of other suitable pharmaceutically acceptable salts are found in Remington: The Science and Practice of Pharmacy, 21st Edition, Lippincott Wiliams and Wilkins, Philadelphia, Pa., 2006.

Examples of "pharmaceutically acceptable salts" of the compounds disclosed herein also include salts derived from an appropriate base, such as an alkali metal, an alkaline earth metal, ammonium and NX₄⁺ (wherein X is C₁-C₄ alkyl). Also included are base addition salts, such as sodium or potassium salts.

Provided are also compounds described herein or pharmaceutically acceptable salts, isomers, or a mixture thereof, in which from 1 to n hydrogen atoms attached to a carbon atom may be replaced by a deuterium atom or D, in which n is the number of hydrogen atoms in the molecule.

Examples of isotopes that can be incorporated into the disclosed compounds also include isotopes, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of Formula (J) can generally be prepared by conventional techniques known to those skilled in the art or by processes analogous to those described in the Examples as set out below using an appropriate isotopically-labeled reagent in place of the non-labeled reagent previously employed.

The compounds of the embodiments disclosed herein, or their pharmaceutically acceptable salts may contain one or more asymmetric centers and may thus give rise to enantiomers, diastereomers, and other stereoisomeric forms that may be defined, in terms of absolute stereochemistry, as (R)- or (*S*)- or, as (D)- or (L)- for amino acids. The present disclosure is meant to include all such possible isomers, as well as their racemic and optically pure forms. When the compounds described herein contain olefinic double bonds or other centres of geometric asymmetry, and unless specified otherwise, it is intended that the compounds include both E and Z geometric isomers. Likewise, all tautomeric forms are also intended to be included. Where chirality is not specified but is present, it is understood that the embodiment is directed to either the specific diastereomerically or enantiomerically enriched form; or a racemic or scalemic mixture of such compound(s). As used herein, "scalemic mixture" is a mixture of stereoisomers at a ratio other than 1:1.

"Stereoisomer" as used herein refers to a compound made up of the same atoms bonded by the same bonds but having different three-dimensional structures, which are not interchangeable. The present disclosure includes "enantiomers", which refers to two stereoisomers whose molecules are non-superimposable mirror images of one another.

"Tautomer" as used herein refers to a proton shift from one atom of a molecule to another atom of the same molecule. The present disclosure includes tautomers of any said compounds.

"Solvate" as used herein refers to the result of the interaction of a solvent and a compound. Solvates of salts of the compounds described herein are also provided. Hydrates of the compounds described herein are also provided.

"Hydrate" as used herein refers to a compound of the disclosure that is chemically associated with one or more molecules of water.

In some embodiments, the compound of formula (J) has the structure of formula (I): 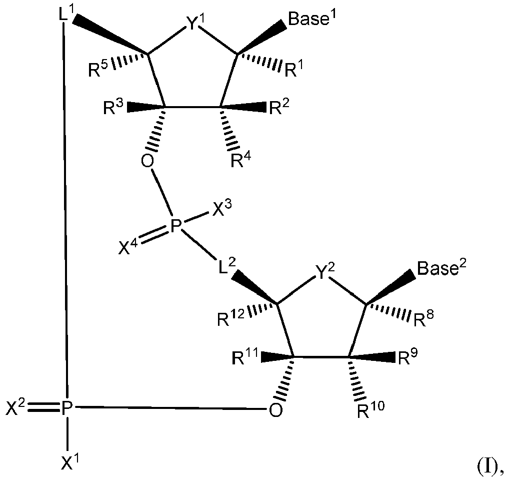 or an enantiomer, hydrate, solvate, or pharmaceutically acceptable salt thereof.

In some embodiments of the compound of formula (J) and/or (I), X² and X⁴ are each O.

In some embodiments of the compound of formula (J) and/or (I), Y² is -O- or -CH₂-. In some embodiments, Y² is -O-.

In some embodiments of the compound of formula (J) and/or (I), R¹, R², R⁵, R⁸, R¹¹, and R¹² are each independently H, OH, F, Cl, CN, N₃, or C₁-C₆ alkyl. In some embodiments, R¹, R², R⁵, R⁸, R¹¹, and R¹² are each independently H, OH, F, CN, or C₁-C₆ alkyl. In some embodiments, R¹, R², R⁵, R⁸, R¹¹, and R¹² are each independently H, CN, or C₁-C₆ alkyl. In some embodiments, R¹, R², R⁵, R⁸, R¹¹, and R¹² are each H.

In some embodiments of the compound of formula (J) and/or (I), Y¹ is -O- or -CH₂-. In some embodiments, Y¹ is -O-. In some embodiments, Y¹ is -CH₂-.

In some embodiments of the compound of formula (J) and/or (I), (Ia), X¹ and X³ are each independently OR¹⁵ or SH. In some embodiments, X¹ and X³ are each independently OR¹⁵. In some embodiments, R¹⁵ is C₁-C₆ alkyl optionally substituted with 1 or 2 -O(C=O)OR^{B}; -O(C=O)R^{B}; or -COOR^{B}, for example, R¹⁵ can be a C₁-C₆ alkyl substituted with a -O(C=O)OR^{B}. In some embodiments, R^{B} is a C₁-C₆ alkyl, such as methyl, ethyl, n-propyl, isopropyl, n-butyl, tert-butyl, sec-butyl, n-pentyl, n-hexyl, sec-hexyl, or tert-hexyl.

In some embodiments, X¹ and X³ are each OR¹⁵, wherein R¹⁵ is CH₂ substituted with an -O(C=O)R^{B}, wherein R^{B} is tert-butyl. In some embodiments, X¹ and X³ are each OR¹⁵, wherein R¹⁵ is CH₂ substituted with an -O(C=O)OR^{B}, wherein R^{B} is isopropyl. In some embodiments, X¹ is OH or SH, and X³ is OH. In some embodiments, X¹ and X³ are each OH. In some embodiments, X¹ is SH and X³ is OH.

In some embodiments, the compound of formula (J) has a structure of formula (IIa): 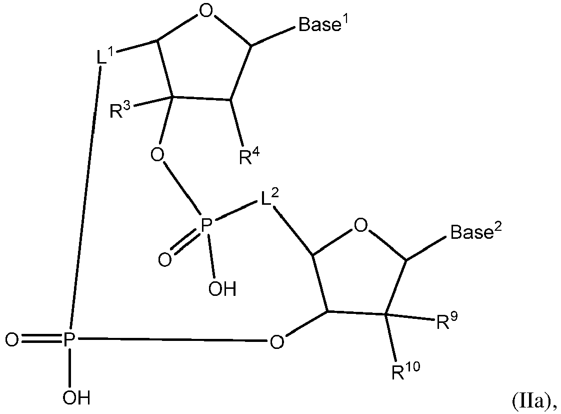 or an enantiomer, hydrate, solvate, or pharmaceutically acceptable salt thereof.

In some embodiments, the compound of formula (J), (I) and/or (IIa) has a structure of formula (Ia): 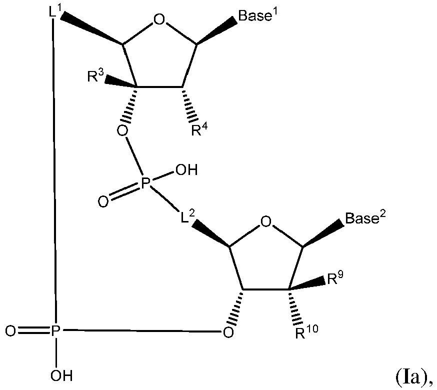 or an enantiomer, hydrate, solvate, or pharmaceutically acceptable salt thereof.

In some embodiments of the compound of formula (J), (I), (Ia), and/or (IIa), R³ and R⁴ are each independently H, OR¹⁵, F, Cl, CN, N₃, or C₁-C₆ alkyl. In some embodiments, R³ and R⁴ are each independently H, OH, or F. In some embodiments, at least one of R³ and R⁴ is H. In some embodiments, R¹⁵ is each independently H or C₁-C₆ alkyl.

In some embodiments of the compound of formula (J), (I), (Ia), and/or (IIa), R⁹ is H, OH, F, Cl, CN, N₃, or C₁-C₆ alkyl. In some embodiments, R⁹ is H, OH, F, Cl, CN, or C₁-C₆ alkyl. In some embodiments, R⁹ is H, OH, or F. In some embodiments, R⁹ is H. In some embodiments, R⁹ is OH. In some embodiments, R⁹ is F.

In some embodiments of the compound of formula (J), (I), (Ia), and/or (IIa), R⁹ and R¹⁰ are each independently H, OR¹⁵, F, Cl, CN, N₃, or C₁-C₆ alkyl. In some embodiments, R⁹ and R¹⁰ are each independently H, OH, or F. In some embodiments, at least one of R⁹ and R¹⁰ is H. In some embodiments, R¹⁵ is each independently H or C₁-C₆ alkyl.

In some embodiments of the compound of formula (J), (I), (Ia), and/or (IIa), R⁹ is OH and R¹⁰ is H. In some embodiments, R⁹ is F and R¹⁰ is H. In some embodiments, R⁹ is H and R¹⁰ is OH. In some embodiments, R⁹ is H and R¹⁰ is F.

In some embodiments of the compound of formula (J), (I), (Ia), and/or (IIa), R⁹ is H, OH, F, Cl, CN, N₃, or C₁-C₆ alkyl, and R¹⁰ is H. In some embodiments, R⁹ is H, OH, F, Cl, CN, or C₁-C₆ alkyl, and R¹⁰ is H. In some embodiments, R⁹ is H, OH, or F, and R¹⁰ is H. In some embodiments, R⁹ and R¹⁰ are each H. In some embodiments, R⁹ is OH, and R¹⁰ is H. In some embodiments, R⁹ is F, and R¹⁰ is H.

In some embodiments of the compound of formula (J), (I), (Ia), and/or (IIa), R⁹ is H, and R¹⁰ is H, OH, F, Cl, CN, N₃, or C₁-C₆ alkyl. In some embodiments, R⁹ is H, and R¹⁰ is H, OH, F, Cl, CN, or C₁-C₆ alkyl. In some embodiments, R⁹ is H, and R¹⁰ is H, OH, or F. In some embodiments, R⁹ and R¹⁰ are each H. In some embodiments, R⁹ is H, and R¹⁰ is OH. In some embodiments, R⁹ is H, and R¹⁰ is F.

As depicted in the formula (J), (I), (Ia), and/or (IIa), the connection of the atoms in L¹ and L² are as read from left to right. For example, in a compound of formula (IIa), when L² is -O-C(R⁶R⁷)-, the oxygen atom is attached to the phosphorus atom and the carbon in -C(R⁶R⁷)- is attached to the tetrahydrofuran ring, as distinct from when L² is -C(R⁶R⁷)-O-, wherein the oxygen atom is attached to the tetrahydrofuran ring, and the carbon in - C(R⁶R⁷)- is attached to the phosphorus atom.

In some embodiments of the compound of formula (J), (I), (Ia), and/or (IIa), L¹ and L² are each independently -O-C(R⁶R⁷)-, -C(R⁶R⁷)-O-, or -C(R¹³)=C(R¹⁴)-, wherein at least one of L¹ and L² is - C(R¹³)=C(R¹⁴)-. In some embodiments, L¹ and L² are each independently -O-C(R⁶R⁷)-, or - C(R¹³)=C(R¹⁴)-, wherein at least one of L¹ and L² is not -O-CH₂-. In some embodiments, L¹ is -C(R⁶R⁷)-O- and L² is -C(R¹³)=C(R¹⁴)-, L¹ is -O-C(R⁶R⁷)- and L² is -C(R¹³)=C(R¹⁴)-, L¹ is -C(R¹³)=C(R¹⁴)- and L² is -C(R⁶R⁷)-O-, or L¹ is -C(R¹³)=C(R¹⁴)- and L² is -O-C(R⁶R⁷)-. In some embodiments, L¹ is - C(R⁶R⁷)-O- and L² is -C(R¹³)=C(R¹⁴)- or L¹ is -O-C(R⁶R⁷)- and L² is -C(R¹³)=C(R¹⁴)-. In some embodiments, L¹ is -C(R⁶R⁷)-O- and L² is -C(R¹³)=C(R¹⁴)-. In some embodiments, L¹ is -O-C(R⁶R⁷)- and L² is -C(R¹³)=C(R¹⁴)-. In some embodiments, at least one of L¹ and L² is not -O-CH₂-.

In some embodiments, the compound of formula (J) has the structure of formula (IIIb): 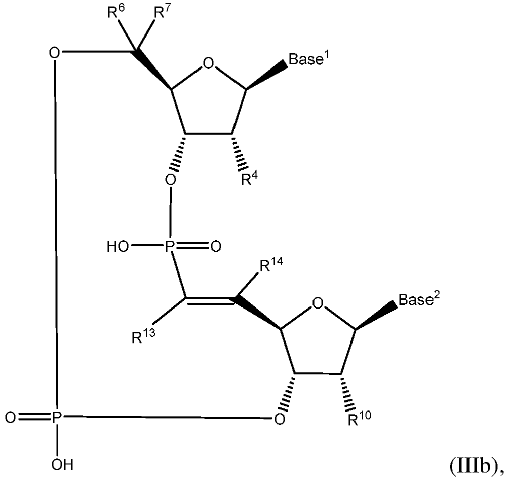 or an enantiomer, hydrate, solvate, or pharmaceutically acceptable salt thereof.

In some embodiments of the compound of formula (J), (I), (Ia), (IIa), and/or (IIIb), R⁶ and R⁷ are each independently H, CN, F, Cl, COOR¹⁵, CON(R¹⁵)₂, OR¹⁵, SR¹⁵, N(R¹⁵)₂, C₁-C₆ alkyl, C₂-C₆ alkenyl, or C₂-C₆ alkynyl. In some embodiments, R⁶ and R⁷ are each independently H, CN, F, Cl, COOR¹⁵, CON(R¹⁵)₂, OR¹⁵, SR¹⁵, N(R¹⁵)₂, or C₁-C₆ alkyl, wherein each R¹⁵ is independently H or C₁-C₆ alkyl. In some embodiments, R⁶ and R⁷ are each independently H, CN, F, Cl, CH₂OH, or CH₂N₃. In some embodiments, R⁶ and R⁷ are each H.

In some embodiments of the compound of formula (J), (I), (Ia), (IIa), and/or (IIIb), R¹³ and R¹⁴ are each independently H, CN, F, Cl, COOR¹⁵, CON(R¹⁵)₂, OR¹⁵, SR¹⁵, N(R¹⁵)₂, C₁-C₆ alkyl, C₂-C₆ alkenyl, or C₂-C₆ alkynyl. In some embodiments, R¹³ and R¹⁴ are each independently H, CN, F, Cl, COOR¹⁵, CON(R¹⁵)₂, OR¹⁵, SR¹⁵, N(R¹⁵)₂, or C₁-C₆ alkyl, wherein each R¹⁵ is independently H or C₁-C₆ alkyl. In some embodiments, R¹³ and R¹⁴ are each independently H, CN, F, Cl, CH₂OH, or CH₂N₃. In some embodiments, R ¹³ and R¹⁴ are each H.

In some embodiments of the compound of formula (J), (I), (Ia), (IIa), and/or (IIIb), R⁶, R⁷, R¹³ and R¹⁴ are each independently H, CN, F, Cl, COOR¹⁵, CON(R¹⁵)₂, OR¹⁵, SR¹⁵, N(R¹⁵)₂, C₁-C₆ alkyl, C₂-C₆ alkenyl, or C₂-C₆ alkynyl. In some embodiments, R⁶, R⁷, R¹³ and R¹⁴ are each independently H, CN, F, Cl, COOR¹⁵, CON(R¹⁵)₂, OR¹⁵, SR¹⁵, N(R¹⁵)₂, or C₁-C₆ alkyl, wherein each R¹⁵ is independently H or C₁-C₆ alkyl. In some embodiments, R⁶, R⁷, R¹³ and R¹⁴ are each independently H, CN, F, Cl, CH₂OH, or CH₂N₃. In some embodiments, R⁶, R⁷, R¹³ and R¹⁴ are each H.

In some embodiments of the compound of formula (J), (I), (Ia), (IIa), and/or (IIIb), R¹⁰ is H, OR¹⁵, F, Cl, CN, N₃, or C₁-C₆ alkyl. In some embodiments, R¹⁰ is H, F, Cl, CN, N₃, OR¹⁵, SR¹⁵, or N(R¹⁵)₂. In some embodiments, R¹⁰ is H, F, CN, N₃, OR¹⁵, SR¹⁵, or N(R¹⁵)₂. In some embodiments, R¹⁰ is H, F, CN, OR¹⁵, SR¹⁵, or N(R¹⁵)₂, wherein R¹⁵ is each independently H or C₁-C₆ alkyl. In some embodiments, R¹⁰ is H, OH, F, Cl, CN, or C₁-C₆ alkyl. In some embodiments, R¹⁰ is H, OH, or F. In some embodiments, R¹⁰ is H. In some embodiments, R¹⁰ is OH. In some embodiments, R¹⁰ is F.

In some embodiments of the compound of formula (J), (I), (Ia), (Ila), and/or (IIIb), R⁴ is H, OR¹⁵, F, Cl, CN, N₃, or C₁-C₆ alkyl. In some embodiments, R⁴ is H, F, Cl, CN, N₃, OR¹⁵, SR¹⁵, or N(R¹⁵)₂. In some embodiments, R⁴ is H, F, CN, N₃, OR¹⁵, SR¹⁵, or N(R¹⁵)₂. In some embodiments, R⁴ is H, F, CN, OR¹⁵, SR¹⁵, or N(R¹⁵)₂, wherein R¹⁵ is each independently H or C₁-C₆ alkyl. In some embodiments, R⁴ is H, OH, F, Cl, CN, or C₁-C₆ alkyl. In some embodiments, R⁴ is H, OH, or F. In some embodiments, R⁴ is H. In some embodiments, R⁴ is OH. In some embodiments, R⁴ is F.

In some embodiments of the compound of formula (J), (I), (Ia), (IIa), and/or (IIIb), R⁴ and R¹⁰ are each independently H, OR¹⁵, F, Cl, CN, N₃, or C₁-C₆ alkyl. In some embodiments, R⁴ and R¹⁰ are each independently H, F, Cl, CN, N₃, OR¹⁵, SR¹⁵, or N(R¹⁵)₂. In some embodiments, R⁴ and R¹⁰ are each independently H, F, CN, N₃, OR¹⁵, SR¹⁵, or N(R¹⁵)₂. In some embodiments, R⁴ and R¹⁰ are each independently H, F, CN, OR¹⁵, SR¹⁵, or N(R¹⁵)₂, wherein R¹⁵ is each independently H or C₁-C₆ alkyl. In some embodiments, R⁴ and R¹⁰ are each independently H, OR¹⁵, F, or CN. In some embodiments, R⁴ and R¹⁰ are each independently H, OH, F, or CN. In some embodiments, R⁴ and R¹⁰ are each independently H, OH, or F. In some embodiments, R⁴ is OH, and R¹⁰ is H, OH, or F. In some embodiments, R⁴ and R¹⁰ are each independently H or OH. In some embodiments, R⁴ and R¹⁰ are each independently OH or F. In some embodiments, R⁴ and R¹⁰ are each OH. In some embodiments, R⁴ is H, and R¹⁰ is OH. In some embodiments, R⁴ is OH, and R¹⁰ is H. In some embodiments, R⁴ is OH, and R¹⁰ is F.

In some embodiments of the compound of formula (J), (I), (Ia), (IIa), and/or (IIIb), Base¹ and Base² are each independently: 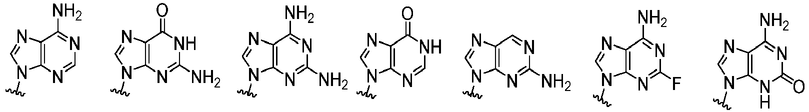 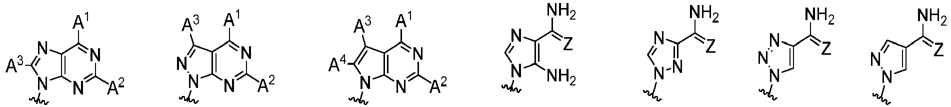 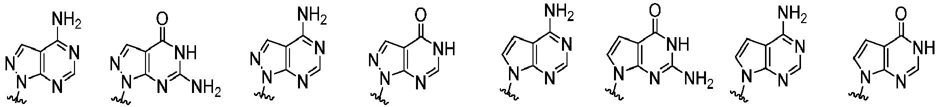 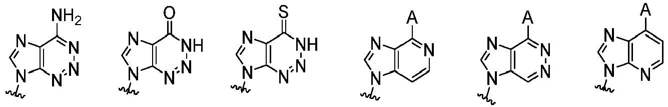 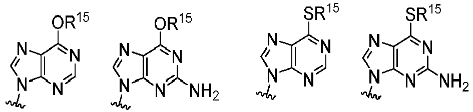 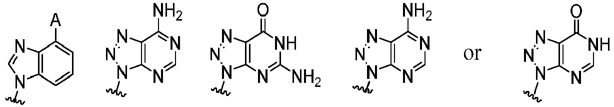 wherein
A, A¹, A², A³ and A⁴ are each independently H, OH, SH, F, Cl, Br, I, NH₂, OR¹⁵, SR¹⁵, NHR¹⁵, N(R¹⁵)₂, or R¹⁶.

In some embodiments, Base¹ and Base² are each independently: 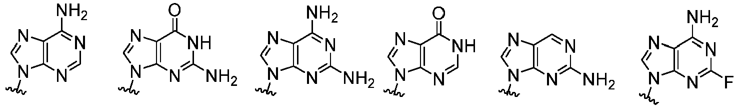 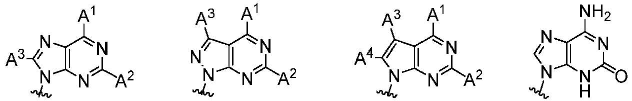 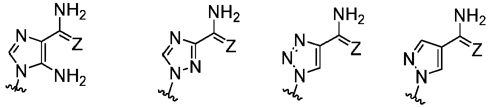 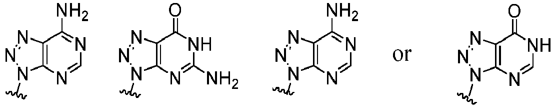 wherein
A, A¹, A², A³ and A⁴ are each independently H, OH, SH, F, Cl, Br, I, NH₂, OR¹⁵, SR¹⁵, NHR¹⁵, N(R¹⁵)₂, or R¹⁶.

In some embodiments, Base¹ and Base² are each independently:

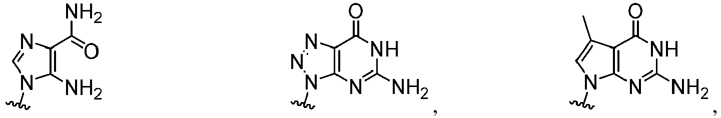

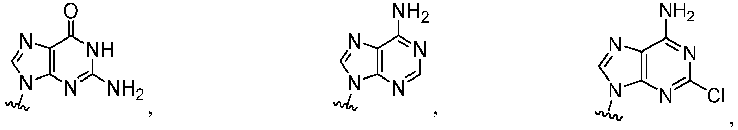

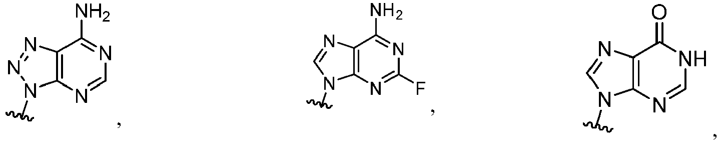

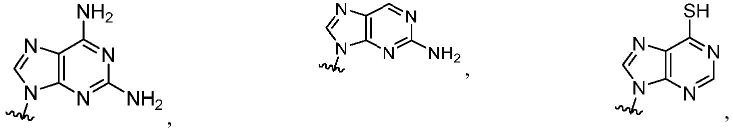

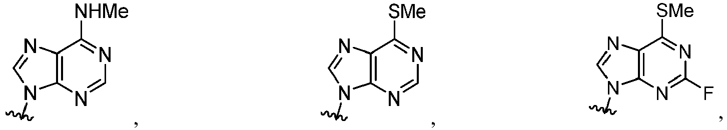

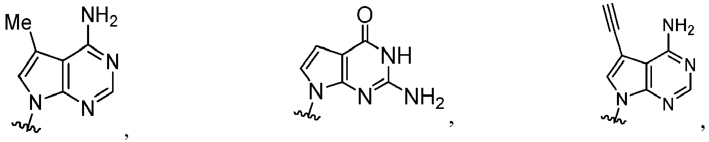

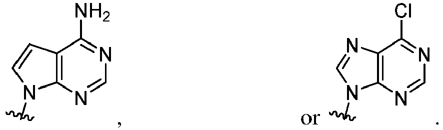

In some embodiments, Base¹ is: 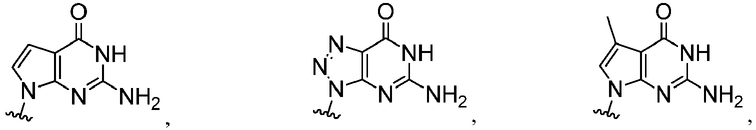 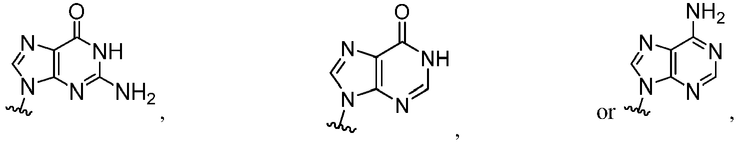 and Base² is:

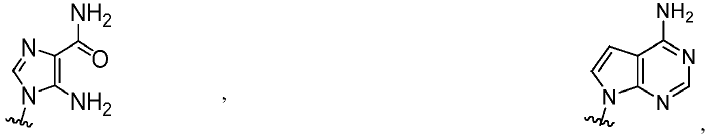

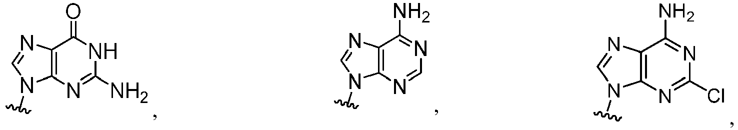

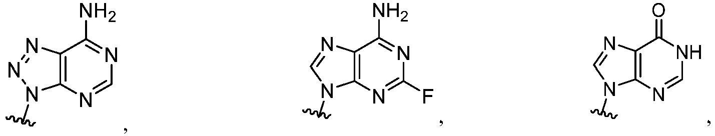

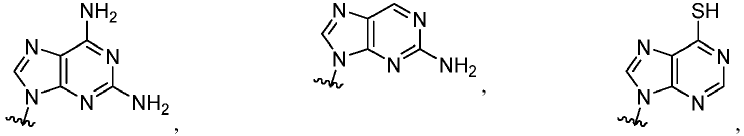

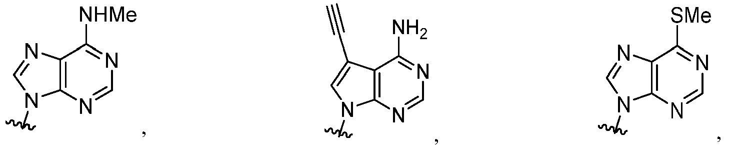

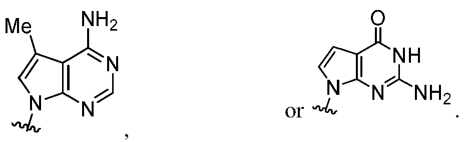

In some embodiments, Base¹ is: 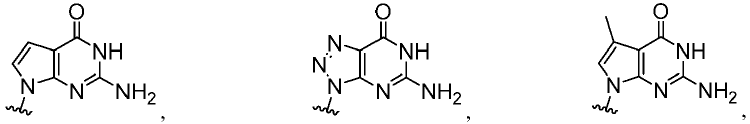 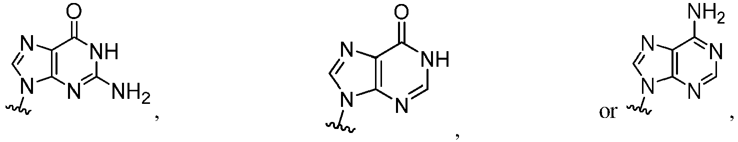 and Base² is

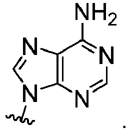

In some embodiments, Base¹ is 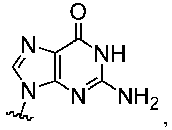 and Base² is:

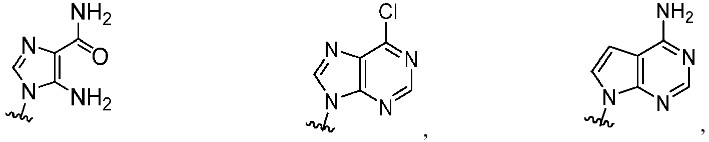

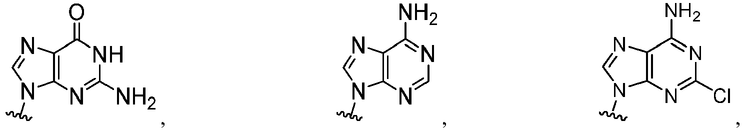

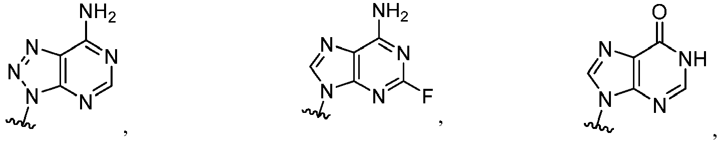

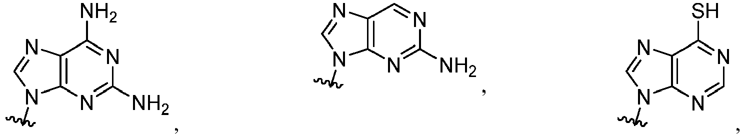

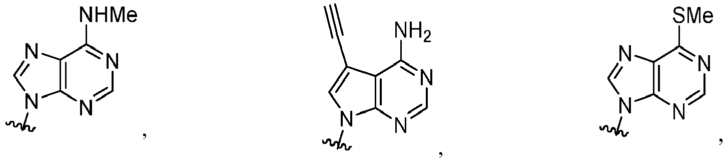

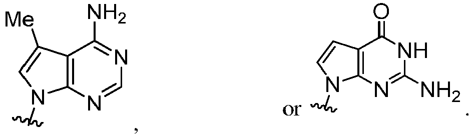

In some embodiments,

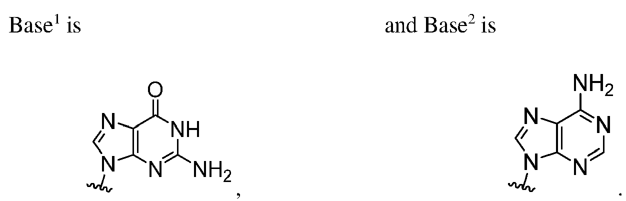

In some embodiments,

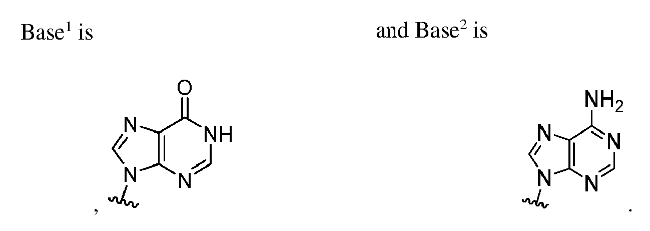

In some embodiments of the compound of formula (J), (I), (Ia), (Ila), and/or (IIIb), Base¹ and Base² are each independently: 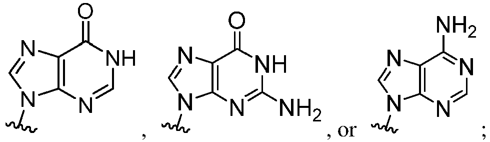 R⁶, R⁷, R¹³ and R¹⁴ are each H; and R⁴ and R¹⁰ are each independently H, OH, or F.

In some embodiments of the compound of formula (J), (I), (Ia), (IIa), and/or (IIIb), Base¹ is Base² is 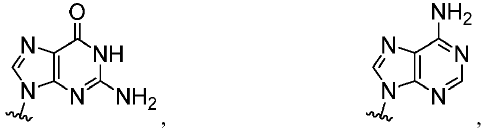 R⁶, R⁷, R¹³ and R¹⁴ are each H; and R⁴ and R¹⁰ are each independently H, OH, or F.

In some embodiments of the compound of formula (J), (I), (Ia), (IIa), and/or (IIIb), Base¹ is Base² is 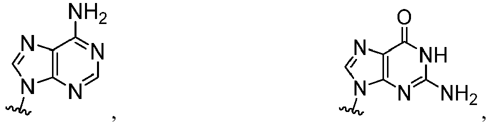 R⁶, R⁷, R¹³ and R¹⁴ are each H; and R⁴ and R¹⁰ are each independently H, OH, or F.

In some embodiments of the compound of formula (J), the compound has the structure: 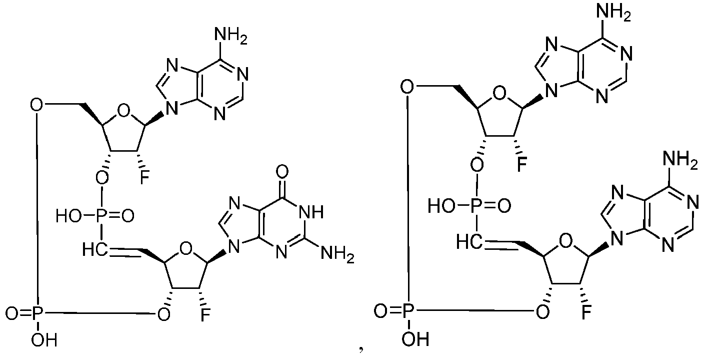 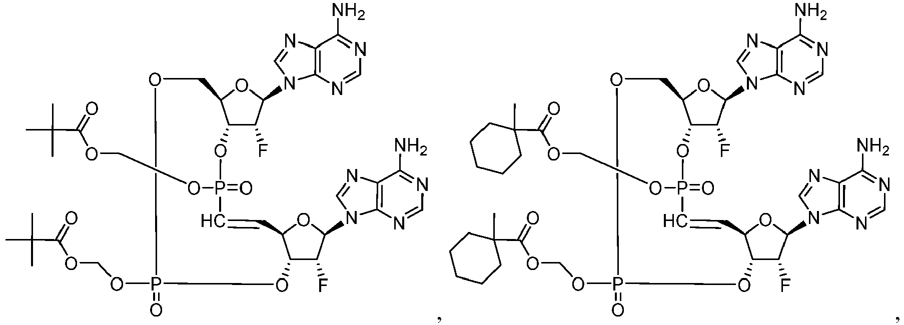 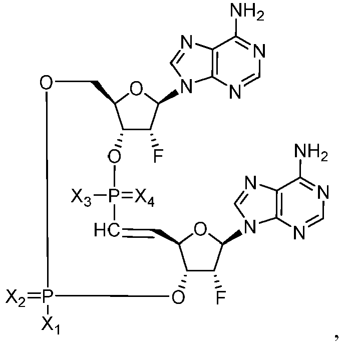
wherein X¹, X², X³, X⁴ are as defined for structure (J);
or an enantiomer, hydrate, solvate, or pharmaceutically acceptable salt thereof.

A compound of the disclosure, e.g, a compound of formula (J), (I), (Ia), (IIa), and/or (IIIb), can be shown in a number of equivalent depictions. For example, a compound of Formula (IIIb) is typically depicted herein as shown above wherein each nucleoside is shown in the 5' to 3'-direction from upper left to lower right:

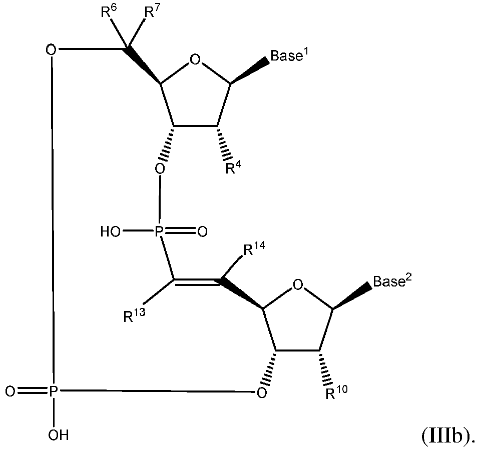

The above compound of Formula (IIIb) is equivalent to a compound of Formula (IIIb) as shown below:

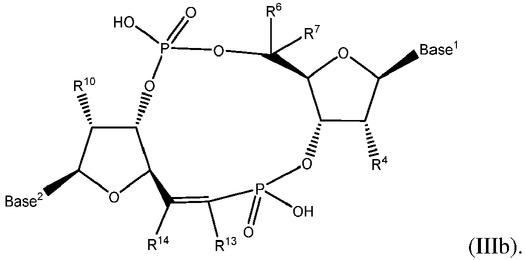

Further, each of the previous depictions are equivalent to the below depiction of a compound of Formula (IIIb) :

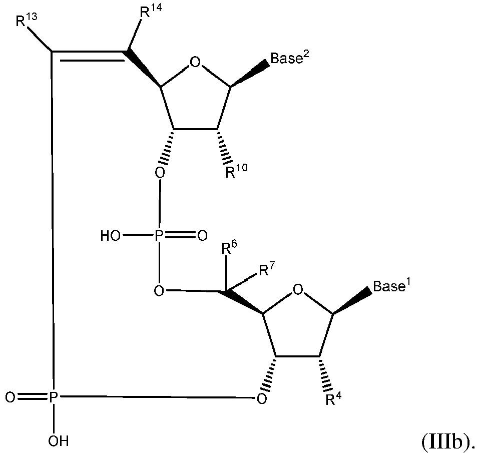

### I. COMPOSITIONS

In certain embodiments, the present disclosure provides a pharmaceutical composition comprising a compound of the present disclosure (e.g. a compound of Formula (J), (I), (Ia), (IIa), and/or (IIIb)), or a pharmaceutically acceptable salt thereof, and a pharmaceutically acceptable excipient.

In certain embodiments, the pharmaceutical composition comprises one or more additional therapeutic agent, as more fully set forth below.

Pharmaceutical compositions comprising the compounds disclosed herein, or pharmaceutically acceptable salts thereof, may be prepared with one or more pharmaceutically acceptable excipients which may be selected in accord with ordinary practice. Tablets may contain excipients including glidants, fillers, binders and the like. Aqueous compositions may be prepared in sterile form, and when intended for delivery by other than oral administration generally may be isotonic. Excipients can include ascorbic acid and other antioxidants, chelating agents such as EDTA, carbohydrates such as dextrin, hydroxyalkylcellulose, hydroxyalkylmethylcellulose, stearic acid and the like. In certain embodiments, the composition is provided as a solid dosage form, including a solid oral dosage form, including capsules, sachets or tablets each containing a predetermined amount of the active ingredient. In one embodiment, the pharmaceutical composition is a tablet.

The compositions include those suitable for various administration routes, including oral administration. The compositions may be presented in unit dosage form and may be prepared by any of the methods well known in the art of pharmacy.

Pharmaceutical compositions disclosed herein comprise one or more compounds disclosed herein, or a pharmaceutically acceptable salt thereof, together with a pharmaceutically acceptable excipient and optionally other therapeutic agents. Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more excipients including sweetening agents, flavoring agents, coloring agents and preserving agents, in order to provide a palatable preparation. Tablets containing the active ingredient in admixture with non-toxic pharmaceutically acceptable excipients which are suitable for manufacture of tablets are acceptable. These excipients may be, for example, inert diluents, such as calcium or sodium carbonate, lactose, lactose monohydrate, croscarmellose sodium, povidone, calcium or sodium phosphate; granulating and disintegrating agents, such as maize starch, or alginic acid; binding agents, such as cellulose, microcrystalline cellulose, starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

The amount of active ingredient that may be combined with the inactive ingredients to produce a dosage form may vary depending upon the intended treatment subject and the particular mode of administration. For example, in some embodiments, a dosage form for oral administration to humans may contain approximately 1 to 1000 mg of active material formulated with an appropriate and convenient amount of a pharmaceutically acceptable excipient. In certain embodiments, the pharmaceutically acceptable excipient varies from about 5 to about 95% of the total compositions (weight: weight).

The compounds of the present disclosure (also referred to herein as the active ingredients) can be administered by any route appropriate to the condition to be treated. Suitable routes include oral, rectal, nasal, topical (including buccal and sublingual), transdermal, vaginal and parenteral (including subcutaneous, intramuscular, intravenous, intradermal, intratumoral, intrathecal and epidural), and the like. It will be appreciated that the preferred route may vary with for example the condition of the recipient. An advantage of certain compounds disclosed herein is that they are orally bioavailable and can be dosed orally.

A compound of the present disclosure may be administered to an individual in accordance with an effective dosing regimen for a desired period of time or duration, such as at least about one month, at least about 2 months, at least about 3 months, at least about 6 months, or at least about 12 months or longer

A compound of the present disclosure may be combined with one or more additional therapeutic agents in any dosage amount of the compound of the present disclosure (e.g., from 1 mg to 1000 mg of compound).

Kits that comprise a compound of the present disclosure, or an enantiomer, or pharmaceutically acceptable salt thereof, or a pharmaceutical composition containing any of the above, are also included in the present disclosure.

In one embodiment, kits comprising a compound of the present disclosure, or an enantiomer, hydrate, solvate, or a pharmaceutically acceptable salt thereof, in combination with one or more (e.g., one, two, three, four, one or two, or one to three, or one to four) additional therapeutic agents are provided.

The disclosure further includes a compound of the invention or a pharmaceutical composition as described above for use in modulating STING protein activity, to induce STING-dependent production of type I interferons, cytokines or chemokines.

The disclosure further includes a compound of the invention or a pharmaceutical composition as described above for use in treating or preventing viral infection, infection caused by hepatitis B virus, by HIV, allergic diseases, inflammatory diseases, hyperproliferative disease or cancer.

In some embodiments, the compound of the invention or the pharmaceutical compositions described above are for use in a human or an animal.

The Stimulator of interferon genes (STING) adaptor protein, also known as STING, STING protein, transmembrane protein 173 (TMEM173), MPYS, mediator of IRF3 activation (MITA), or endoplasmic reticulum interferon stimulator (ERIS), is a protein that in humans is encoded by the TMEM173 gene (UniProt code Q86WV6; NCBI Reference Sequences: NP_938023.1 (isoform 1) and NP_001288667 (isoform 2)). STING adaptor protein is believed to function as both a direct cytosolic DNA sensor (CDS) and an adaptor protein in Type I interferon signaling through different molecular mechanisms. STING adaptor protein has been shown to activate downstream transcription factors STAT6 and IRF3 through TBK1, and NF-κB through IKKβ, which can effect an antiviral response or innate immune response against an intracellular pathogen. STING adaptor protein plays a role in innate immunity by inducing type I interferon production when cells are infected with intracellular pathogens, such as viruses, mycobacteria and intracellular parasites. Type I interferon, mediated by STING adaptor protein, protects infected cells and nearby cells from local infection by autocrine and paracrine signaling.

Activation of STING adaptor protein in turn activates protein kinase TBK1, which subsequently activates downstream transcription factors NF-κB and IRF-3. Activation of STING adaptor protein ultimately is believed to result in the release of type I and III interferons as well as a variety of cytokines and chemokines such as IL-6, TNF-α and INF-γ. Accordingly, induction of a STING adaptor protein-dependent type I interferon, cytokine or chemokine in a human or animal results in the activation of one or more of NF-κB, IRF-3, a type I interferon, a type III interferon, IL-6, TNF-α, and INF-γ in said human or animal.

Viral infections that can be treated or prevented by the compounds of the present disclosure can be any infection caused by a virus, e.g., a virus from the *Hepadnaviridae* family of viruses, e.g., hepatitis B; or any retrovirus, e.g., an alpharetrovirus, such as Rous sarcoma virus; a betaretrovirus, such as simian retrovirus; a deltaretrovirus, such as bovine leukemia virus or human T-lymphotrophic virus (HTLV) including HTLV-1, HTLV-2, and HTLV-3; a gammaretrovirus, such as murine leukemia virus or feline leukemia virus; or a lentivirus, such as human immunodeficiency virus (HIV) including HIV-1 and HIV-2, simian immunodeficiency virus, equine infectious anemia virus, bovine immunodeficiency virus, rabbit endogenous lentivirus type K (RELIK), or feline immunodeficiency virus.

Hyperproliferative diseases that can be treated or prevented by the compounds of the present disclosure include diseases caused by excessive growth of non-cancer cells. Such conditions include but are not limited to psoriasis, actinic keratoses, and seborrheic keratoses, warts, keloids, and eczema.

Cancers that can be treated or prevented by the compounds of the disclosure include solid tumors and lymphomas, including but not limited to adrenal cancer, bladder cancer, bone cancer, brain cancer, breast cancer, colon cancer, colorectal cancer, eye cancer, head-and-neck cancer, kidney cancer such as renal cell carcinoma, liver cancer, lung cancer such as non-small cell lung cancer, ovarian cancer, pancreatic cancer, prostate cancer, skin cancer such as squamous cell carcinoma and melanoma, thyroid cancer, uterine cancer, vaginal cancer, and myeloma such as multiple myeloma. The cancer can be naive, or relapsed and/or refractory.

In some embodiments, the cancer is Burkitt's lymphoma, Hodgkin's lymphoma, non-Hodgkin's lymphoma (NHL), indolent non-Hodgkin's lymphoma (iNHL), refractory iNHL, multiple myeloma (MM), chronic myeloid leukemia (CML), acute lymphocytic leukemia (ALL), B-cell ALL, acute myeloid leukemia (AML), chronic lymphocytic leukemia (CLL), small lymphocytic lymphoma (SLL), myelodysplastic syndrome (MDS), myeloproliferative disease (MPD), mantle cell lymphoma (MCL), follicular lymphoma (FL), Waldestrom's macroglobulinemia (WM), T-cell lymphoma, B-cell lymphoma, diffuse large B-cell lymphoma (DLBCL), or marginal zone lymphoma (MZL). In one embodiment, the cancer is minimal residual disease (MRD). In some embodiments, the cancer is selected from Hodgkin's lymphoma, non-Hodgkin's lymphoma (NHL), indolent non-Hodgkin's lymphoma (iNHL), and refractory iNHL. In some embodiments, the cancer is indolent non-Hodgkin's lymphoma (iNHL). In some embodiments, the cancer is refractory iNHL. In some embodiments, the cancer is chronic lymphocytic leukemia (CLL). In some embodiments, the cancer is diffuse large B-cell lymphoma (DLBCL).

In some embodiments, the cancer is a solid tumor selected from the group consisting of pancreatic cancer; bladder cancer; colorectal cancer; breast cancer, including metastatic breast cancer; prostate cancer, including androgen-dependent and androgen-independent prostate cancer; kidney or renal cancer, including, e.g., metastatic renal cell carcinoma; hepatocellular cancer; lung cancer, including, e.g., non-small cell lung cancer (NSCLC), bronchioloalveolar carcinoma (BAC), and adenocarcinoma of the lung; ovarian cancer, including, e.g., progressive epithelial or primary peritoneal cancer; cervical cancer; gastric cancer; esophageal cancer; head and neck cancer, including, e.g., squamous cell carcinoma of the head and neck; melanoma; neuroendocrine cancer, including metastatic neuroendocrine tumors; brain tumors, including, e.g., glioma, anaplastic oligodendroglioma, adult glioblastoma multiforme, and adult anaplastic astrocytoma; bone cancer; and soft tissue sarcoma, hepatic carcinoma, rectal cancer, penile carcinoma, vulval cancer, thyroid cancer, salivary gland carcinoma, endometrial or uterine carcinoma, hepatoma, hepatocellular cancer, liver cancer, gastric or stomach cancer including gastrointestinal cancer, cancer of the peritoneum, squamous carcinoma of the lung, gastroesophagal cancer, biliary tract cancer, gall bladder cancer, colorectal/appendiceal cancer, squamous cell cancer (e.g., epithelial squamous cell cancer).

Any of the compounds provided herein may be used to treat cancer at various stages. By way of example, the cancer stage includes but is not limited to early, advanced, locally advanced, remission, refractory, reoccurred after remission and progressive.

### Subjects

The compounds of the invention provided herein may be used to treat a subject (e.g., human) who has been diagnosed with or is suspected of having cancer. As used herein, a subject refers to a mammal, including, for example, a human.

The disclosure includes a cyclic dinucleotide provided herein, including compounds of the disclosure, or an enantiomer, hydrate, solvate, or pharmaceutically acceptable salt thereof for use as a medicament in a human or animal.

In certain embodiments, a method for treating or preventing an infectious disease, a viral infection, hepatitis B infection, HIV infection, cancer, or a hyperproliferative disease in a human having or at risk of having the disease is provided, comprising administering to the human a therapeutically effective amount of a compound disclosed herein, e.g., a compound of Formula (J), (I), (Ia), (IIa), and/or (IIIb), or a pharmaceutically acceptable salt thereof, in combination with a therapeutically effective amount of one or more (e.g., one, two, three, four, one or two, one to three, or one to four) additional therapeutic agents. In one embodiment, a method for treating an infectious disease, a viral infection, hepatitis B infection, HIV infection, cancer, or a hyperproliferative disease in a human having or at risk of having the disease is provided, comprising administering to the human a therapeutically effective amount of a compound disclosed herein, or a pharmaceutically acceptable salt thereof, in combination with a therapeutically effective amount of one or more (e.g., one, two, three, four, one or two, one to three, or one to four) additional therapeutic agents.

### EXAMPLES

The embodiments are also directed to processes and intermediates useful for preparing the subject compounds or pharmaceutically acceptable salts thereof.

Compounds as described herein can be purified by any of the means known in the art, including chromatographic means, such as high performance liquid chromatography (HPLC), preparative thin layer chromatography, flash column chromatography and ion exchange chromatography. Any suitable stationary phase can be used, including normal and reversed phases as well as ionic resins. Most typically the disclosed compounds are purified via silica gel and/or alumina chromatography. See, *e.g.,* Introduction to Modern Liquid Chromatography, 2nd ed., ed. L. R. Snyder and J. J. Kirkland, John Wiley and Sons, 1979; and Thin Layer Chromatography, E. Stahl (ed.), Springer-Verlag, New York, 1969.

During any of the processes for preparation of the subject compounds, it may be necessary and/or desirable to protect sensitive or reactive groups on any of the molecules concerned. This may be achieved by means of conventional protecting groups as described in standard works, such as T. W. Greene and P. G. M. Wuts, "Protective Groups in Organic Synthesis," 4th ed., Wiley, New York 2006. The protecting groups may be removed at a convenient subsequent stage using methods known from the art.

Exemplary chemical entities useful in methods of the embodiments will now be described by reference to illustrative synthetic schemes for their general preparation herein and the specific examples that follow. Artisans will recognize that, to obtain the various compounds herein, starting materials may be suitably selected so that the ultimately desired substituents will be carried through the reaction scheme with or without protection as appropriate to yield the desired product. Alternatively, it may be necessary or desirable to employ, in the place of the ultimately desired substituent, a suitable group that may be carried through the reaction scheme and replaced as appropriate with the desired substituent. Furthermore, one of skill in the art will recognize that the transformations shown in the schemes below may be performed in any order that is compatible with the functionality of the particular pendant groups. Each of the reactions depicted in the general schemes is preferably run at a temperature from about 0 °C to the reflux temperature of the organic solvent used. In view of pressure 1psi = 6894 Pa.

The Examples provided herein describe the synthesis of compounds disclosed herein as well as intermediates used to prepare the compounds. It is to be understood that individual steps described herein may be combined. It is also to be understood that separate batches of a compound may be combined and then carried forth in the next synthetic step.

The methods of the present disclosure generally provide a specific enantiomer or diastereomer as the desired product, although the stereochemistry of the enantiomer or diastereomer was not determined in all cases. When the stereochemistry of the specific stereocenter in the enantiomer or diastereomer is not determined, the compound is drawn without showing any stereochemistry at that specific stereocenter even though the compound can be substantially enantiomerically or disatereomerically pure.

Representative syntheses of compounds of the present disclosure are described in schemes below, and the particular examples that follow.

The specific 3'3'-cyclic dinucleotides detailed in the Examples were synthesized according to the general synthetic method described below. Compounds were named using ChemAxon (Budapest, HU) unless otherwise indicated.

Analytical HPLC, mass spectra, UV absorbancy and purity of compounds were measured on Waters UPLC-MS system consisted of Waters UPLC H-Class Core System, UPLC PDA detector and Mass spectrometer Waters SQD2. MS method used was ESI+ and/or ESI-, cone voltage = 30 V, mass detector range 100 - 1000 Da or 500 - 1600 Da. Two sets of HPLC conditions were used as indicated: (a) C18 (column: ProntoSIL, Prontopearl C18 SH 2.2 µm, 100 × 2.0 mm; LC method: H₂O/CH₃CN, 0.1% formic acid as modifier, gradient 0 - 100 %, run length 7 min, flow 0.5 ml/min) and (b) HILIC (column: SeQuant ZIC-pHILIC, 5 µm, polymeric, 50 × 2.1 mm; LC method: CH₃CN/0.01M aqueous ammonium acetate gradient 10 - 60 %, run length 7 min, flow 0.3 ml/min).

The abbreviations used in the Examples shown below include the following:

| **Abbreviations** | |
|---|---|
| Ac | acetyl |
| ACN | acetonitrile |
| AcOH | Acetic acid |
| Bz | benzoyl |
| CE | 2-cyanoethyl |
| DCA | Dichloroacetic acid |
| DCC | Dicyclohexylcarbodiimide |
| DCE | 1,2-dichloroethane |
| DCM | dichloromethane |
| DMF | Dimethylformamide |
| DMTr | 4,4-dimethoxytrityl |
| DMOCP | 2-Chloro-5,5-dimethyl-1,3,2-dioxaphosphorinane 2-oxide |
| DMSO | dimethylsulfoxide |
| EDC | *N*-(3-Dimethylaminopropyl)-N'-ethylcarbodiimide |
| EtOH | ethanol |
| HILIC | hydrophilic interaction liquid chromatography |
| iPr | isopropyl |
| MeOH | methanol |
| Ph | Phenyl |
| PicOH | (4-Methoxy-pyridin-2-yl)-methanol |
| TBDMS | *tert*-butyldimethylsilyl |
| TBDMSCl | *tert*-butyldimethylsilyl chloride |
| TES | Triethylsilane |
| THF | tetrahydrofuran |
| TBAF | Tetrabutylammonium fluoride |
| TBHP | tert-Butyl hydroperoxide solution |
| TCA | Trichloroacetic acid |
| TEA | Triethylamine |
| TEAB | triethylammonium bicarbonate |
| TFA | Trifluoroacetic acid |
| TMSBr | Bromotrimethylsilane |
| FBS | fetal bovine serum |
| HEPES | 4-(2-hydroxyethyl)-1-piperazineethanesulfonic acid |
| BSA | bovine serum albumin |

### Synthesis of ((E)-2-((2R,3S,4R,5R)-5-(6-amino-9H-purin-9-yl)-3,4-dihydroxytetrahydrofuran-2-yl)vinyl)phosphonic diphosphoric anhydride, trisodium salt (7)

The synthesis of compound 7 from compound 1 was accomplished according to the synthetic scheme depicted in Scheme 3 below, and detailed in the following description.

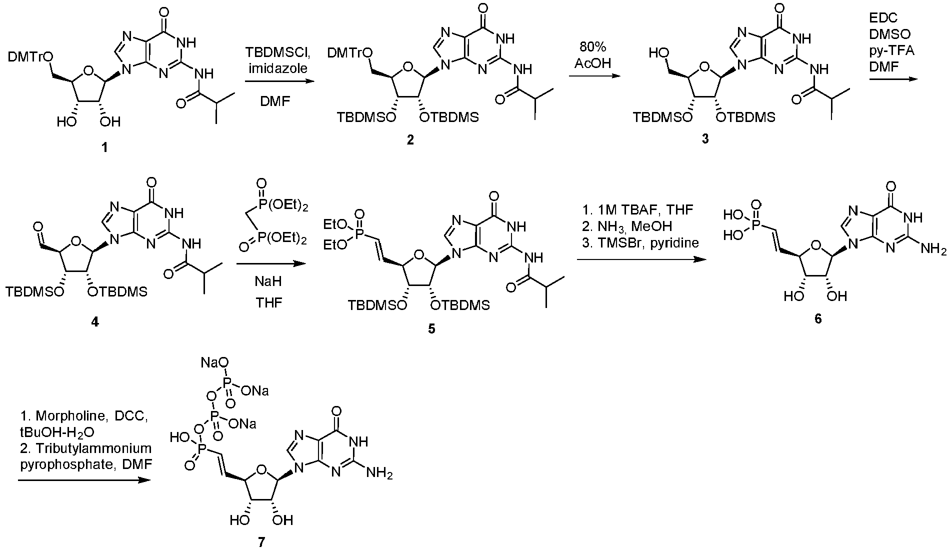

### V-(9-((2R.3R.4R.5R)-5-((bis(4-methoxyphenyl)(phenyl)methoxy)methyl)-3,4-bis((tert-butyldimethylsilyl)oxy)tetrahydrofuran-2-yl)-6-oxo-6,9-dihydro-1 H-purin-2-yl)isobutyramide (2)

To a solution of commercially available 1 (20 g, 30.5 mmol) and imidazole (12.5 g, 183 mmol) in anhydrous DMF (200 mL) was added TBDMSCl (13.8 g, 91.5 mmol) in one portion and the reaction mixture was stirred at ambient temperature overnight. Volatiles were evaporated, oily residue was diluted with ethyl acetate (1 L) and washed with sat aqueous NaHCO₃ solution (500 mL) and water (2x 500 mL). Organic phase was dried over sodium sulfate and evaporated, yielding **2** (24.8 g, HPLC purity >95%), which was used in the next reaction without further purification.

### 2',3'-Bis-O-(tert-butyldimethylsilyl)-N²-isobutyrylguanosine (3)

Crude **2** (16 g, 18 mmol) was dissolved in 80% acetic acid (200 mL) and stirred at ambient temperature for 5 hours. Reaction mixture was diluted with ethyl acetate (1 L), neutralized with solid sodium carbonate, moved to a separatory funnel and washed with water (2× 500 mL). Flash chromatography (MeOH in DCM, 2-7%) afforded **3** (10.1 g). NMR spectra were consistent with the literature (Flockerzi, Dieter; Schlosser, Wilhelm; Pfleiderer, Wolfgang. Helvetica Chimica Acta 1983 vol. 66(7), 2069 - 2085).

### Diethyl ((E)-2-((2R,3R,4R,5R)-3,4-bis((tert-butyldimethylsilyl)oxy)-5-(2-isobutyrafnido-6-oxo-1,6-dihydro-9H-purin-9-yl)tetrahydrofuran-2-yl)vinyl)phosphonate (4)

To a solution of **3** (8.1 g, 13.9 mmol) in anhydrous DMF (80 mL) was added DMSO (5.93 mL, 83.5 mmol) and EDC.HCl (8 g, 41.8 mmol) followed by dropwise addition of a solution of pyridine (1.11 mL) and TFA (0.56 mL) in DMF (28 mL). Reaction mixture was stirred at ambient temperature for 2 hours, diluted with ethyl acetate (500 mL) and washed with saturated aqueous NaHCO₃ solution (250 mL) and water (2× 250 mL). Column chromatography in toluene-acetone gradient 4:1 to 1:4 afforded **4** (6.3 g). LCMS: [598]⁺ (hydrate).

To an ice cooled suspension of NaH (223 mg, 60% dispersion in oil, 5.6 mmol) in anhydrous THF (80 mL) under argon was dropwise added tetraethyl methylenebis(phosphonate) (2.08 mL, 8.3 mmol) and mixture was stirred at 0 °C for 20 minutes. A solution of **4** (3.23 g, 5.6 mmol) in anhydrous THF (40 mL) was slowly added and reaction mixture was stirred at ambient temperature overnight. Reaction was quenched by adding 50 mL of saturated aqueous NH₄Cl solution, diluted with water (250 mL) and extracted with ethyl acetate (2× 250 mL). Combined organic phases were dried over sodium sulfate and evaporated. Flash chromatography (MeOH in DCM, 2-10 %) afforded **5** (2.5 g): HRMS ESI (C₃₁H₅₇O₈N₅PSi₂) calculated: 714.34778; found: 714.34838; ¹H NMR (401 MHz, DMSO) δ 12.18 (s, 1H), 11.37 (s, 1H), 8.27 (s, 1H), 7.42 (ddd, *J* = 23.1, 17.2, 6.9 Hz, 1H), 6.19 (ddd, *J* = 20.9, 17.2, 1.3 Hz, 1H), 5.86 (d, *J* = 7.3, 1H), 4.88 (dd, *J* = 7.2, 4.2 Hz, 1H), 4.60 (ddd, *J* = 6.9, 2.9, 1.4, Hz, 1H), 4.23 (dd, *J* = 4.3, 1.5 Hz, 1H), 4.09 - 3.95 (m, 4H), 2.89 (hept, *J* = 6.8 Hz, 1H), 1.26 and 1.23 (t, 3H, *J* = 7.0 Hz), 1.14 (d, J= 6.8 Hz, 6H), 0.93 and 0.73 (s, 9H), 0.15, 0.13, -0.10, -0.41 (s, 3H); ¹³C NMR (101 MHz, DMSO) δ 180.50, 154.90, 150.26 (d, *J* = 5.6 Hz), 148.45, 148.03, 139.79, 121.45, 118.58 (d, *J* = 181.8 Hz), 87.86, 85.81 (d, *J* = 24.4 Hz), 76.26, 73.73, 61.95 and 61.90 (d, *J* = 5.1 Hz), 34.84, 25.89, 25.61, 19.06, 19.03, 17.99, 17.68, 16.35 and 16.32 (d, *J* = 5.9 Hz), -4.55, -4.60, -5.68; ³¹P NMR (162 MHz, DMSO) δ 20.77.

### ((E)-2-((2R,3S,4R,5R)-5-(2-amino-6-oxo-1,6-dihydro-9H-purin-9-yl)-3,4-dihydroxytetrahydrofuran-2-yl)vinyl)phosphonic acid (6)

To a solution of **5** (360 mg, 0.5 mmol) in THF (5 mL) was added TBAF (1 M in THF, 2 mL) and the reaction mixture was stirred at ambient temperature for 4 hours. Methanolic ammonia (10 M, 5 mL) was added and reaction mixture was stirred for 24 hours. Volatiles were evaporated, semi-solid residue was coevaporated with pyridine (2× 5 mL), dissolved in dry pyridine (5 mL), and TMSBr (1.32 mL, 10 mmol) was added. After stirring the reaction for 12 hours at ambient temperature pyridine was evaporated and HILIC FCC (SiO₂, water in ACN, 10-60 %) afforded **6** (105 mg).

HRMS ESI (C₁₁H₁₄O₇N₅NaP) calculated: 382.05231; found: 382.05240. ¹H NMR (401 MHz, D₂O) δ 7.85 (s, 1H), 6.39 (ddd, *J* = 19.4, 17.1, 6.0 Hz, 1H), 6.04 (td, *J* = 17.0, 1.4 Hz, 1H), 5.81 (d, *J* = 5.3, 1H), 4.80 - 4.60 (m, 1H), 4.62 - 4.48 (m, 1H), 4.28 (t, *J* = 4.7 Hz, 1H). ¹³C NMR (101 MHz, D₂O) δ 158.81, 153.74, 151.53, 138.34 (d, *J* = 4.9 Hz), 137.68, 128.56 (d, *J* = 170.8 Hz), 116.39, 84.47, 84.74 (d, *J* = 21.4 Hz), 73.50, 72.80. ³¹P NMR (162 MHz, D₂O) δ 12.86.

### Trisodium ((E)-2-((2R,3S,4R,5R)-5-(2-amino-6-oxo-1,6-dihydro-9H-purin-9-yl)-3,4-dihydroxytetrahydrofuran-2-yl)vinyl)phosphonic diphosphoric anhydride (7)

To a solution of **6** (60 mg, 0.17 mmol) and morpholine (0.104 mL, 1.19 mmol) in tBuOH-water mixture (5:4, 5 mL) was added one fifth of a DCC (280 mg, 1.36 mmol) solution in tBuOH (2 mL) and the reaction mixture was heated to 90 °C. Further portions of DCC were added every hour until TLC analysis (*i*PrOH : 30% NH₄OH : water = 14 : 1 : 2) showed complete conversion to appropriate morpholidate. Volatiles were evaporated and the sticky residue was treated with diethyl ether to form a solid, which was collected and washed with further portions of Et₂O. Crude morpholidate was co-evaporated with anhydrous DMF (3× 2 mL), dissolved in anhydrous DMF (2 mL) and mixed with a solution of tributylammonium pyrophosphate (279 mg, 0.51 mmol) and Bu₃N (0.283 mL, 1.19 mmol) in anhydrous DMF (2mL). This reaction mixture was stirred overnight at ambient temperature, evaporated to dryness and the product was purified on preparative HPLC (0.1 M TEAB to ACN) and subsequently resalted to sodium salt using Dowex 50 (Na⁺) to afford 7 (19 mg). HRMS negESI (C₁₁H₁₆O₁₃P₃) calculated: 517.9879; found: 517.9868; ¹H NMR (401 MHz, Deuterium Oxide) δ 7.99 (s, 1H), 6.73 (ddd, *J* = 21.7, = 17.2, = 5,9 Hz, 1H), 6.27 (ddd, *J* = 18.7, = 17.2, = 1,4 Hz, 1H), 5.93 (d, *J* = 5.4 Hz, 1H), 4.83 - 4.78 (m, 1H), 4.73 - 4.68 (m, 1H), 4.45 (t, *J* = 4.7 Hz, 1H); ¹³C NMR (101 MHz, , Deuterium Oxide) δ 159.67, 154.52, 152.30, 142.93 (d, *J* = 5,2 Hz), 138.62, 125.59 (d, *J* = 182,4 Hz), 117.18, 88.28, 85.20 (d, *J* = 23,7 Hz), 74.03, 73.51; ³¹P NMR (162 MHz, Deuterium Oxide) δ 6.75 (d, *J* = 22.6 Hz), -5.49 (d, *J* = 20.8 Hz), -19.61 (t, *J* = 20.8 Hz).

The synthesis of compound **15** from compound **8** was accomplished according to the synthetic scheme depicted below, and detailed in the following description.

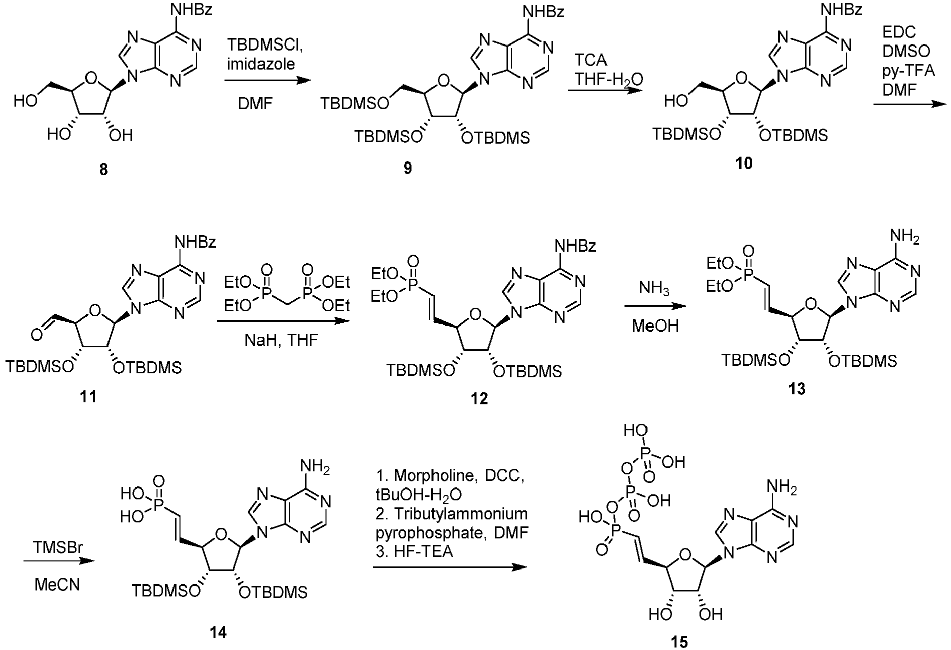

### N-(9-((2R,3R,4R,5R)-3,4-bis((tert-butyldimethylsilyl)oxy)-5-(((tert-butyldimethylsilyl)oxy)methyl)tetrahydrofuran-2-yl)-9H-purin-6-yl (benzamide (9)

To an ice cooled solution of commercial **8** (1.8 g, 4.8 mmol) and imidazole (2.6 g, 38.8 mmol) in dry DMF (20 mL) was added TBDMSCl (5.8 g, 38.6 mmol) in 4 portions over 10 minutes and the reaction mixture was stirred at ambient temperature overnight. The reaction mixture was diluted with ethyl acetate (100 mL) and washed with water (2× 50 mL) and brine (50 mL). Organic phase was dried over sodium sulfate and evaporated, yielding **9** (3.7 g, HPLC purity >95%), which was used in the next reaction without further purification.

### N-(9-((2R,3R,4R,5R)-3,4-bis((tert-butyldimethylsilyl)oxy)-5-(((tert-butyldimethylsilyl)oxy)methyl)tetrahydrofuran-2-yl)-9H-purin-6-vl)benzamide (10)

Trichloroacetic acid (18.5 g, 113 mmol) was dissolved in water (8.5 mL) and added dropwise into an ice cooled solution of crude **9** (3.7 g, 4.8 mmol) in THF (70 mL). The reaction mixture was stirred at 0 °C for 3 hours and then quenched by solid sodium carbonate. The suspension was diluted with dichloromethane (100 mL) and washed with water (50 mL). The aqueous phase was extracted two times with dichloromethane. The combined organic phases were dried over sodium sulfate and evaporated. Silica column chromatography (toluene:acetone, 7:3) afforded **10** (2.57 g,). NMR spectra were consistent with those described in literature (Lambrecht, Michael J.; Brichacek, Matthew; Hergenrother, Paul J. - J. Am. Chem. Soc., 2015, 137 (10), 3558-3564).

### Diethyl ((E)-2-((2R,3R,4R,5R)-5-(6-benzamido-9H-purin-9-yl)-3,4-bis((tert-butyldimethylsilyl)oxy)tetrahydrofuran-2-yl)vinyl)phosphonate (12)

Phosphonate **12** (0.510 g) was synthesized from compound **10** (0.5 g, 0.8 mmol) using the procedure described for the preparation of phosphonate **5**. HRMS ESI (C₃₄H₅₄O₇N₅PSi₂) calculated: 754.31916; found: 754.31890; ¹H NMR (401 MHz, DMSO-*d*₆) δ 11.23 (s, 1H), 8.77 (s, 1H), 8.73 (s, 1H), 8.05 (d, *J* = 7.0 Hz, 2H), 7.65 (t, *J* = 7.4 Hz, 1H), 7.55 (t, *J* = 7.5 Hz, 2H), 6.92 (ddd, *J* = 21.8, 17.0, 6.1 Hz, 1H), 6.18 (ddd, *J* = 20.1, 17.1, 1.4 Hz, 1H), 6.10 (d, *J* = 5.5 Hz, 1H), 5.09 (dd, *J* = 5.6, 4.4 Hz, 1H), 4.58 (ddd, *J* = 6.1, 3.0, 1.8 Hz, 1H), 4.54 (t, *J* = 4.0 Hz, 1H), 4.00 (m, 4H), 1.24 (t, *J* = 7.0 Hz, 6H), 0.94 (s, 9H), 0.75 (s, 9H), 0.16 (s, 3H), 0.13 (s, 3H), -0.05 (s, 3H), -0.30 (s, 3H); ¹³C NMR (101 MHz, DMSO-*d*₆) δ 165.75, 152.11, 151.61, 150.85, 147.77 (d, *J* = 5.7 Hz) 144.55, 133.47, 132.65, 128.67, 128.64, 126.42, 120.03 (d, *J* = 182.7 Hz), 88.58, 84.68 (d, *J* = 23.4 Hz), 75.18, 73.40, 61.49, 61.79 (m), 61.90 (m), 25.92, 25.66, 17.92, 17.70, 16.41 (d, *J* = 6.0 Hz), 16.35 (d, *J* = 6.0 Hz), -4.42, -4.55, -4.58, - 5.16; ³¹P NMR (162 MHz, DMSO-*d*₆) δ 19.57.

### Diethyl ((E)-2-((2R,3R,4R,5R)-5-(6-amino-9H-purin-9-yl)-3,4-bis((tert-butyldimethylsilyl)oxy)tetrahydrofuran-2-yl)vinyl)phosphonate (13)

A solution of **12** (500 mg, 0.8 mmol) in methanolic ammonia (5 mL) was stirred at ambient temperature overnight and evaporated. Flash chromatography (MeOH in DCM, 2-10%) afforded **13** (400 mg): HRMS ESI (C₂₇H₅₀O₆N₅NaPSi₂) calculated: 650.29294; found: 650.29305; ¹H NMR (401 MHz, DMSO-*d*₆) δ 8.40 (s, 1H), 8.11 (s, 1H), 7.34 (s, 2H), 6.90 (ddd, *J* = 21.8, 17.0, 6.0 Hz, 1H), 6.20 (ddd, *J* = 20.2, 17.1, 1.4 Hz, 1H), 5.94 (d, *J* = 5.8 Hz, 1H), 5.05 (dd, *J* = 5.9, 4.4 Hz, 1H), 4.53 (dtd, *J* = 6.1, 2.9, 1.4 Hz, 1H), 4.48 (dd, *J* = 4.4, 3.2 Hz, 1H), 3.98 (m, 4H), 1.23 (dt, *J* = 9.9, 7.0 Hz, 6H), 0.92 (s, 9H), 0.73 (s, 9H), 0.15 (s, 3H), 0.12 (s, 3H), -0.08 (s, 3H), -0.34 (s, 3H); ¹³C NMR (101 MHz, DMSO-*d*₆) δ 156.37, 152.55, 149.32, 148.01 (d, *J* = 5.5 Hz), 141.09, 119.81, 119.73 (d, *J* = 182.7 Hz), 88.33, 84.73 (d, *J* = 23.1 Hz), 75.29, 73.17, 61.47 (d, *J* = 5.1 Hz), 61.43 (d, *J* = 5.1 Hz), 25.91, 25.66, 17.94, 17.71, 16.39 (d, *J* = 5.6 Hz), 16.34 (d, *J* = 5.6 Hz), -4.45, -4.56, -4.62, -5.25; ³¹P NMR (162 MHz, DMSO-*d*₆) δ 19.73.

### ((E)-2-((2R.3R.4R.5R)-5-(6-amino-9H-purin-9-yl)-3.4-bis((tert-butyldimethylsilyl)oxy)tetrahydrofuran-2-yl)vinyl)phosphonic acid (14)

To a solution of **13** (237 mg, 0.4 mmol) and 2,6-lutidine (0.14 mL, 1.2 mmol) in anhydrous acetonitrile (10 mL) was added TMSBr (0.8 mL, 6 mmol) and reaction mixture was stirred at ambient temperature for 2 hours. Volatiles were evaporated and reverse phase flash chromatography (H₂O to ACN, 10 to 100%) afforded **14** (164 mg): HRMS negESI (C₂₃H₄₁O₆N₅PSi₂) calculated: 570.23385; found: 570.23366; ¹H NMR (401 MHz, DMSO-*d*₆) δ 8.38 (s, 1H), 8.15 (s, 1H), 7.41 (s, 2H), 6.70 (ddd, *J* = 20.8, 16.9, 6.7 Hz, 1H), 6.03 (t, *J* = 17.6 Hz, 1H), 5.95 (d, *J* = 5.8 Hz, 1H), 5.00-4.96 (m, 1H), 4.45 (dt, *J* = 5.4, 2.0 Hz, 1H), 4.39 - 4.30 (m, 1H), 0.92 (s, 9H), 0.73 (s, 9H), 0.13 (s, 3H), 0.11, (s, 3H), - 0.07 (s, 3H), -0.33 (s, 3H); ¹³C NMR (101 MHz, DMSO-*d*₆) δ 156.15, 152.59, 149.50, 142.49 (d, *J* = 5.0 Hz), 140.51, 125.54 (d, *J* = 179.3 Hz), 119.51, 87.83, 85.30 (d, *J* = 22.8 Hz), 75.54, 73.79, 25.93, 25.67, 17.94, 17.70, -4.42, -4.44, -4.56, -5.23; ³¹P NMR (162 MHz, DMSO-*d*₆) δ 14.20.

### Trisodium ((E)-2-((2R,3S,4R,5R)-5-(6-amino-9H-purin-9-yl)-3,4-dihydroxytetrahydrofuran-2-yl)vinyl)phosphonic diphosphoric anhydride (15)

To a solution of **14** (60 mg, 0.1 mmol) and morpholine (0.104 mL, 1.19 mmol) in tBuOH-water mixture (5:4, 5 mL) was added one fifth of a DCC (280 mg, 1.36 mmol) solution in tBuOH (2 mL) and the reaction mixture was heated to 90 °C. Further portions of DCC were added every hour until TLC analysis (iPrOH:30% NH₄OH:water 14:1:2) showed complete conversion. Volatiles were evaporated and the sticky residue was treated with diethyl ether to form a solid, which was collected and washed with further portions of EtzO. Crude morpholidate was co-evaporated with anhydrous DMF (3× 2 mL), dissolved in anhydrous DMF (2 mL) and mixed with a solution of tributylammonium pyrophosphate (279 mg, 0.51 mmol) and *n*Bu₃N (0.283 mL, 1.19 mmol) in anhydrous DMF (2mL). This reaction mixture was stirred overnight at ambient temperature and evaporated to dryness.

Crude protected compound was co-evaporated with dry pyridine (3× 2 mL) and dissolved in pyridine (2 mL) and TEA (1 mL). HF-TEA (0.276 mL, 1.7 mmol) was added dropwise and the reaction mixture was stirred at 40 °C for 2 hours. Volatiles were evaporated and product was purified on preparative HPLC (0.1 M TEAB to ACN) and subsequently resalted to sodium salt using Dowex 50 (Na⁺) to afford **15** (12 mg): HRMS negESI (C₁₁H₁₅O₁₂N₅P₃) calculated: 501.99355; found: 501.99270; ¹H NMR (401 MHz, Deuterium Oxide) δ 8.41 (s, 1H), 8.22 (s, 1H), 6.65 (ddd, *J* = 21.7, 17.2, 5.5 Hz, 1H), 6.22 (ddd, *J* = 18.7, 17.2, 1.5 Hz, 1H), 6.15 (d, *J* = 5.3 Hz, 1H), 4.96 - 4.62 (m, 2H), 4.43 (t, *J* = 4.9 Hz, 1H); ¹³C NMR (101 MHz, Deuterium Oxide) δ 155.53, 152.82, 148.96, 141.95 (d, *J* = 5.3 Hz) 139.79, 124.87 (d, *J* = 182.6 Hz), 118.69, 87.08, 84.29 (d, *J* = 23.2 Hz), 75.29, 73.17; ³¹P NMR (162 MHz, Deuterium Oxide) δ 6.52 (d, *J* = 22.5 Hz), -5.19 (d, *J* = 19.9 Hz), -19.54 (t, *J* = 21.3 Hz).

The synthesis of compound **20** from compound **16** was accomplished according to the synthetic scheme depicted below, and detailed in the following description.

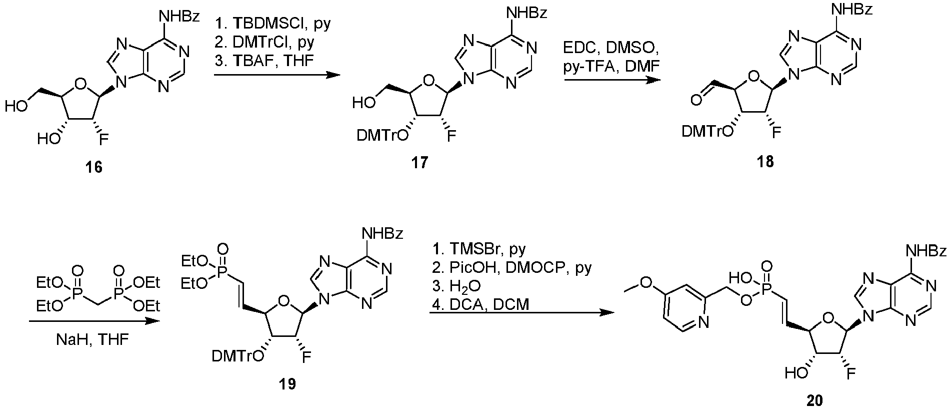

### N-(9-((2R,3R,4R,5R)-4-(bis(4-methoxyphenyl)(phenyl)methoxy)-3-fluoro-5-(hydroxymethyl)tetrahydrofuran-2-yl)-9H-purin-6-yl)benzamide (17)

A commercially available **16** (Carbosynth Ltd., 4 g, 10.7 mmol) was azeotroped with pyridine (2x 20 mL), dissolved in pyridine (50 mL) and TBDMSCl (3.23 g, 21.4 mmol) was added in one portion. Reaction mixture was stirred at ambient temperature for 12 hours, quenched with MeOH and all volatiles were evaporated. Residue was dissolved in ethyl acetate, washed with sat. aq. NaHCO₃ and brine, dried oved sodium sulfate and evaporated. Resulting sticky syrup was azeotroped with pyridine (2x 20 mL), dissolved in pyridine (50 mL) and DMTrCl (7.3 g, 21.4 mmol) was added in one portion. Reaction mixture was stirred at 55°C for 12 hours, after which all volatiles were evaporated. Residue was dissolved in ethyl acetate, washed with sat. aq. NaHCO₃ and brine, dried oved sodium sulfate and evaporated. Resulting crude protected nucleoside was dissolved in THF (50 mL), TBAF (1M in THF, 32 mL) was added in one portion and reaction mixture was stirred at ambient temperature for 12 hours. All volatiles were evaporated and subsequent FCC on silica gel (acetone in cyclohexane, 10-100%) afforded **17** (5.6 g). NMR spectra were consistent with literature reported data: WO2019/0434634)

### Diethyl ((E)-2-((2R,3R,4R,5R)-5-(6-benzamido-9H-purin-9-yl)-3-(bis(4-methoxyphenyl)(phenyl)methoxy)-4-fluorotetrahydrofuran-2-yl)vinyl)phosphonate (19)

Phosphonate **19** (2.7 g) was synthesized from compound **17** (4 g, 5.9 mmol) using the procedure described for the preparation of phosphonate **5:** HRMS ESI (C₄₃H₄₄FN₅O₈P) calculated: 808.8236; found: 808.8241; ¹H NMR (401 MHz, DMSO-*d*₆) δ 11.23 (s, 1H), 8.63 (s, 1H), 8.51 (s, 1H), 8.06 - 7.99 (m, 2H), 7.68 - 7.61 (m, 1H), 7.58 - 7.48 (m, 4H), 7.42 - 7.36 (m, 4H), 7.36 - 7.29 (m, 1H), 7.29 - 7.21 (m, 1H), 6.94 - 6.83 (m, 4H), 6.51 (ddd, *J* = 21.2, 16.9, 6.7 Hz, 1H), 6.35 (dd, *J* = 20.5, 1.6 Hz, 1H), 6.17 (dd, *J =* 20.1, 17.2 Hz, 1H), 4.88 (ddd, *J =* 19.4, 7.8, 4.3 Hz, 1H), 4.70 (t, *J* = 7.3 Hz, 1H), 4.16 (dd, *J* = 51.2, 3.2 Hz, 1H), 4.00 - 3.85 (m, 4H), 3.71 (s, 3H), 3.69 (s, 3H), 1.19 (t, *J* = 7.1 Hz, 6H), 1.17 (t, *J=* 7.1 Hz, 6H). ¹³C NMR (101 MHz, DMSO) δ 165.78, 158.64, 151.73, 151.18, 150.77, 146.71 (d, *J* = 5.5 Hz), 145.02, 144.07, 135.41, 135.37, 133.40, 132.72, 130.45, 130.40, 128.68, 128.08, 127.27, 125.95, 122.10 (d, *J* = 182.6 Hz), 113.48, 113.42, 91.91 (d, *J =* 188.2 Hz), 87.30 (d, *J =* 35.6 Hz), 87.07, 81.46 (d, *J =* 23.6 Hz), 75.01 (d, *J =* 15.0 Hz), 61.59, 61.57 (d, *J =* 5.7 Hz), 55.22, 16.38, 16.34 (d, *J* = 5.7 Hz). ³¹P NMR (162 MHz, DMSO) δ 18.76. ¹⁹F NMR (377 MHz, DMSO-*d*₆) δ -195.11 (dt, *J =* 51.0, 19.9 Hz).

### (4-Methoxypyridin-2-yl)methyl hydrogen ((E)-2-((2R,3R,4R,5R)-5-(6-benzamido-9H-purin-9-yl)-4-fluoro-3-hydroxytetrahydrofuran-2-yl)vinyl)phosphonate (20)

To a solution of **19** (200 mg, 0.25 mmol) in pyridine (2 mL) wasa added TMSBr (217 µL, 2 mmol) and the reaction mixture was stirred at ambient temperature 12 hour. Volatiles were evaporated, residue was dissolved in chloroform (50 mL), washed with 0.2M TEAB (3x 10 mL), dried over sodium sulfate and evaporated. This crude free phosphonate was combined with 4-Methoxypyridine N-oxide hydrate (180 mg, 1.25 mmol), azeotroped with pyridine (2x 2 mL) and dissolved in pyridine (2 mL). (4-Methoxy-pyridin-2-yl)-methanol (104 mg, 0.75 mmol) was added followed by DMOCP (231 mg, 1.25 mmol) and the reaction mixture was stirred at ambient temperature for 1 hour and subsequently quenched with 1 mL of water. This resulting mixture was heated to 60°C for 24 h and evaporated to dryness forming crude phosphonate monoester. To a solution of this crude intermediate in DCM (5 mL) was added water (45 µL, 2.5 mmol) and DCA (184 µL, 2.2 mmol). After stirring at ambient temperature for 2 hours pyridine (2 mL) was added to quench the reaction, volatiles were evaporated and product was purified by RP FCC (ACN in water 0-50%) to afford 87 mg of **20:** HRMS ESI (C₂₅H₂₅FN₆O₇P) calculated: 571.4816; found: 571.4808; ¹H NMR (401 MHz, DMSO-*d*₆) δ 11.24 (s, 1H), 8.73 (s, 1H), 8.63 (s, 1H), 8.26 (d, J = 5.7 Hz, 1H), 8.08 - 8.02 (m, 2H), 7.68 - 7.61 (m, 1H), 7.58 - 7.52 (m, 2H), 7.01 (d, J = 2.6 Hz, 1H), 6.81 (dd, J = 5.8, 2.6 Hz, 1H), 6.42 (ddd, J = 19.1, 16.9, 6.3 Hz, 1H), 6.38 (dd, J = 20.5, 1.6 Hz, 1H), 5.99 (t, J = 16.5 Hz, 1H), 5.57 (ddd, J = 52.4, 4.7, 1.8 Hz, 1H), 4.72 (d, J = 7.5 Hz, 2H), 4.65 (ddd, J = 22.1, 8.1, 4.7 Hz, 1H), 4.42 (bt, J = 7.2 Hz, 1H), 3.78 (s, 3H). ¹³C NMR (101 MHz, DMSO-*d*₆) δ 166.14, 165.81, 160.89 (d, J = 7.5 Hz), 152.01, 151.80, 150.74, 149.88, 143.59, 140.06, 133.52, 132.66, 128.71, 128.66, 128.56 (d, J = 171.3 Hz), 125.96, 108.81, 106.70,93.58 (d, J = 185.9 Hz), 85.59 (d, J = 34.7 Hz), 82.85 (d, J = 21.1 Hz), 72.80 (d, J = 16.0 Hz), 65.95 (d, J = 4.5 Hz), 55.39. ³¹P NMR (162 MHz, DMSO) δ 11.26. ¹⁹F NMR (377 MHz, DMSO-d6) δ -200.74 (dt, J = 52.6, 21.2 Hz).

The synthesis of compound **21** from compound **17** was accomplished according to the synthetic scheme depicted below, and detailed in the following description.

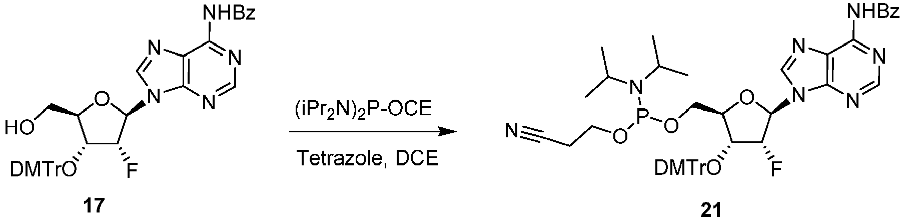

### ((2R,3R,4R,5R)-5-(6-benzamido-9H-purin-9-yl)-3-(bis(4-methoxyphenyl)(phenyl)methoxy)-4-fluorotetrahydrofuran-2-yl)methyl (2-cyanoethyl) diisopropylphosphoramidite (21)

To a solution of **17** (1 g, 1.5 mmol) in DCE (10 mL) under argon atmosphere was *via syringe* added 2-cyanoethyl *N*,*N*,*N*',*N*'-tetraisopropylphosphorodiamidite (0.94 mL, 3 mmol) followed by tetrazole (0.45M in ACN, 8.22 mL). Reactio mixture was stirred at ambient temperature for 30 minutes, diluted with DCM (150 mL), washed with sat. aq. NaHCO₃ (50 mL), dried over sodium sulfate and evaporated. Crude product was dissolved in DCM (2 mL) and applied to a preconditioned (10% acetone in cyclohexane, 1% TEA) silicagel column and eluted with a gradient of acetone in cyclohexane 10-50%. Product was freeze-dried from benzene to afford **21** (920 mg) as a mixture of diastereomers. ³¹P NMR (162 MHz, DMSO) δ 151.69, 151.66. ¹⁹F NMR (377 MHz, DMSO-*d*₆) δ -198.55 (dt, *J =* 51.2, 17.7 Hz), -198.96 (ddd, *J =* 51.1, 21.6, 15.6 Hz).

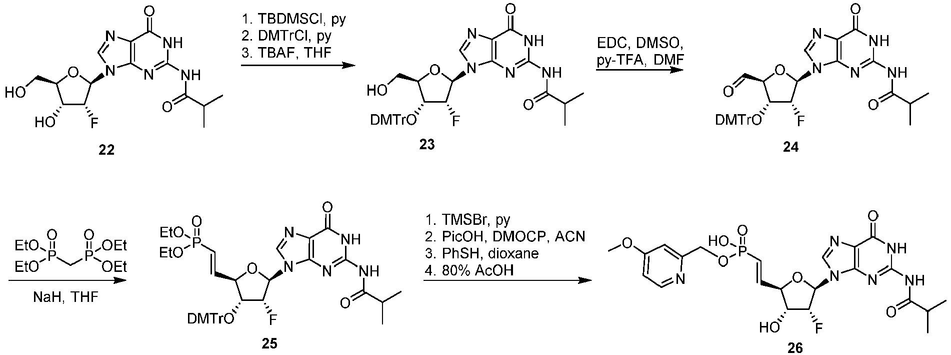

### N-(9-((2R,3R,4R,5R)-4-(Bis(4-methoxyphenyl)(phenyl)methoxy)-3-fluoro-5-(hydroxymethyl)tetrahydrofuran-2-yl)-6-oxo-6,9-dihydro-1H-purin-2-yl)isobutyramide (23)

Compound **23** (5.5 g) was synthesized from compound **17** (4 g, 11.3 mmol) using the procedure described for the preparation of **5:** HRMS ESI (C₃₅H₃₇FN₅O₇) calculated: 658.7069; found: 658.7073; ¹H NMR (401 MHz, DMSO-*d*₆) δ 12.14 (s, 1H), 11.70 (s, 1H), 8.15 (s, 1H), 7.49 - 7.44 (m, 2H), 7.36 - 7.28 (m, 6H), 7.27 - 7.21 (m, 1H), 6.90 - 6.83 (m, 4H), 6.09 (dd, *J=* 13.2, 3.7 Hz, 1H), 5.13 (t, *J* = 4.8 Hz, 1H), 4.31 (dt, *J=* 12.4, 4.4 Hz, 1H), 4.31 (dt, *J =* 51.8, 4.3 Hz, 1H), 3.73 - 3.70 (m, 1H), 3.71 (s, 3H), 3.70 (s, 3H), 3.54 (ddd, *J =* 12.8, 4.9, 2.4 Hz, 1H), 3.39 (ddd, *J =* 12.4, 4.4, 2.8 Hz, 1H), 2.79 (hept, *J* = 6.8 Hz, 1H), 1.14 (d, *J =* 6.8 Hz, 3H), 1.12 (d, *J =* 6.8 Hz, 3H). ¹³C NMR (101 MHz, DMSO) δ 180.46, 158.70, 154.89, 148.54, 148.30, 145.14, 136.75, 135.51, 135.47, 130.23, 128.15, 127.82, 127.21, 120.41, 113.52, 113.48, 91.82 (d, *J* = 192.0 Hz), 86.95, 85.08 (d, *J =* 31.8 Hz), 83.70, 70.94 (d, *J =* 14.1 Hz), 59.76, 55.27, 34.93, 19.09, 19.02. ¹⁹F NMR (377 MHz, DMSO-*d*₆) δ -202.82 (dtd, *J =* 52.1, 12.8, 3.8 Hz).

### Diethyl ((E)-2-((2R,3R,4R,5R)-3-(bis(4-methoxyphenyl)(phenyl)methoxy)-4-fluoro-5-(2-isobutyramido-6-oxo-1,6-dihydro-9H-purin-9-yl)tetrahydrofuran-2-yl)vinyl)phosphonate (25)

Compound **25** (800 mg) was synthesized from compound **23** (1.15 g, 1.75 mmol) using the procedure described for the preparation of **19:** HRMS ESI (C₄₀H₄₆FN₅O₉P) calculated: 790.8056; found: 790.8068; ¹H NMR (401 MHz, DMSO-*d*₆) δ 12.15 (s, 1H), 11.51 (s, 1H), 8.05 (s, 1H), 7.52 - 7.45 (m, 2H), 7.38 - 7.29 (m, 6H), 7.29 - 7.22 (m, 1H), 6.92 - 6.84 (m, 4H), 6.75 (ddd, *J =* 21.6, 17.0, 6.6 Hz, 1H), 6.16 (dd, *J =* 16.3, 3.3 Hz, 1H), 6.08 (ddd, *J =* 20.3, 16.9, 0.7 Hz, 1H), 4.41 - 4.33 (m, 2H), 4.18 (dt, *J=* 50.6, 3.3 Hz, 1H), 4.06 - 3.93 (m, 4H), 3.71 (s, 3H), 3.70 (s, 3H), 2.78 (hept, *J* = 6.9 Hz, 1H), 1.24 (t, *J =* 7.0 Hz, 3H), 1.21 (t, *J* = 7.0 Hz, 3H), 1.12 (d, *J* = 6.8 Hz, 3H), 1.11 (d, *J =* 6.8 Hz, 3H). ¹³C NMR (101 MHz, DMSO) δ 180.44, 158.75, 154.97, 148.15, 147.00 (d, *J* = 5.6 Hz), 144.94, 137.72, 135.30, 130.37, 130.34, 128.17, 127.93, 127.30, 121.95 (d, *J* = 182.8 Hz), 120.76, 113.55, 113.50, 91.03 (d, J = 192.6 Hz), 87.16, 85.87 (d, *J* = 33.0 Hz), 81.95 (d, *J* = 24.8 Hz), 74.61 (d, *J* = 14.8 Hz), 61.81 (d, *J* = 5.6 Hz), 55.28, 55.26, 34.97, 19.13, 18.98, 16.43 (d, *J* = 5.9 Hz), 16.40 (d, *J* = 5.9 Hz). ³¹P NMR (162 MHz, DMSO) δ 19.09. ¹⁹F NMR (377 MHz, DMSO-*d*₆) δ -201.14.

### (4-Methoxypyridin-2-yl)methyl hydrogen ((E)-2-((2R,3R,4R,5R)-4-fluoro-3-hydroxy-5-(2-isobutyramido-6-oxo-1,6-dihydro-9H-purin-9-yl)tetrahydrofuran-2-yl)vinyl)phosphonate (26)

Compound **26** (78 mg) was synthesized from compound **25** (200 mg, 0.2 mmol) using the procedure described for the preparation of **20:** HRMS ESI (C₂₂H₂₇FN₆O₈P) calculated: 553.4636; found: 553.4643; ¹H NMR (401 MHz, DMSO-*d*₆) δ 12.96 (bs, 1H), 12.24 (bs, 1H), 8.25 (d, *J* = 5.7 Hz, 1H), 8.14 (s, 1H), 6.98 (d, *J* = 2.6 Hz, 1H), 6.81 (dd, *J =* 5.7, 2.6 Hz, 1H), 6.80 (ddd, *J =* 19.7, 17.0, 6.6 Hz, 1H), 6.17 (dd, *J* = 17.7, 3.9 Hz, 1H), 6.04 (td, *J =* 17.0, 1.2 Hz, 1H), 5.48 (dt, *J =* 52.8, 4.2 Hz, 1H), 4.72 (d, *J =* 8.3 Hz, 2H), 4.78 - 4.67 (m, 1H), 4.45 - 4.39 (m, 1H), 3.74 (s, 3H), 2.98 (hept, *J* = 6.8 Hz, 1H), 1.00 (d, *J* = 6.8 Hz, 3H), 0.87 (d, *J* = 6.8 Hz, 3H). ¹³C NMR (101 MHz, DMSO-*d*₆) δ 181.38, 166.24, 160.82 (d, *J* = 6.7 Hz), 155.11, 149.86, 148.36, 148.18, 141.54 (d, *J* = 4.8 Hz), 140.10, 126.96 (d, *J* = 172.5 Hz), 121.57, 108.64, 106.83, 93.11 (d, *J* = 189.7 Hz), 86.95 (d, *J* = 33.2 Hz), 84.52 (d, *J* = 22.9 Hz), 72.11 (d, *J* = 15.3 Hz), 65.77 (d, *J* = 4.6 Hz), 55.39, 34.32, 19.08, 18.90. ³¹P NMR (162 MHz, DMSO) δ 11.76. ¹⁹F NMR (377 MHz, DMSO-*d*₆) δ -203.55.

The synthesis of compound **27** from compound **23** was accomplished according to the synthetic scheme depicted below, and detailed in the following description.

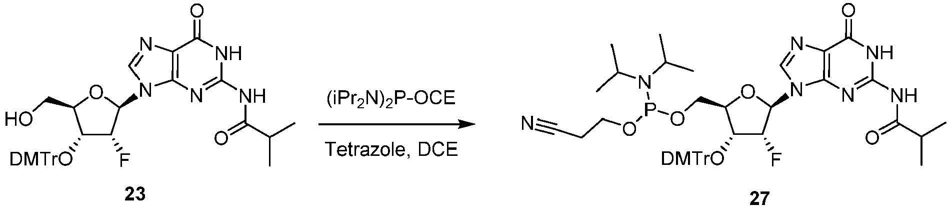

### ((2R,3R,4R,5R)-3-(bis(4-methoxyphenyl)(phenyl)methoxy)-4-fluoro-5-(2-isobutyramido-6-oxo-1,6-dihydro-9H-purin-9-yl)tetrahydrofuran-2-yl)methyl (2-cyanoethyl) diisopropylphosphoramidite (27)

Compound **27** (805 mg) was synthesized from compound **23** (1 g, 1.5 mmol) using the procedure described for the preparation of **21:** ³¹P NMR (162 MHz, DMSO) δ 152.19, 151.77. ¹⁹F NMR (377 MHz, DMSO-*d*₆) δ -199.94 (ddd, *J =* 51.2, 20.5, 14.6 Hz), -200.82 (dt, *J =* 51.7, 15.6 Hz).

The synthesis of compound **31** from compound **28** was accomplished according to the synthetic scheme depicted below, and detailed in the following description.

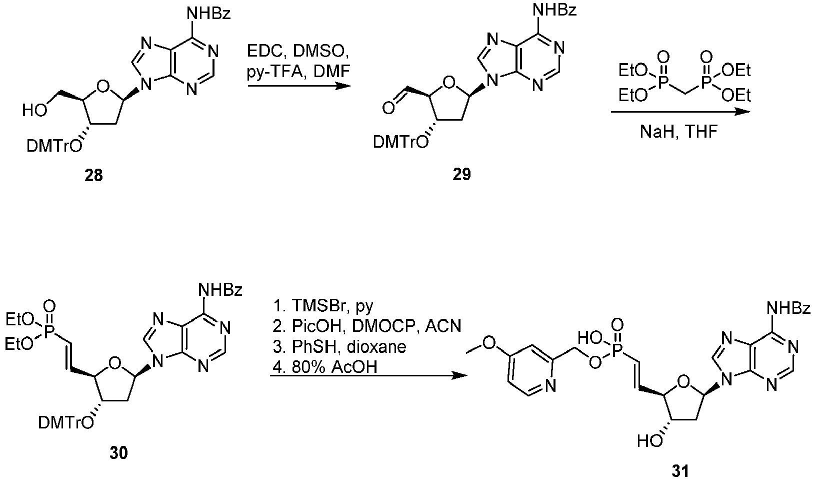

### Diethyl ((E)-2-((2R,3S,5R)-5-(6-benzamido-9H-purin-9-yl)-3-(bis(4-methoxyphenyl)(phenyl)methoxy)tetrahydrofuran-2-yl)vinyl)phosphonate (30)

Phosphonate **30** (3 g) was synthesized from compound **28** (Carbosynth Ltd., 3 g, 4.6 mmol) using the procedure described for the preparation of phosphonate **5:** HRMS ESI (C₄₃H₄₅N₅O₈P) calculated: 790.8332; found: 790.8333; ¹³C NMR (101 MHz, DMSO) δ 165.76, 158.54, 152.05, 151.68, 150.66, 148.06 (d, *J=* 5.5 Hz), 145.32, 143.90, 135.95, 135.91, 133.47, 132.65, 130.22, 128.65, 128.22, 128.05, 127.16, 126.22, 119.14 (d, *J =* 183.4 Hz, 113.63, 87.06, 85.30 (d, *J =* 22.6 Hz), 84.75, 77.11, 61.49, 61.43 (d, *J=* 5.0 Hz), 55.25, 36.23, 16.36, 16.30. ¹H NMR (401 MHz, DMSO-*d*₆) δ 11.19 (s, 1H), 8.68 (s, 1H), 8.59 (s, 1H), 8.06 - 8.01 (m, 2H), 7.67 - 7.61 (m, 1H), 7.58 - 7.47 (m, 4H), 7.42 - 7.32 (m, 6H), 7.29 - 7.23 (m, 1H), 6.96 - 6.90 (m, 4H), 6.55 (dd, *J=* 7.9, 6.2 Hz, 1H), 6.42 (ddd, *J =* 21.6, 17.0, 5.9 Hz, 1H), 5.74 (ddd, *J =* 20.4, 17.0, 1.4 Hz, 1H), 4.44 (dt, *J* = 5.7, 2.7 Hz, 1H), 4.40 - 4.35 (m, 1H), 3.93 - 3.83 (m, 4H), 3.74 (s, 3H), 3.73 (s, 3H), 2.69 - 2.59 (m, 1H), 2.13 - 2.02 (m, 1H), 1.20 - 1.14 (m, 6H). ³¹P NMR (162 MHz, DMSO) δ 19.44.

### (4-Methoxypyridin-2-yl)methyl hydrogen ((E)-2-((2R,3S,5R)-5-(6-benzamido-9H-purin-9-yl)-3-hydroxytetrahydrofuran-2-yl)vinyl)phosphonate (31)

Compound **31** (3 g) was synthesized from compound **30** (3 g, 4.6 mmol) using the procedure described for the preparation of **20:** HRMS ESI (C₂₅H₂₆N₆O₇P) calculated: 553.4912; found: 553.4919; ¹H NMR (401 MHz, DMSO-*d*₆) δ 11.25 (s, 2H), 8.72 (s, 1H), 8.61 (s, 1H), 8.28 - 8.23 (m, 1H), 8.08 - 8.02 (m, 2H), 7.68 - 7.61 (m, 1H), 7.58 - 7.51 (m, 2H), 6.99 (d, J = 2.4 Hz, 1H), 6.80 (dd, *J* = 5.7, 2.6 Hz, 1H), 6.49 (t, J = 6.6 Hz, 1H), 6.39 (ddd, *J* = 18.8, 16.9, 6.1 Hz, 1H), 5.84 (ddd, *J* = 17.1, 16.0, 1.4 Hz, 1H), 4.69 (d, *J* = 7.5 Hz, 2H), 4.43 (dt, *J* = 6.0, 4.1 Hz, 1H), 4.37 - 4.31 (m, 1H), 3.77 (s, 3H), 2.97 - 2.86 (m, 7H), 2.39 (ddd, *J* = 13.3, 6.6, 4.3 Hz, 1H), 1.12 (t, *J* = 7.3 Hz, 9H). ¹³C NMR (101 MHz, DMSO-*d*₆) δ 165.95, 165.84, 161.40 (d, *J* = 7.2 Hz), 152.17, 151.76, 150.57, 150.09, 143.34, 140.78 (d, *J* = 4.2 Hz), 133.58, 132.59, 128.66, 128.63, 127.61 (d, *J* = 171.3 Hz), 126.10, 108.58, 106.57, 87.46 (d, *J* = 20.3 Hz), 83.61, 74.12, 66.09 (d, *J* = 4.6 Hz), 55.31, 45.22, 38.34, 8.63. ³¹P NMR (162 MHz, DMSO) δ 11.30.

The synthesis of compound **53** from compounds **20** and **21** was accomplished according to the synthetic scheme depicted below, and detailed in the following description.

### (1R,6R,8R,9R,10R,13Z,15R,17R,18R)-8,17-bis(6-amino-9H-purin-9-yl)-9,18-difluoro-3,12-dihydroxy-2,4,7,11,16-pentaoxa-3λ⁵,12λ⁵-diphosphatricyclo[13.3.0.0⁶,¹⁰]octadec-13-ene-3,12-dione (53)

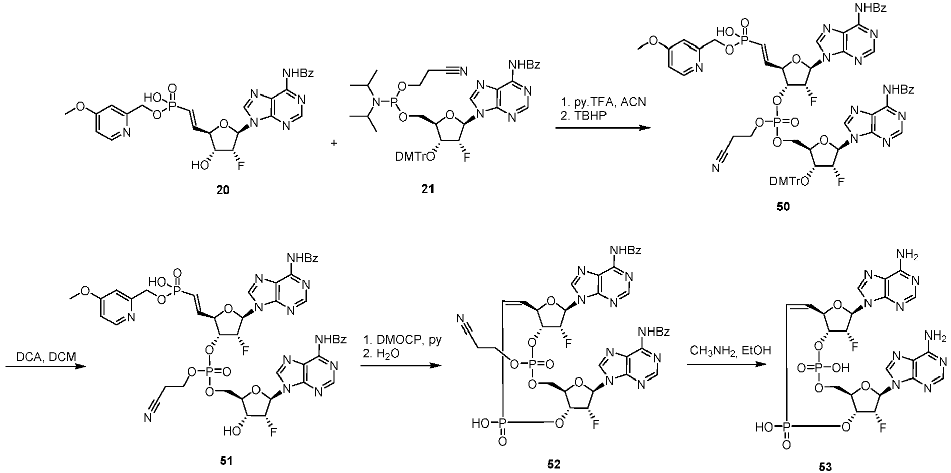

A mixture of **20** (57 mg, 0.1 mmol) and pyridinium trifluoroacetate (29 mg, 0.15 mmol) was codistilled with dry ACN (3x 3 mL), suspended in dry ACN (1 mL) and stirred overnight in a sealed vessel over activated molecular sieves. In a separate flask, **21** (96 mg, 0.11 mmol) was codistilled with dry ACN (3x 3 mL), dissolved in dry ACN (1 mL) and stirred overnight in a sealed vessel over activated molecular sieves. A solution of phosphoramidite was transferred *via syringe* to the flask with the suspension of **20** with py-TFA and the resulting solution was stirred for 1 hour at ambient temperature. TBHP (5.5M solution in decane, 55 µL, 0.3 mmol) was added and the reaction mixture was stirred for further 30 minutes. Reaction mixture was quenched with NaHSO₃ (39% in water, 54 µL, 0.27 mmol), filtered and evaporated to afford crude linear dimer **50,** which was used in the next reaction without further purification. To a solution of crude **50** in DCM (3 mL) was added water (18 µL, 1 mmol) and a solution of DCA (74 µL, 0.9 mmol) in DCM (3 mL) dropwise. After stirring the reaction mixture for 30 minutes, TES (1.5 mL) was added and the reaction mixture was stirred for further 90 minutes, after which it was quenched by the addition of pyridine (1.5 mL). Volatiles were evaporated and crude **51** was codistilled with dry pyridine (3x 3 mL) and used in the next reaction without further purification.

To a solution of crude **51** in pyridine (2 mL) was added DMOCP (65 mg, 0.35 mmol) and the reaction mixture was stirred at ambient temperature for 1 hour. Water (1 mL) was added and the reaction mixture was stirred at 55°C for 12 hours. All volatiles were evaporated to afford crude **52,** which was used in the next reaction without further purification.

CH₃NH₂ (33% in ethanol, 1 mL) was added to the crude **52** and the reaction mixture was stirred at ambient temperature for 3 hours. Volatiles were evaporated and the product was purified on a preparative HPLC (ACN in 0.1M TEAB, 0-30%). Appropriate fractions were pooled, evaporated, codistilled with water (3x 20 mL) and methanol (3x 20 mL), dissolved in water (10 mL) and slowly passed through a 10 mL column of Dowex 50 (Na⁺ cycle). Freeze-drying the eluent afforded the sodium salt of **53** (18 mg).

The following compounds were synthesized according to the protocols described above.

| **Compound** | **Structure** / **Name** | **Mass [M-H]⁻** |
|---|---|---|
| **53** | 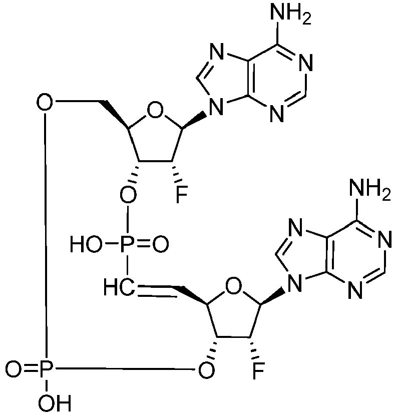 (1R,6R,8R,9R,10R,13E,15R,17R,18R)-8,17-bis(6-amino-9H-purin-9-yl)-9,18-difluoro-3,12-dihydroxy-2,4,7,11,16-pentaoxa-3λ⁵,12λ⁵-diphosphatricyclo[13.3.0.0⁶,¹⁰]octadec-13-ene-3,12-dione. | 657.1 |
| **53:** HPLC retention time (HILIC, min): 4.02. ¹H NMR (501 MHz, D₂O) δ 8.40 (s, 1H), 8.16 (s, 1H), 8.11 (s, 1H), 8.07 (s, 1H), 6.69 (ddd, *J* = 19.6, 17.0, 8.2 Hz, 1H), 6.39 (d, *J* = 16.5 Hz, 1H), 6.36 (dd, *J* = 19.4, 0.8 Hz, 1H), 6.24 (td, *J* = 17.0, 1.0 Hz, 1H), 5.61 (ddd, *J =* 52.0, 4.6, 0.8 Hz, 1H), 5.45 (dd, *J* = 51.6, 4.2 Hz, 1H), 5.01 (dddd, *J* = 23.0, 10.4, 9.2, 4.6 Hz, 1H), 4.80 (m, 2H), 4.45 (m, 2H), 4.04 (dd, *J* = 11.0, 2.5 Hz, 1H). ³¹P NMR (202 MHz, D₂O) δ 13.76, -0.60. ¹⁹F NMR (377 MHz, D₂O) δ -196.80, -197.47. | | |
| **54** | 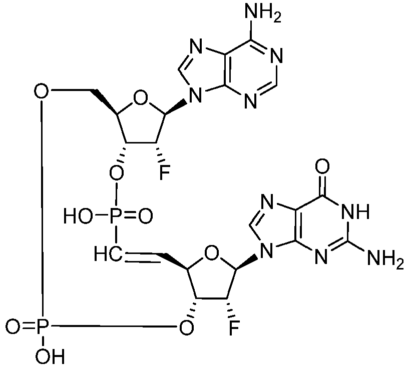 (1R,6R,8R,9R,10R,13E,15R,17R,18R)-17-(2-amino-6-oxo-6,9-dihydro-1H-purin-9-yl)-8-(6-amino-9H-purin-9-yl)-9,18-difluoro-3,12-dihydroxy-2,4,7,11,16-pentaoxa-3λ⁵,12λ⁵-diphosphatricyclo[13.3.0.0⁶,¹⁰]octadec-13-ene-3,12-dione | 673.1 |
| **54:** HPLC retention time (HILIC, min): 4.55. ¹H NMR (501 MHz, D₂O) δ 8.46 (s, 1H), 8.21 (s, 1H), 7.91 (s, 1H), 6.96 (m, 1H), 6.46 (d, *J* = 16.7 Hz, 1H), 6.30 (d, *J* = 21.1 Hz, 1H), 6.18 (t, *J* = 17.2 Hz, 1H), 5.53 (dd, *J* = 52.5, 4.6 Hz, 1H), 5.40 (dd, *J* = 51.9, 4.0 Hz, 1H), 5.06 - 5.15 (m, 2H), 4.42 - 4.70 (m, 2H), 4.06 (dd, *J* = 11.3, 2.4 Hz, 1H). ³¹P NMR (202 MHz, D₂O) δ 14.12, -1.15. ¹⁹F NMR (377 MHz, D₂O) δ -194.91, -197.60. | | |
| **55** | 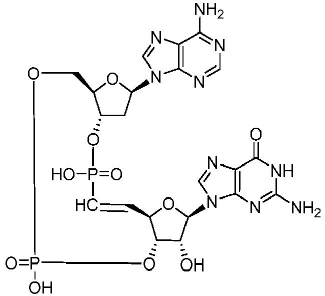 (1S,6R,8R,10S,13E,15R,17R,18R)-17-(2-amino-6-oxo-6,9-dihydro-1H-purin-9-yl)-8-(6-amino-9H-purin-9-yl)-3,12,18-trihydroxy-2,4,7,11,16-pentaoxa-3λ⁵,12λ⁵-diphosphatricyclo[13.3.0.0⁶,¹⁰]octadec-13-ene-3,12-dione | 653.1 |
| **55:** HPLC retention time (HILIC, min): 4.65. ¹H NMR (501 MHz, D₂O) δ 8.47 (s, 1H), 8.24 (s, 1H), 7.94 (s, 1H), 6.82 (ddd, *J =* 19.7, 17.1, 8.3 Hz, 1H), 6.48 (dd, *J =* 7.1, 2.9 Hz, 1H), 6.15 (td, *J*= 17.1, 0.6 Hz, 1H), 5.98 (d, *J =* 1.3 Hz, 1H), 4.99 (ddd, *J =* 10.0, 9.1, 5.5 Hz, 1H), 4.84 (m, 1H), 4.73 (dd, *J =* 5.5, 1.3 Hz, 1H), 4.67 (dd, *J =* 9.1, 8.3 Hz, 1H), 4.20 (m, 2H), 4.00 (m, 1H), 2.85 (ddd, *J* = 14.0, 7.6, 2.9 Hz, 1H), 2.80 (ddd, *J =* 14.0, 8.3, 7.1 Hz, 1H). ³¹P NMR (202 MHz, D₂O) δ 14.38, - 0.37. | | |
| **56** | 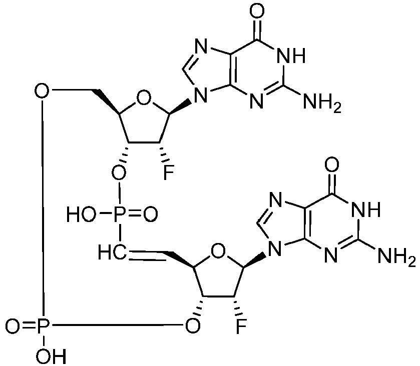 (1R,6R,8R,9R,10R,13E,15R,17R,18R)-8,17-bis(2-amino-6-oxo-6,9-dihydro-1H-purin-9-yl)-9,18-difluoro-3,12-dihydroxy-2,4,7,11,16-*p*entaoxa-3λ⁵,12λ⁵-diphosphatricyclo[13.3.0.0⁶,¹⁰]octadec-13-ene-3,12-dione. | 689.1 |
| **56:** HPLC retention time (HILIC, min): 4.78. | | |
| **57** | 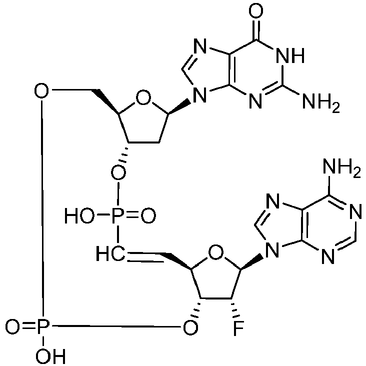 (1R,6R,8R,10S,13E,15R,17R,18R)-8-(2-amino-6-oxo-6,9-dihydro-1H-purin-9-yl)-17-(6-amino-9H-purin-9-yl)-18-fluoro-3,12-dihydroxy-2,4,7,11,16-pentaoxa-3λ⁵,12λ⁵-diphosphatricyclo[13.3.0.0⁶,¹⁰]octadec-13-ene-3,12-dione. | 655.1 |
| **57:** HPLC retention time (HILIC, min): 4.69. ¹H NMR (501 MHz, D₂O) δ 8.27 (s, 1H), 8.21 (s, 1H), 8.06 (s, 1H), 6.67 (ddd, *J* = 19.2, 17.0, 8.3 Hz, 1H), 6.41 (d, *J* = 20.0, 0.9 Hz, 1H), 6.27 (dd, *J* = 7.4, 3.9 Hz, 1H), 6.22 (td, *J* = 16.8, 0.8 Hz, 1H), 5.55 (ddd, *J* = 52.0, 4.8, 0.9 Hz, 1H), 5.10 (dddd, *J* = 22.4, 10.4, 9.2, 4.8 Hz, 1H), 4.86 (dddd, *J* = 10.1, 7.7, 7.4, 6.5 Hz, 1H), 4.80 (ddd, *J* = 9.2, 8.3, 0.8 Hz, 1H), 3.99 (m, 1H), 4.16 (m, 2H), 2.89 (ddd, *J =* 14.2, 2.5, 3.9 Hz, 1H), 2.74 (dt, *J =* 14.2, 7.4 Hz, 1H). ³¹P NMR (202 MHz, D₂O) δ 13.49, -0.37. ¹⁹F NMR (377 MHz, D₂O) δ -196.19, - 205.14. | | |
| **58** | 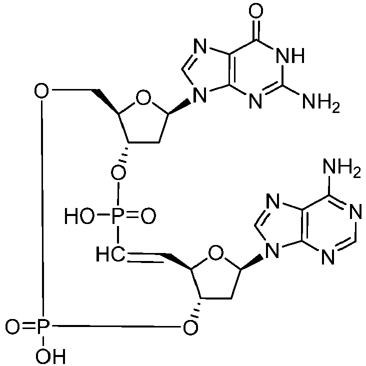 (1S,6R,8R,10S,13E,15R,17R)-8-(2-amino-6-oxo-6,9-dihydro-1H-purin-9-yl)-17-(6-amino-9H-purin-9-yl)-3,12-dihydroxy-2,4,7,11,16-pentaoxa-3λ⁵,12λ⁵-diphosphatricyclo[13.3.0.0⁶,¹⁰]octadec-13-ene-3,12-dione. | 637.1 |
| **58:** HPLC retention time (HILIC, min): 4.73. ¹H NMR (501 MHz, D₂O) δ 8.38 (s, 1H), 8.23 (s, 1H), 8.07 (s, 1H), 6.55 (ddd, *J* = 19.1, 17.0, 8.6 Hz, 1H), 6.47 (dd, *J* = 8.2, 3.3 Hz, 1H), 6.29 (dd, *J* = 7.3, 4.3 Hz, 1H), 6.13 (td, *J* = 17.0, 0.7 Hz, 1H), 4.99 (dq, *J* = 9.3, 5.2 Hz, 1H), 4.83 (m, 1H), 4.56 (t, *J* = 8.3 Hz, 1H), 4.10 - 4.16 (m, 2H), 3.95 (ddd, *J* = 11.4, 3.2, 1.9 Hz, 1H), 3.04 (ddd, *J* = 14.2, 8.4, 3.3 Hz, 1H), 2.88 (ddd, *J =* 14.1, 7.6, 4.3 Hz, 1H), 2.81 (dt, *J =* 14.2, 8.2 Hz, 1H), 2.71 (ddd, *J* = 14.1, 7.0, 7.0 Hz, 1H). ³¹P NMR (202 MHz, D₂O) δ 13.92, 0.17. | | |
| **59** | 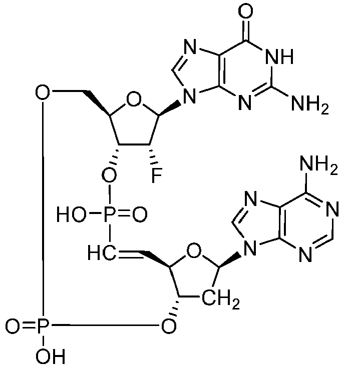 (1S,6R,8R,9R,10R,13E,15R,17R)-8-(2-amino-6-oxo-6,9-dihydro-1H-purin-9-yl)-17-(6-amino-9H-purin-9-yl)-9-fluoro-3,12-dihydroxy-2,4,7,11,16-pentaoxa-3λ⁵,12λ⁵-diphosphatricyclo[13.3.0.0⁶,¹⁰]octadec-13-ene-3,12-dione. | 655.1 |
| **59:** HPLC retention time (HILIC, min): 4.13. ¹H NMR (501 MHz, D₂O) δ 8.37 (s, 1H), 8.20 (s, 1H), 8.08 (s, 1H), 6.55 (ddd, *J* = 19.3, 16.9, 8.5 Hz, 1H), 6.48 (dd, *J=* 8.3, = 3.0 Hz, 1H), 6.27 (d, *J* = 17.9 Hz, 1H), 6.16 (ddd, *J* = 17.1, 16.9, 0.8 Hz, 1H), 5.44 (dd, *J=* 52.1, 4.4 Hz, 1H), 4.95 (p, *J* = 8.7 Hz, 1H), 4.79 (m, 1H), 4.54 (tt, *J* = 8.3, 0.8 Hz, 1H), 4.35 (m, 1H), 4.33 (m, 1H), 3.96 (m, 1H), 3.02 (ddd, *J* = 14.4, 8.3, 3.0 Hz, 1H), 2.81 (dt, *J* = 14.4, 8.5 Hz, 1H). ³¹P NMR (162 MHz, D₂O) δ 13.93, -0.21. ¹⁹F NMR (470.4 MHz, D₂O) δ -196.99. | | |
| **60** | 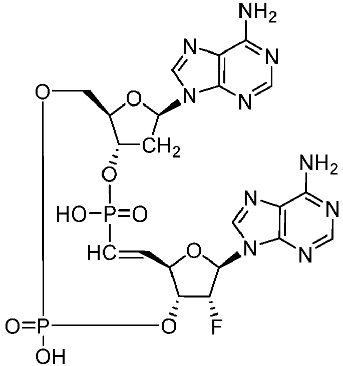 (1R,6R,8R,10S,13E,15R,17R,18R)-8,17-bis(6-amino-9H-purin-9-yl)-18-fluoro-3,12-dihydroxy-2,4,7,11,16-pentaoxa-3λ⁵,12λ⁵-diphosphatricyclo[13.3.0.0⁶,¹⁰]octadec-13-ene-3,12-dione. | 639.1 |
| **60:** HPLC retention time (HILIC, min): 4.11. ¹H NMR (501 MHz, D₂O) δ 8.38 (s, 1H), 8.19 (s, 1H), 8.12 (s, 1H), 8.10 (s, 1H), 6.69 (ddd, *J=* 19.2, 17.0, 8.2 Hz, 1H), 6.40 (dd, *J=* 7.3, 2.7 Hz, 1H), 6.36 (dd, *J* = 19.2, 0.7 Hz, 1H), 6.21 (td, *J* = 16.8, 0.9 Hz, 1H), 5.60 (dd, *J=* 51.8, 4.6 Hz, 1H), 5.12 (dddd, *J* = 22.7, 10.3, 9.2, 4.6 Hz, 1H), 4.87 (m, 1H), 4.80 (m, 1H), 4.22 (m, 1H), 4.21 (m, 1H), 4.00 (m, 1H), 2.92 (ddd, *J =* 14.2, 7.7, 2.7 Hz, 1H), 2.82 (ddd, *J=* 14.2, 8.4, 7.3 Hz, 1H). ³¹P NMR (162 MHz, D₂O) δ 13.65, -0.37. ¹⁹F NMR (470.4 MHz, D₂O) δ -196.86. | | |
| **61** | 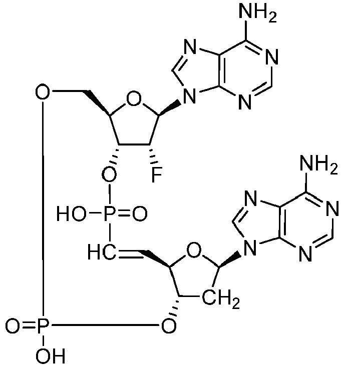 (1S,6R,8R,9R,10R,13E,15R,17R)-8,17-bis(6-amino-9H-purin-9-yl)-9-fluoro-3,12-dihydroxy-2,4,7,11,16-pentaoxa-3λ⁵,12^{λ}5-diphosphatricyclo[13.3.0.0⁶,¹⁰]octadec-13-ene-3,12-dione. | 639.1 |
| **61:** HPLC retention time (HILIC, min): 4.08. ¹H NMR (501 MHz, D₂O) δ 8.42 (s, 1H), 8.27 (s, 1H), 8.13 (s, 1H), 8.09 (s, 1H), 6.57 (ddd, *J =* 19.5, 17.0, 8.5 Hz, 1H), 6.41 (dd, *J =* 8.2, 2.8 Hz, 1H), 6.40 (d, *J* = 16.6 Hz, 1H), 6.16 (ddd, *J=* 19.5, 17.0, 0.8 Hz, 1H), 5.41 (dd, *J =* 51.8, 4.2 Hz, 1H), 4.99 (p, *J* = 8.7 Hz, 1H), 4.76 (m, 1H), 4.56 (td, *J* = 8.4, 0.8 Hz, 1H), 4.42 (m, 1H), 4.41 (m, 1H), 4.00 (m, 1H), 3.07 (ddd, *J* = 14.2, 8.2, 2.8 Hz, 1H), 2.82 (ddd, *J=* 14.2, 8.8, 8.2 Hz, 1H). ³¹P NMR (162 MHz, D₂O) δ 14.10, -0.12. ¹⁹F NMR (470.4 MHz, D₂O) δ -197.41. | | |

### Enzymatic Preparation of 3'3' CDNs

2-Aminoadenosine-5'-triphosphate (cat.# N-1001) and 2'-Amino-2'-deoxyadenosine-5'-Triphosphate (cat.# N-1046) were purchased from TriLink Biotechnologies (San Diego, USA). 2'-Deoxy-2-fluoroadenosine 5'-triphosphate (cat.# 107-02) was obtained from Metkinen Chemistry (Kuopio, Finland). 6-Methylthio-ITP (cat.# NU-1131S) was from Jena Bioscience (Jena, Germany).

1 µmol of the appropriate nucleoside triphosphate and 1 pmol trisodium ((*E*)-2-((2*R*,3*S*,4*R*,5*R*)-5-(2-amino-6-oxo-1,6-dihydro-9*H*-purin-9-yl)-3,4-dihydroxytetrahydrofuran-2-yl)vinyl)phosphonic diphosphoric anhydride (**7**) as described above were dissolved in 500 µl 20 mM Tris-HCl buffer containing pH 8.0, 20 mM MgCl₂, 5 µM mouse or human cGAS, and 0.1 mg/ml herring testes DNA (Sigma Aldrich, Prague, Czech Republic) and incubated at 37°C overnight on a shaker. The reactions resulted in mixture of 2'3' and 3'3' CDNs which were spun 25,000 g for 20 minutes and supernatants were passed through 3,000 Da filter concentrator (cat. # 88512, ThermoFisher, Waltham, USA). Triethyl ammonium bicarbonate buffer (TEAB, cat. # T7408, Sigma Aldrich, Czech Republic) was added to the flow through fractions to 0.1 M final concentration. The samples were then purified on semipreparative C18 column (Luna 5 µm C18 250x10 mm) using 50 min gradient of 0-10% ACN in 0.1 M TEAB (3 mL/min). TEAB was removed from the collected fractions by 3 cycles of evaporation/dissolving in 50% methanol and evaporates were dissolved in endotoxin free water (cat. # TMS-011-A, Merck Millipore, Prague, Czech Republic). Analytical HPLC was performed as described above.

### Enzymatic Preparation of 68

2 pmol of **15** as described above was dissolved in 500 µl 50 mM HEPES K buffer containing pH 8.0, 10 mM MgCl₂, 100 mM NaCl, 1mM DTT, 20µM BMB171 Diadenylate Cyclase and incubated at 50°C overnight in a shaker. The reaction mixture was spun at 25,000 g for 20 minutes and supernatants were passed through 3,000 Da filter concentrator (cat. # 88512, ThermoFisher, Waltham, USA). Triethyl ammonium bicarbonate buffer (TEAB, cat. # T7408, Sigma Aldrich, Czech Republic) was added to the flow through fractions to 0.1 M final concentration. The samples were then purified on semipreparative C18 column (Luna 5 µm C18 250x10 mm) using 50 min gradient of 0-10% ACN in 0.1M TEAB (3 mL/min). TEAB was removed from the collected fractions by 3 cycles of evaporation/dissolving in 50% methanol and evaporates were dissolved in endotoxin free water (cat. # TMS-011-A, Merck Millipore, Prague, Czech Republic). Analytical HPLC was performed as described above.

| **Compound** | **Structure / Name** | **Mass [M-H]⁻** |
|---|---|---|
| **65** | 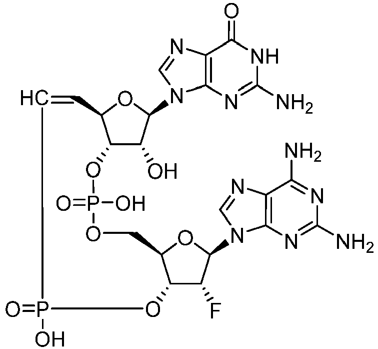 (1S,6R,8R,9R,10R,13Z,15R,17R,18R)-17-(2-amino-6-oxo-6,9-dihydro-1H-purin-9-yl)-8-(2,6-diamino-9H-purin-9-yl)-9-fluoro-3,12,18-trihydroxy-2,4,7,11,16-pentaoxa-3λ⁵,12λ⁵-diphosphatricyclo[13.3.0.0⁶,¹⁰]octadec-13-ene-3,12-dione. | 686.1 |
| **65:** HPLC retention time (HILIC, min): 4.08. ¹H NMR (501 MHz, Deuterium Oxide) δ 8.15 (s, 1H), 7.91 (s, 1H), 6.97 (ddd, *J* = 20.3, 17.2, 8.0 Hz, 1H), 6.30 (d, *J* = 16.6 Hz, 1H), 6.15 (td, *J* = 7.3, 0.9 Hz, 1H), 6.00 (d, *J* = 1.1 Hz, 1H), 5.33 (dd, *J* = 51.9, 4.1 Hz, 1H), 4.94 (ddd, *J* = 10.2, 9.1, 5.4 Hz, 1H), 4.75 (m, 1H), 4.70 (m, 1H), 4.68 (m, 1H), 4.38 - 4.41 (m, 2H), 4.02 (m, 1H). ³¹P NMR (202 MHz, Deuterium Oxide) δ 14.44, -1.05. ¹⁹F NMR (377 MHz, DMSO-*d*₆) δ -197.45. | | |
| **66** | 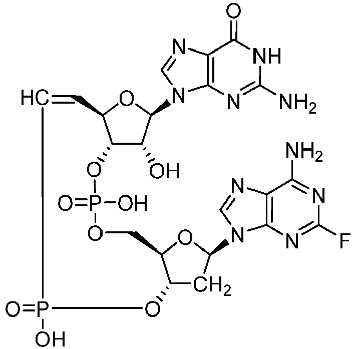 (1S,6R,8R,10S,13Z,15R,17R,18R)-8-(6-amino-2-fluoro-9H-purin-9-yl)-17-(2-amino-6-oxo-6,9-dihydro-1H-purin-9-yl)-3,12,18-trihydroxy-2,4,7,11,16-pentaoxa-3λ⁵,12λ⁵-diphosphatricyclo[13.3.0.0⁶,¹⁰]octadec-13-ene-3,12-dione. | 671.1 |
| **66:** HPLC retention time (HILIC, min): 3.99. ¹H NMR (501 MHz, Deuterium Oxide) δ 8.39 (s, 1H), 7.94 (s, 1H), 6.82 (ddd, *J =* 18.7, 17.2, 8.4 Hz, 1H), 6.37 (dd, *J* = 8.9, 2.7 Hz, 1H), 6.15 (t, *J* = 17.1 Hz, 1H), 5.98 (d, *J =* 1.3 Hz, 1H), 4.98 (m, 1H), 4.81 (m, 1H), 4.74 (dd, *J =* 5.5, 1.4 Hz, 1H), 4.67 (t, *J* = 8.8 Hz, 1H), 4.16 - 4.21 (m, 2H), 3.99 (m, 1H), 2.84 (ddd, *J* = 13.9, 7.6, 2.7 Hz, 1H), 2.78 (m, 1H). ³¹P NMR (202 MHz, Deuterium Oxide) δ 13.89, -0.79. | | |
| **67** | 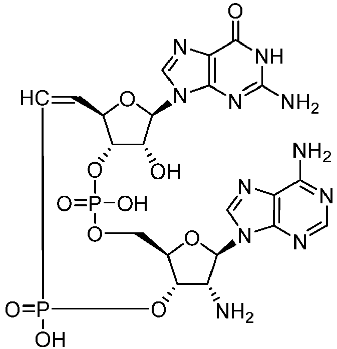 (1S,6R,8R,9R,10S,13Z,15R,17R,18R)-9-amino-17-(2-amino-6-oxo-6,9-dihydro-1H-purin-9-yl)-8-(6-amino-9H-purin-9-yl)-3,12,18-trihydroxy-2,4,7,11,16-pentaoxa-3λ⁵,12λ⁵-diphosphatricyclo[13.3.0.0⁶,¹⁰]octadec-13-ene-3,12-dione. | 668.1 |
| **67:** HPLC retention time (HILIC, min): 4.45. ¹H NMR (501 MHz, Deuterium Oxide) δ 8.46 and 8.22 (s, 1H), 7.88 (s, 1H), 6.82 (m, 1H), 6.13 (t, *J* = 17.2 Hz, 1H), 6.02 (d, *J* = 3.2 Hz, 1H), 5.95 (s, 1H), 4.95 (m, 1H), 4.63 - 4.73 (m, 3H), 4.36 (m, 1H), 4.29 (m, 1H), 4.00 - 4.04 (m, 2H). ³¹P NMR (202 MHz, Deuterium Oxide) δ 14.23, -0.76. | | |
| **68** | 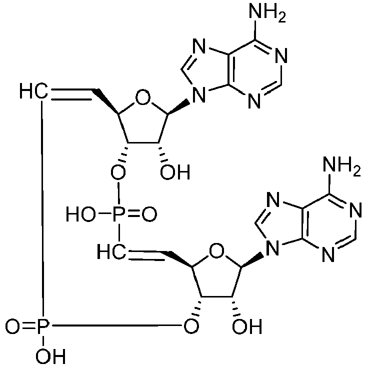 (1S,4Z,6R,8R,9R,10S,13E,15R,17R,18R)-8,17-bis(6-amino-9H-purin-9-yl)-3,9,12,18-tetrahydroxy-2,7,11,16-tetraoxa-3λ⁵,12λ⁵-diphosphatricyclo[13.3.0.0⁶,¹⁰]octadeca-4,13-diene-3,12-dione. | 649.1 |
| **68:** HPLC retention time (HILIC, min): 4.13. | | |
| **69** | 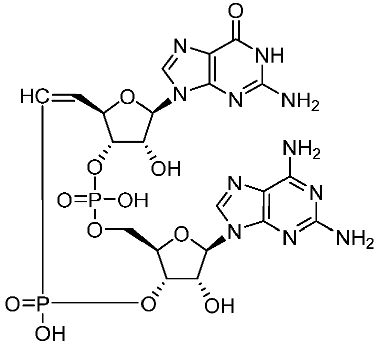 (1S,6R,8R,9R,10S,15R,17R,18R)-17-(2-amino-6-oxo-6,9-dihydro-1H-purin-9-yl)-8-(2,6-diamino-9H-purin-9-yl)-3,9,12,18-tetrahydroxy-2,4,7,11,16-pentaoxa-3λ⁵,12λ⁵-diphosphatricyclo[13.3.0.0⁶,¹⁰]octadec-13-ene-3,12-dione. | 684.1 |
| **69:** HPLC retention time (HILIC, min): 4.21. ¹H NMR (501 MHz, Deuterium Oxide) δ 7.93 (s, 1H), 7.87 (s, 1H), 6.97 (m, 1H), 6.15 (t, *J =* 17.3 Hz, 1H), 6.02 (s, 1H), 6.01 (s, 1H), 4.96 (m, 1H), 4.62 - 4.71 (m, 3H), 4.54 (d, *J* = 4.8 Hz, 1H), 4.31 - 4.38 (m, 1H), 4.01 (m, 1H). ¹³C NMR (126 MHz, D₂O) δ 160.90, 156.87, 151.87, 151.14, 146.28, 139.03, 137.58, 125.64 (d, *J* = 172.2 Hz), 117.55, 113.97, 91.33, 89.59, 82.45 (dd, *J =* 25.1, 6.7 Hz), 80.38 (m), 77.43 (d, *J* = 4.5 Hz), 74.76, 74.70, 70.20 (d, *J* = 3.4 Hz), 62.52 (d, *J* = 4.6 Hz). ³¹P NMR (162 MHz, Deuterium Oxide) δ 14.24, -0.94. | | |
| **70** | 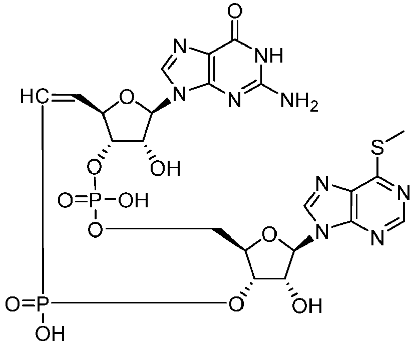 (1S,6R,8R,9R,10S,15R,17R,18R)-17-(2-amino-6-oxo-6,9-dihydro-1H-purin-9-yl)-3,9,12,18-tetrahydroxy-8-[6-(methylsulfanyl)-9H-purin-9-yl]-2,4,7,11,16-pentaoxa-3λ⁵,12λ⁵-diphosphatricyclo[13.3.0.0⁶,¹⁰]octadec-13-ene-3,12-dione. | 700.1 |
| **70:** HPLC retention time (HILIC, min): 4.05. ¹H NMR (501 MHz, Deuterium Oxide) δ 8.74 (s, 1H), 8.69 (s, 1H), 7.90 (s, 1H), 7.00 (ddd, *J* = 19.9, 17.3, 7.9 Hz, 1H), 6.27 (s, 1H), 6.13 (t, *J* = 17.4 Hz, 1H), 6.00 (s, 1H), 4.94 (td, *J* = 9.0, 5.0 Hz, 1H), 4.64 - 4.71 (m, 4H), 4.39 - 4.43 (m, 2H), 4.03 (bd, 1H, *J* = 11.2 Hz), 2.71 (s, 3H). ¹³C NMR (126 MHz, D₂O) δ 162.97, 154.44, 152.28, 151.72, 147.28, 146.75, 142.81, 139.22, 131.63, 125.00 (d, *J* = 177.0 Hz), 117.63, 90.45, 90.45, 82.56 (m), 80.47 (m), 77.46 (d, *J* = 3.7 Hz), 74.79, 74.63, 70.14 (d, *J =* 3.0 Hz), 62.47 (d, *J =* 4.4 Hz), 12.49. ³¹P NMR (202 MHz, Deuterium Oxide) δ 14.53, -1.08. | | |

### Example 1. Biological evaluation

A cyclic dinucleotide was determined to be a STING agonist: (A) if it demonstrated binding to the AQ allelic form of human STING protein with thermal shift of > 0.5 °C in the STING Differential Scanning Fluorimetry Assay (DSF), and (B) if it demonstrated STING activation through IRF-3 dependent expression of firefly luciferase reporter with EC₅₀ <100 µmol.l⁻¹.

### ISRE reporter plasmid (pGL64.27-4xISRE)

Two complementary oligonucleotides of the sequence AAAGATCTTGGAAAGTGAAACCTTGGAAAACGAAACTGGACAAAGGGAAACTGCAGAA ACTGAAACAAAGCTTAA (SEQ ID NO: 1) and TTAAGCTTTGTTTCAGTTTCTGCAGTTTCCCTTTGTCCAGTTTCGTTTTCCAAGGTTTCACT TTCCAAGATCTTT (SEQ ID NO:2) containing four interferon-sensitive response elements (ISRE) were synthesized by Sigma Aldrich (Czech Republic, Prague). The oligonucleotides were mixed in equal molar amounts, hybridized, and cleaved by restriction endonucleases HindIII (cat. #. R0104S, NEB, Ipswich, USA) and BglII (cat. # R0144S, NEB, Ipswich, USA). Ultimately, they were ligated into plasmid pGL4.27 (cat. # E6651, Promega, Madison, USA) linearized with the same enzymes. As result the sequence with four ISRE sites was placed upstream of the minimum promoter of firefly luciferase reporter gene.

### 293T wtSTING-FL reporter cells

293T cells (cat. # CRL-3216, ATCC, Manassas, USA) were seeded a day before transfection at density 125,000 cells per cm² onto poly-D-lysine (cat. # P6407, Sigma Aldrich, Czech Republic) coated six well plates in antibiotic free DMEM with high glucose (cat. # D5796, Sigma Aldrich, Czech Republic) supplemented with 10% heat inactivated FBS (cat. # S1520, Biowest, Riverside, USA). On the day of transfection, 2.5 µg of the plasmid pUNO1-hSTING-WT (cat. # puno1-hstingwt, InvivoGen, San Diego, USA,) encoding human wild type STING (WT STING) was diluted in 125 µL OptiMEM medium (cat. # 31985062, ThermoFisher, Waltham, USA) and mixed with 125 µL of the same medium containing 12.5 µL of Lipofectamine 2000 (cat. # 11668019, ThermoFisher, Waltham, USA). After 5 minutes incubation at room temperature (RT), 250 µL of the mixture was added dropwise to the cells in one well. Cells were incubated 36 hours at 37 °C with 5% CO₂, and then detached with 0.05% Trypsin and 0.22 g/L EDTA (both cat. # L0941, Biowest, Riverside, USA).

Transfected cells were seeded onto poly-D-lysine coated six well plates at density 50,000 cells per 1 cm² in DMEM medium with high glucose containing 10% heat inactivated FBS, 30µg/mL blasticidin (cat. # ant-bl-05, InvivoGen, San Diego, USA), 0.06 mg/ml Penicillin G and 0.1mg/ml Streptomycin Sulfate (both cat. #. L0018, Biowest, Riverside, USA). The medium was replenished every 3 - 4 days until visible colonies of cells resistant to blasticidin were formed.

Blasticidin resistant cells stably expressing WT STING were further transfected with pGL64.27-4xISRE plasmid following the same procedure as described above. The transfected cells were selected for the resistance to 300 µg/mL hygromycin (cat. #. 10687010, ThermoFisher, Waltham, USA) in DMEM with high glucose containing 10% heat inactivated FBS, 30µg/mL blasticidin, 0.06 mg/ml Penicillin G and 0.1 mg/ml Streptomycin Sulfate. Homogeneous culture of stably double transfected cells was prepared by limiting dilution of cells in 96 well plates and wells with cells were selected that originated from a single cell. These cells were expanded, and expression of WT STING was confirmed by western blot using monoclonal mouse anti STING antibodies (cat. #. MAB7169, 1:1000 dilution; 2° antibody cat. #. HAF007, 1:2000 dilution, both from R&D Systems, Minneapolis, USA), and by induction of firefly luciferase expression in the presence of 50 µM STING agonist 2'3' cGAMP (cat. # tlrl-nacga23, InvivoGen, San Diego, USA). Genomic DNA from the transfected cells was amplified with primers pUNO1_Seq_F (TGCTTGCTCAACTCTACGTC) (SEQ ID NO:3) and pUNO1_Seq_R (GTGGTTTGTCCAAACTCATC) (SEQ ID NO:4) that were complementary to pUNO1 plasmid and the presence of WT STING gene in the transfected cells was confirmed by DNA sequencing.

### Digitonin Assay using 293T wtSTING-FL reporter cells

293T wtSTING-FL cells were seeded at density of 250,000 cells per cm² onto 96 well poly-D-lysine coated plates in 100 µl DMEM with high glucose supplemented with 10% heat inactivated FBS. The medium was removed next day and three fold serial dilutions of compounds in Digitonin buffer containing 50 mmol.l⁻¹ HEPES (cat. # H3375, Sigma Aldrich, Czech Republic) pH 7.0, 100 mmol.l⁻¹ KCl, 3 mmol.l⁻¹ MgCl₂, 0.1 mmol.l⁻¹ DTT (cat. # D0632, Sigma Aldrich, Czech Republic), 85 mmol.l⁻¹ Sucrose (cat. # S7903, Sigma Aldrich, Czech Republic), 0.2% BSA (cat. # A2153, Sigma Aldrich, Czech Republic), 1 mmol.l⁻¹ ATP (cat. # A1852, Sigma Aldrich, Czech Republic), 0.1 mmol.l⁻¹ GTP (cat. # G8877, Sigma Aldrich, Czech Republic), and 10 µg/mL Digitonin A (cat. # D141, Sigma Aldrich, Czech Republic) were added to the cells. The buffer was removed after 30 minutes incubation at 37°C with 5% CO₂, the cells were washed once with 100 µl of cultivation medium, and 100 µl of medium was added to each well. The plates with cells were incubated for 5 hours at 37°C with 5% CO₂, 50µl of the medium was removed and 30 µl of ONE-Glo^{™} Luciferase Assay System reagent (cat. # E6120, Promega, Madison, USA) was added to each well. Luminescence was read on Synergy H1 (Biotek, Winooski, USA). GrafPad Prism (La Jolla, USA) was used to calculate the 50% effective concentration (EC₅₀) from an 8-point dose-response curve. Control compounds 3'3'-c-di-GMP (cat. # tlrl-nacdg), 3'3'-c-di-AMP (cat. # tlrl-nacda), 3'3'-cGAMP (cat. # tlrl-nacga), 2'3'-cGAMP (cat. # tlrl-nacga23), and 2'2'-cGAMP (cat. # tlrl-nacga22) were purchased from Invivogen (San Diego, USA).

### WT STING and AQ STING proteins

Both WT and AQ human STING (G230A-R293Q) cDNA were amplified by the use of PCR (Phusion^{®} High-Fidelity DNA Polymerase, cat. # M0530S, NEB, Ipswich, USA) using oligonucleotides hSTING140-BamH-For (GTGGGATCCGCCCCAGCTGAGATCTCTGCAG) (SEQ ID NO:5) and hSTING379-Not-Rev3 (TATGCGGCCGCCTATTACACAGTAACCTCTTCCTTTTC) (SEQ ID NO:6) from pUNO1-hSTING-WT (cat. # puno1-hstingwt, InvivoGen, San Diego, USA) and pUNO1-hSTING-HAQ plasmids (puno1-hsting-haq, InvivoGen, San Diego, USA). Purified PCR products were cleaved with restriction enzymes BamHI (cat. # R0136S, NEB, Ipswich, USA) and NotI (cat. # R0189S, NEB, Ipswich, USA) and cloned into the pSUMO vector linearized with the identical enzymes. Plasmid pSUMO was created by introducing 8-His-SUMO sequence between NdeI and BamHI sites of pHis-parallel2 plasmid (Clontech, Moutain View, USA). pSUMO-STING WT or pSUMO-STING AQ plasmids thus encoded truncated human WT STING or AQ STING (amino acid residues 140-343) with N-terminal 8xHis and SUMO tag.

The recombinant WT STING and AQ STING proteins were overexpressed in Rosetta-gami B (DE3) competent cells (cat. # 71136-3, Merck Millipore, Billerica, USA). Bacterial pellets were re-suspended in ice-cold lysis buffer containing 50 mmol.l⁻¹ TrisCl (cat. # T1503, Sigma Aldrich, Czech Republic) pH 8.0, 300 mmol.l⁻¹ NaCl, 3 mmol.l⁻¹ β-mercaptoethanol (cat. # M6250, Sigma Aldrich, Czech Republic), 10% glycerol (cat. # G5516, Sigma Aldrich, Czech Republic) and 20 mmol.l⁻¹ imidazole (cat. # I5513, Sigma Aldrich, Czech Republic) using Dounce homogenizer. DNase I (cat. # D5025, Sigma Aldrich, Czech Republic) and RNase A (cat. # R6513, Sigma Aldrich, Czech Republic) were added (final concentration 50 µg/ml) together with MgCl₂ (final concentration 5 mmol.l⁻¹) to the homogenate and bacteria were lysed using French Press G-M^{™} High-Pressure Cell Press Homogenizer (1500 psi, 3 cycles). Lysate was spun 30,000 g for 20 minutes and supernatant was gently stirred with Ni-NTA resin (cat. # 745400.25 Macherey-Nagel, Düren, Germany) for 30 minutes. The resin was poured into a chromatography column, washed with 50 ml buffer A (50 mmol.l⁻¹ TrisCl (pH 8.0), 800 mmol.l⁻¹ NaCl, 3 mmol.l⁻¹ β-mercaptol; 10% glycerol; 20 mmol.l⁻¹ imidazole) and 8-His-SUMO tagged STING proteins were eluted with 15 ml buffer A containing 300 mmol.l⁻¹ imidazole. The eluted proteins were cleaved with recombinant SUMO protease (80 µg/ml of protein solution, cat. # 12588018, ThermoFisher, Waltham, USA). The proteins were further purified by size exclusion chromatography using HiLoad 16/60 Superdex 75 (cat. # 28989333, GE Healthcare Bio-Sciences, Pittsburgh, USA) in 50 mmol.l⁻¹ Tris Cl buffer pH 7.4 containing 150 mmol.l⁻¹ NaCl, and 10% glycerol. Proteins were concentrated with Amicon^{®} Ultra-15 10 K device (cat. # UFC901008, Merck Millipore, Billerica, USA) and flash frozen in liquid N₂.
DNA sequence of 8-His-SUMO 
ATGTCGCATCACCATCATCATCACCACCATGGGATGTCGGACTCAGAAGTCAATCAAGA
AGCTAAGCCAGAGGTCAAGCCAGAAGTCAAGCCTGAGACTCACATCAATTTAAAGGTGT
CCGATGGATCTTCAGAGATCTTCTTCAAGATCAAAAAGACCACTCCTTTAAGAAGGCTGA
TGGAAGCGTTCGCTAAAAGACAGGGTAAGGAAATGGACTCCTTAAGATTCTTGTACGAC
GGTATTAGAATTCAAGCTGATCAGACCCCTGAAGATTTGGACATGGAGGATAACGATAT
TATTGAGGCTCACCGCGAACAGATTGGTGGATCC (SEQ ID NO:7).
Amino acid sequence of 8-His-SUMO 
MSHHHHHHHHGMSDSEVNQEAKPEVKPEVKPETHINLKVSDGSSEIFFKIKKTTPLRRLMEA
FAKRQGKEMDSLRFLYDGIRIQADQTPEDLDMEDNDIIEAHREQIGGS
(SEQ ID NO:8).
Amino acid sequence of truncated WT STING 
APAEISAVCEKGNFNVAHGLAWSYYIGYLRLILPELQARIRTYNQHYNNLLRGAVSQRLYILL
PLDCGVPDNLSMADPNIRFLDKLPQQTGDRAGIKDRVYSNSIYELLENGQRAGTCVLEYATP
LQTLFAMSQYSQAGFSREDRLEQAKLFCRTLEDILADAPESQNNCRLIAYQEPADDSSFSLSQ
EVLRHLRQEEKEEVTV (SEQ ID NO:9).
Amino acid sequence of truncated AQ STING

APAEISAVCEKGNFNVAHGLAWSYYIGYLRLILPELQARIRTYNQHYNNLLRGAVSQRLYILL
PLDCGVPDNLSMADPNIRFLDKLPQQTADRAGIKDRVYSNSIYELLENGQRAGTCVLEYATP
LQTLFAMSQYSQAGFSREDRLEQAKLFCQTLEDILADAPESQNNCRLIAYQEPADDSSFSLSQ
EVLRHLRQEEKEEVTV (SEQ ID NO:10).

### Differential Scanning Fluorimetry with WT STING and AQ STING

WT and AQ allelic forms of STING protein were diluted to the final concentration 0.1 mg/ml in 100 mmol.l⁻¹ TrisCl buffer pH 7.4 containing, 150 mmol.l⁻¹ NaCl, 1:500 SYPRO Orange (cat. # S6650, ThermoFisher, Waltham, USA) and 150 µM CDN or water. 20 µL solutions of the reaction mixtures were pipetted in triplicates into 96 well optical reaction plates and thermal denaturation of samples were performed on real time PCR cycler (LightCycler ^{®} 480 Instrument II - Roche, Basel, Switzerland). The first derivative of the thermal denaturation curves was performed to calculate denaturing temperatures of STING - CDN complexes and STING apoproteins. The thermal shift for each CDN was calculated by subtracting the average denaturing temperature of STING apoprotein from the average denaturing temperature of STING CDN complex.

### Mouse cGAS Enzyme

A recombinant DNA encoding cDNA of murine cGAS (residues 147-507) was chemically synthesized by GenScript (Piscataway, NJ). This sequence was cloned into the vector pET-22b(+) in-frame between pelB leader and His-tag by homologous recombination using Gibson Assembly^{®} Master Mix (New England Biolabs). The protein was overexpressed in E. coli BL21 (DE3) (ThermoFisher, Waltham, USA). Bacterial pellet was re-suspended in ice-cold lysis buffer containing 20 mM Phosphate Na buffer (pH 7.4), 500 mM NaCl, 10% glycerol, and 20 mM imidazole using Dounce homogenizer. DNase I and RNase A were added (final concentration 50 µg/ml) together with MgCl₂ (final concentration 5mM) to the homogenate and bacteria were lysed using MSE Soniprep 150 (3 mm Tip Solid Titanium Exponential Probe, 2 min, 50% power, amplitude 12 microns). The lysate was spun 30,000 x g for 20 minutes and supernatant was loaded onto 5 mL HisTrap column (GE Healthcare BioSciences, Pittsburgh, USA). The resin was washed with 50 ml lysis buffer and mcGAS was eluted with 15 ml 20 mM Phosphate-Na buffer (pH7.4) buffer containing 500 mM NaCl; 10% glycerol, and 300 mM imidazole. The protein was further purified by size exclusion chromatography using HiLoad 16/60 Superdex 75 in buffer containing 150 mM NaCl; 50 mM Tris (pH 7.4), and 10% glycerol. The protein buffer was exchanged for 50% glycerol, 50 mM Tris (pH 7.6), 100 mM NaCl, 1 mM DTT, 1 mM EDTA with Amicon^{®} Ultra-15 10 K Device (Merck Millipore Ltd.), and mcGAS was flash frozen in liquid N₂.
Murine cGAS amino acid sequence including pelB leader and His-tag

MKYLLPTAAAGLLLLAAQPAMAMPDKLKKVLDKLRLKRKDISEAAETVNKVVERLLRRMQ
KRESEFKGVEQLNTGSYYEHVKISAPNEFDVMFKLEVPRIELQEYYETGAFYLVKFKRIPRGN
PLSHFLEGEVLSATKMLSKFRKIIKEEVKEIKDIDVSVEKEKPGSPAVTLLIRNPEEISVDIILAL
ESKGSWPISTKEGLPIQGWLGTKVRTNLRREPFYLVPKNAKDGNSFQGETWRLSFSHTEKYIL
NNHGIEKTCCESSGAKCCRKECLKLMKYLLEQLKKEFQELDAFCSYHVKTAIFHMWTQDPQ
DSQWDPRNLSSCFDKLLAFFLECLRTEKLDHYFIPKFNLFSQELIDRKSKEFLSKKIEYERNNG
FPIFDKLLEHHHHHH (SEQ ID NO:11).

### BMB171 Diadenylate Cyclase

A recombinant codon optimized cDNA encoding diadenylate cyclase BMB171 from Bacillus sp. was chemically synthesized and cloned between NcoI and XhoI sites of vector pET-28b(+) by GenScript (Piscataway, NJ). The protein was overexpressed in E. coli BL21 (DE3) (ThermoFisher, Waltham, USA). Bacterial pellet was re-suspended in ice-cold lysis buffer containing 20 mM Phosphate Na buffer (pH 7.4), 500 mM NaCl, 10% glycerol, and 20 mM imidazol using Dounce homogenizer. DNase I and RNase A were added (final concentration 50 µg/ml) together with MgCl₂ (final concentration 5 mM) to the homogenate and bacteria were lysed using MSE Soniprep 150 (3 mm Tip Solid Titanium Exponential Probe, 2 min, 50% power, amplitude 12 microns). The lysate was spun 30,000 x g for 20 minutes and supernatant was loaded onto 5 mL HisTrap column (GE Healthcare BioSciences, Pittsburgh, USA). The resin was washed with 50 ml lysis buffer and 50 ml wash buffer (20 mM Phosphate Na buffer (pH 7.4), 500 mM NaCl, 10% glycerol, and 125 mM imidazol) and BMB171 was eluted with 15 ml 20 mM Na-Phosphate buffer (pH7.4) buffer containing 500 mM NaCl; 10% glycerol, and 300 mM imidazol. The protein was further purified by size exclusion chromatography using HiLoad 16/60 Superdex 75 in buffer containing 150 mM NaCl; 50 mM Tris (pH 7.4), and 10% glycerol. The protein buffer was exchanged for 50% glycerol, 50 mM Tris (pH 7.6), 100 mM NaCl, 1 mM DTT, 1 mM EDTA with Amicon^{®} Ultra-15 10 K Device (Merck Millipore Ltd.), and BMB171 was flash frozen in liquid N₂.
BMB171 amino acid sequence including C-terminal His-tag

MEENKQRVKSMINILQLVAPGTPLREGIDNVLRAQTGGLIVLGYNEQIKSIVDGGFHINCAFSP
ASLYELAKMDGALILNETGSKILIANAQLVPDSSIDSIETGMRHRTAERVAKQTGSLVVAISQR
RNVITLYQGNLRYTLKDIGVILTKANQAIQTLEKYKAVWNDGITNLGILEFEEVVTMSEVVH
VLHSVEMVLRIKNEILSYIHELGTEGRLIRLQLTELLADLEAEAALLIKDYHQEKTQDHHQILK
KLQDLANTQLLEDSDLVKLLGYPGQTSLEESVTPRGYRITSKISRVPPLIIENLINRFKTLQGVC
RATINELDDVEGIGEVRAKKIREGLKRIQEHLYMSRHNLEHHHHHH (SEQ ID NO:12).

**Table 5. Biological Data**

| Compound | DSF ΔTₘ (°C) | | Digitonin assay EC₅₀ (µmol.l⁻¹) |
|---|---|---|---|
| | WT STING | AQ STING | WT STING |
| **53** | 6.9 | 11.6 | 0.03 |
| **54** | 9.2 | 15.0 | 0.02 |
| **55** | 1.9 | 8.6 | 0.78 |
| **56** | 4.1 | 10.6 | 0.50 |
| **57** | 6.1 | 11.3 | 0.12 |
| **59** | - | - | 0.70 |

| Compound | DSF ΔTₘ (°C) | | Digitonin assay EC₅₀ (Δmol.l⁻¹) |
|---|---|---|---|
| | WT STING | AQ STING | WT STING |
| **60** | - | - | 0.28 |
| **61** | - | - | 0.75 |
| **65** | 3.1 | 8.0 | 5 |
| **66** | 0.5 | 1.7 | 15 |
| **67** | 0.3 | 2.9 | 6.4 |
| **69** | 1.2 | 3.5 | 32.8 |
| **70** | 0.5 | 1.1 | 16.1 |
| 3'3'c-di-GMP | 2.6 | 7.7 | 5.8 |
| 3'3'c-di-AMP | 2.6 | 9.3 | 0.3 |
| 3'3'-cGAMP | 5.1 | 13.3 | 0.16 |
| 2'2'-cGAMP | 11.5 | 19.4 | 0.03 |
| 2'3'-cGAMP | 15.2 | 22.7 | 0.03 |

### Synthesis and Characterization of 3'3' phosphonate CDN prodrugs

CDN (1 umol, tetrabutylammonium salt) was azeotroped with dry toluene (1x500 uL) and dry ACN (2x500 uL), dissolved in dry ACN (700 uL) and treated with pivaloyloxymethyl iodide (10 umol, 2.64 uL, J.E.Coughlin et al.; Bioorg. Med. Chem. Lett. 20 (2010) 1783-1786) under argon atmosphere at RT for 6 hours. The reaction mixture was quenched with 1 ml of 50% H₂O/ACN and further diluted with 3 ml of water and directly applied to the HPLC column (HPLC Method 1). Fractions containing product were pooled, evaporated and freeze-dried from dioxane.

### HPLC method

| **Time (min)** | **Flow (mL/min)** | **H₂O (%)** | **1:1 H₂O/ACN (%)** | **ACN (%)** |
|---|---|---|---|---|
| 0 | 1 | 100 | - | - |
| 3 | 1 | 100 | - | - |
| 10 | 3 | 100 | - | - |
| 40 | 3 | - | 100 | - |
| 55 | 3 | - | - | 100 |
| 60 | 3 | - | - | 100 |

Preparative HPLC purifications were performed on Waters Delta 600 chromatography system with columns packed with C18 reversed phase resin (XTerra^{®} Prep RP18 Column, 5 µm, 10 x 100mm). In all methods were used linear gradients.

The C18 analytical HPLC method described above was used for standard analysis.

The following compounds were synthesized using the aforementioned methods using the compounds described above.

| **Precursor Compound** | **Prodrug Compound(s)** |
|---|---|
| **53** | **80a-c, 81a-c, 82a-c, 83a-c, 84a-c and 85a-b** |
| **60** | **86** |
| **61** | **87a-c** |

**Table 1: Exemplary compounds and characterization data**

| **Compound** | **Structure / Data** | **Mass [M+H]⁺** |
|---|---|---|
| **80a** | 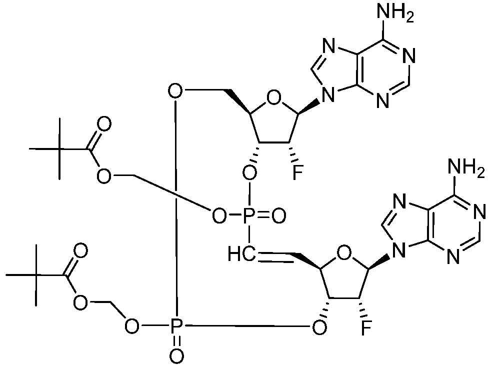 {[(1R,6R,8R,9R,10R,13E,15R,17R,18R)-8,17-bis(6-amino-9H-purin-9-yl)-3-{[(2,2-dimethylpropanoyl)oxy]methoxy}-9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylpropanoate | 887.4 |
| **80a:** HPLC retention time (C18, min): 3.31. | | |
| **80b** | 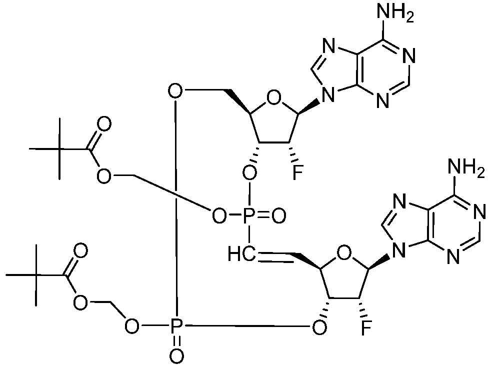 {[(1R,6R,8R,9R,10R,13E,15R,17R,18R)-8,17-bis(6-amino-9H-purin-9-yl)-3-{[(2,2-dimethylpropanoyl)oxy]methoxy} -9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylpropanoate | 887.4 |
| **80b:** HPLC retention time (C18, min): 3.45. | | |
| **80c** | 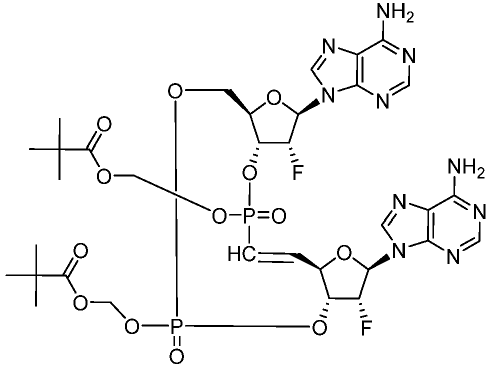 {[(1R,6R,8R,9R,10R,13E,15R,17R,18R)-8,17-bis(6-amino-9H-purin-9-yl)-3-{[(2,2-dimethylpropanoyl)oxy]methoxy}-9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylpropanoate | 887.3 |
| **80c:** HPLC retention time (C18, min): 3.64. ¹H NMR (501 MHz, CD₃CN) δ 8.36 and 8.23 (s, 2H), 8.09 and 7.98 (s, 2H), 7.03 (ddd, *J* = 21.8, 17.2, 8.2 Hz, 1H), 6.18 - 6.54 (m, 7H), 5.58-5.83 (m, 8H), 4.75 (m, 1H), 4.35 - 4.39 (m, 2H), 4.19 (m, 1H), 1.19 (s, 9H), 1.17 (s, 9H). ³¹P NMR (202.4 MHz, CD₃CN) δ 19.09, -3.25. ¹⁹F NMR (470.4 MHz, CD₃CN) δ -194.26, -196.95. | | |
| **81a** | 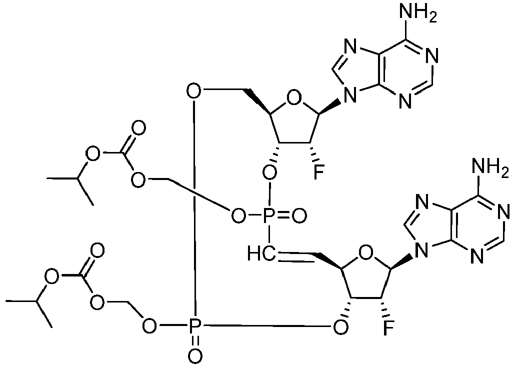 {[(1R,6R,8R,9R,10R,13E,15R,17R,18R)-8,17-bis(6-amino-9H-purin-9-yl)-9,18-difluoro-3,12-dioxo-3-({[(propan-2-yloxy)carbonyl]oxy}methoxy)-2,4,7,11,16-pentaoxa-3λ⁵,12λ⁵-diphosphatricyclo[13.3.0.0⁶,¹⁰]octadec-13-en-12-yl]oxy}methyl propan-2-yl carbonate | 891.1 |
| **81a:** HPLC retention time (C18, min): 3.11. | | |
| **81b** | 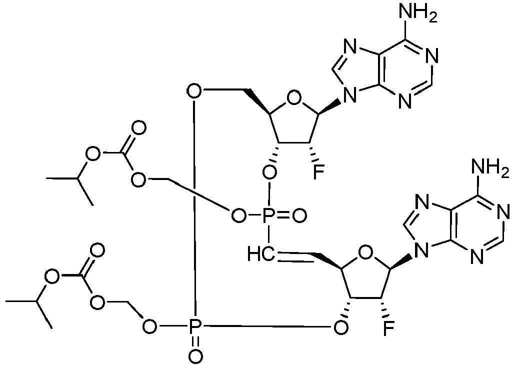 {[(1R,6R,8R,9R,10R,13E,15R,17R,18R)-8,17-bis(6-amino-9H-purin-9-yl)-9,18-difluoro-3,12-dioxo-3-({[(propan-2-yloxy)carbonyl]oxy}methoxy)-2,4,7,11,16-pentaoxa-3λ⁵,12λ⁵-diphosphatricyclo[13.3.0.0⁶,¹⁰]octadec-13-en-12-yl]oxy}methyl propan-2-yl carbonate | 887.2 |
| **81b:** HPLC retention time (C18, min): 3.22. | | |
| **81c** | 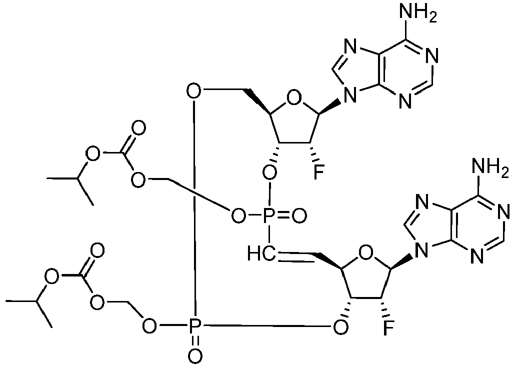 {[(1R,6R,8R,9R,10R,13E,15R,17R,18R)-8,17-bis(6-amino-9H-purin-9-yl)-9,18-difluoro-3,12-dioxo-3-({[(propan-2-yloxy)carbonyl]oxy}methoxy)-2,4,7,11,16-pentaoxa-3λ⁵,12λ⁵-diphosphatricyclo[13.3.0.0⁶,¹⁰]octadec-13-en-12-yl]oxy}methyl propan-2-yl carbonate | 887.1 |
| **81c:** HPLC retention time (C18, min): 3.42. ¹H NMR (501 MHz, CD₃CN) δ 8.38 and 8.27 (s, 2H), 8.06 and 7.99 (s, 2H), 7.02 (ddd, *J* = 21.6, 17.0, 8.1, 1H), 6.13 - 6.36 (m, 7H), 5.60-5.81 (m, 8H), 4.82 - 4.91 (m, 2H), 4.75 (m, 1H) 4.36 - 4.40 (m, 2H), 4.20 (m, 1H), 1.24 - 1.28 (m, 12H). ³¹P NMR (202.4 MHz, CD₃CN) δ 18.66, -3.29. ¹⁹F NMR (470.4 MHz, CD₃CN) δ -194.37, -197.16. | | |
| **82a** | 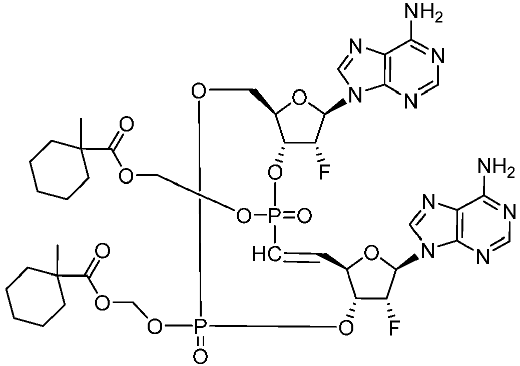 {[(1R,6R,8R,9R,10R,13E,15R,17R,18R)-8,17-bis(6-amino-9H-purin-9-yl)-9,18-difluoro-12-[(1-methylcyclohexanecarbonyloxy)methoxy]-3,12-dioxo-2,4,7,11,16-pentaoxa-3λ⁵,12λ⁵-diphosphatricyclo[13.3.0.0⁶,¹⁰]octadec-13-en-3-yl]oxy}methyl 1-methylcyclohexane-1-carboxylate | 967.4 |
| **82a:** HPLC retention time (C18, min): 3.77. | | |
| **82b** | 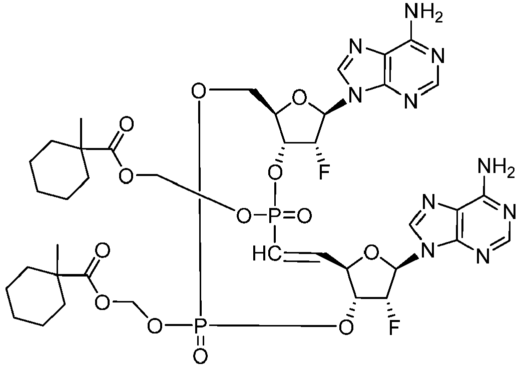 {[(1R,6R,8R,9R,10R,13E,15R,17R,18R)-8,17-bis(6-amino-9H-purin-9-yl)-9,18-difluoro-12-[(1-methylcyclohexanecarbonyloxy)methoxy]-3,12-dioxo-2,4,7,11,16-pentaoxa-3λ⁵,12λ⁵-diphosphatricyclo[13.3.0.0⁶,¹⁰]octadec-13-en-3-yl]oxy}methyl 1-methylcyclohexane-1-carboxylate | 967.3 |
| **82b:** HPLC retention time (C18, min): 3.94. | | |
| **82c** | 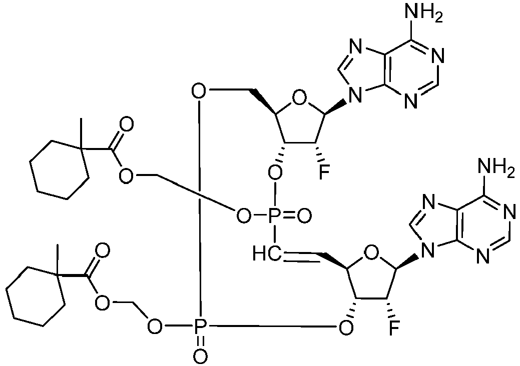 {[(1R,6R,8R,9R,10R,13E,15R,17R,18R)-8,17-bis(6-amino-9H-purin-9-yl)-9,18-difluoro-12-[(1-methylcyclohexanecarbonyloxy)methoxy]-3,12-dioxo-2,4,7,11,16-pentaoxa-3λ⁵,12λ⁵-diphosphatricyclo[13.3.0.0⁶,¹⁰]octadec-13-en-3-yl]oxy}methyl 1-methylcyclohexane-1-carboxylate | 967.5 |
| **82c:** HPLC retention time (C18, min): 4.15. ¹H NMR (501 MHz, CD₃CN) δ 8.40 and 8.27 (s, 2H), 8.07 and 7.99 (s, 2H), 7.02 (ddd, *J* = 21.6, 17.0, 8.1 Hz, 1H), 6.37 (bs, 2H), 6.23 - 6.31 (m, 3H), 6.15 (bs, 2H), 5.61 - 5.77 (m, 8H), 4. 73 (m, 1H), 4.34 - 4.38 (m, 2H), 4.17 (m, 1H), 1.96 (m, 2H), 1.21 - 1.57 (m, 18H), 1.15 (s, 3H), 1.13 (s, 3H). ³¹P NMR (202.4 MHz, CD₃CN) δ 19.03, -3.23. ¹⁹F NMR (470.4 MHz, CD₃CN) δ -194.18, -196.88. | | |
| **83a** | 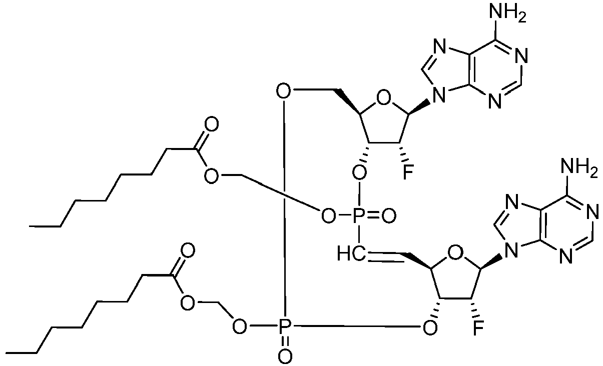 {[(1R,6R,8R,9R,10R,13E,15R,17R,18R)-8,17-bis(6-amino-9H-purin-9-yl)-9,18-difluoro-3-[(octanoyloxy)methoxy]-3,12-dioxo-2,4,7,11,16-pentaoxa-3λ⁵,12λ⁵-diphosphatricyclo[13.3.0.0⁶,¹⁰]octadec-13-en-12-yl]oxy}methyl octanoate | 971.0 |
| **83a:** HPLC retention time (C18, min): 4.17. | | |
| **83b** | 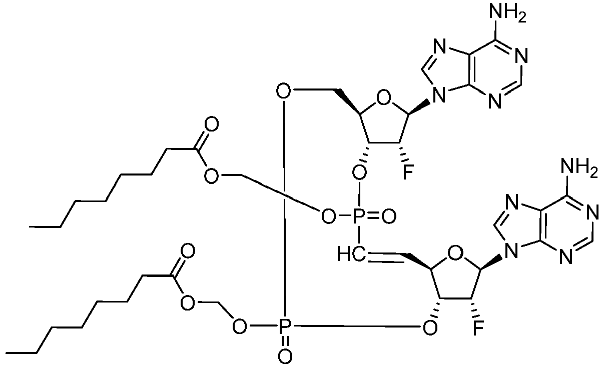 {[(1R,6R,8R,9R,10R,13E,15R,17R,18R)-8,17-bis(6-amino-9H-purin-9-yl)-9,18-difluoro-3-[(octanoyloxy)methoxy]-3,12-dioxo-2,4,7,11,16-pentaoxa-3λ⁵,12λ⁵-diphosphatricyclo[13.3.0.0⁶,¹⁰]octadec-13-en-12-yl]oxy}methyl octanoate | 971.0 |
| **83b:** HPLC retention time (C18, min): 4.33. | | |
| **83c** | 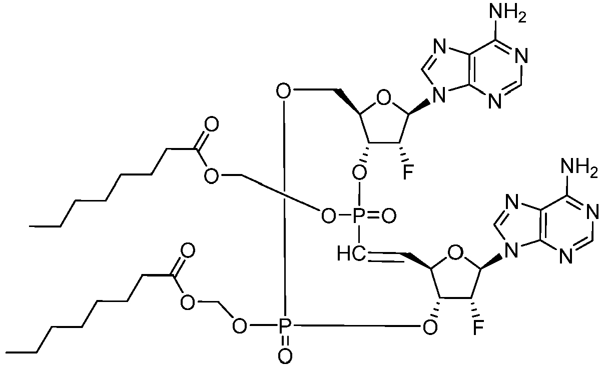 {[(1R,6R,8R,9R,10R,13E,15R,17R,18R)-8,17-bis(6-amino-9H-purin-9-yl)-9,18-difluoro-3-[(octanoyloxy)methoxy]-3,12-dioxo-2,4,7,11,16-pentaoxa-3λ⁵,12λ⁵-diphosphatricyclo[13.3.0.0⁶,¹⁰]octadec-13-en-12-yl]oxy}methyl octanoate | 971.0 |
| **83c:** HPLC retention time (C18, min): 4.52. ¹H NMR (501 MHz, CD₃CN) δ 8.37 and 8.19 (s, 2H), 8.09 and 7.99 (s, 2H), 7.02 (ddd, *J* = 21.9, 7.1, 8.3 Hz, 1H), 6.16 - 6.66 (m, 7H), 5.55 - 5.86 (m, 8H), 4.75 (m, 1H), 4.35 - 4.40 (m, 2H), 4.19 (m, 1H), 2.34 - 2.38 (m, 4H), 1.53 - 1.62 (m, 4H), 1.20 - 1.32 (m, 16H), 0.83 - 0.87 (m, 6H). ³¹P NMR (202.4 MHz, CD₃CN) δ 18.92, -3.10. ¹⁹F NMR (470.4 MHz, CD₃CN) δ -194.31, -197.24. | | |
| **84a** | 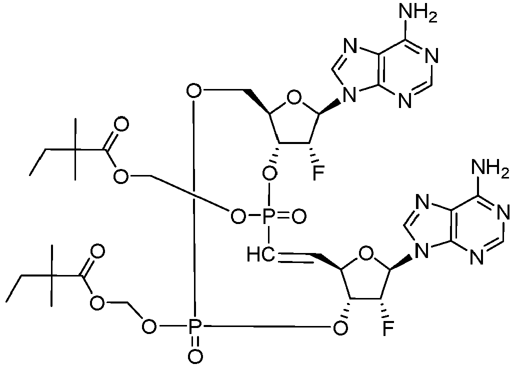 | 915.3 |
| | {[(1R,6R,8R,9R,10R,13E,15R,17R,18R)-8,17-bis(6-amino-9H-purin-9-yl)-3-{[(2,2-dimethylbutanoyl)oxy]methoxy}-9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylbutanoate | |
| **84a:** HPLC retention time (C18, min): 3.50. | | |
| **84b** | 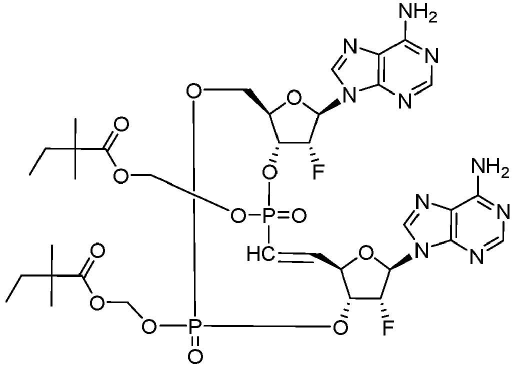 {[(1R,6R,8R,9R,10R,13E,15R,17R,18R)-8,17-bis(6-amino-9H-purin-9-yl)-3-{[(2,2-dimethylbutanoyl)oxy] methoxy}-9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylbutanoate | 915.3 |
| **84b:** HPLC retention time (C18, min): 3.64. | | |
| **84c** | 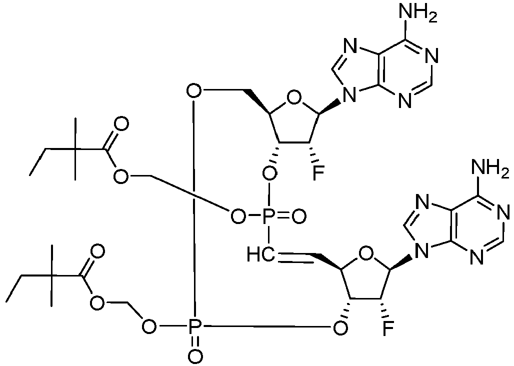 {[(1R,6R,8R,9R,10R,13E,15R,17R,18R)-8,17-bis(6-amino-9H-purin-9-yl)-3-{[(2,2-dimethylbutanoyl)oxy]methoxy}-9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylbutanoate | 915.3 |
| **84c:** HPLC retention time (C18, min): 3.85. | | |
| **85a** | 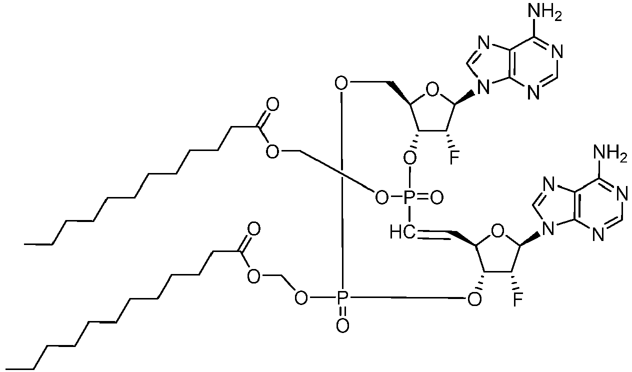 {[(1R,6R,8R,9R,10R,13E,15R,17R,18R)-8,17-bis(6-amino-9H-purin-9-yl)-3-[(dodecanoyloxy)methoxy]-9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl dodecanoate | 1084.4 |
| **85a:** HPLC retention time (C18, min): 5.68. | | |
| **85b** | 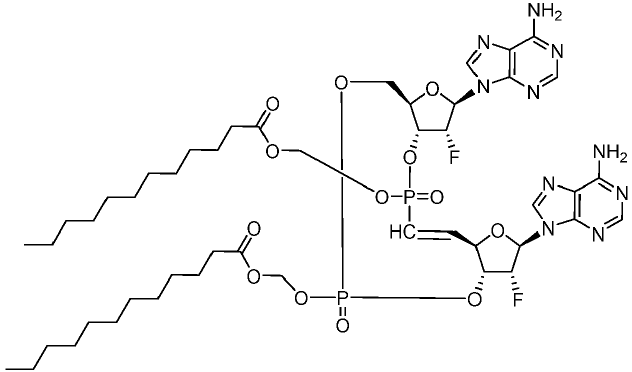 {[(1R,6R,8R,9R,10R,13E,15R,17R,18R)-8,17-bis(6-amino-9H-purin-9-yl)-3-[(dodecanoyloxy)methoxy]-9,18-difluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl dodecanoate. | 1084.3 |
| **85b:** HPLC retention time (C18, min): 5.40. | | |
| **86** | 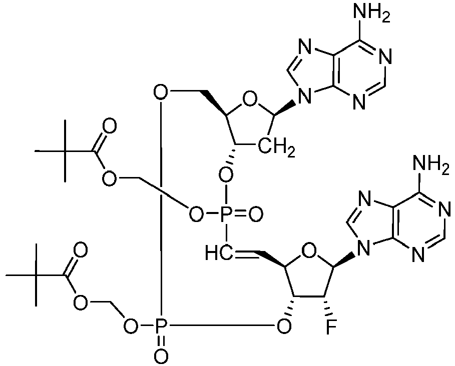 {[(1R,6R,8R,10S,13E,15R,17R,18R)-8,17-bis(6-amino-9H-purin-9-yl)-3-{[(2,2-dimethylpropanoyl)oxy]methoxy}-18-fluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylpropanoate. | 869.3 |
| **86c:** HPLC retention time (C18, min): 3.84. ¹H NMR (501 MHz, CD₃CN) δ 8.31 (s, 1H), 8.26 (s, 1H), 8.01 (s, 1H), 8.01 (s, 1H), 6.94 (ddd, *J* = 21.8, 17.1, 8.1 Hz, 1H), 6.38 (t, *J* = 6.7 Hz, 1H), 6.27 (d, *J* = 22.4 Hz, 1H), 6.21 (ddd, *J* = 19.4, 17.1, 0.8 Hz, 1H), 6.08 (bs, 2H), 6.03 (bs, 2H), 5.88 (dtd, *J* = 22.5, 9.5, 4.7 Hz, 1H), 5.57 - 5.68 (m, 5H), 5.32 (ddt, *J* = 10.1, 6.9, 3.5 Hz, 1H), 4.74 (m, 1H), 4.33 (ddd, *J=* 10.5, 8.1, 5.1 Hz, 1H), 4.25 (m, 1H), 4.09 (ddd, *J* = 10.5, 5.6, 3.6 Hz, 1H), 3.26 (dt, *J* = 14.4, 6.8 Hz, 1H), 2.70 (ddd, *J* = 14.4, 6.7, 3.7 Hz, 1H), 1.20 (s, 9H), 1.16 (s, 9H). ³¹P NMR (202.4 MHz, CD₃CN) δ 18.24, -0.95. ¹⁹F NMR (470.4 MHz, CD₃CN) δ -194.04. | | |
| **87a** | 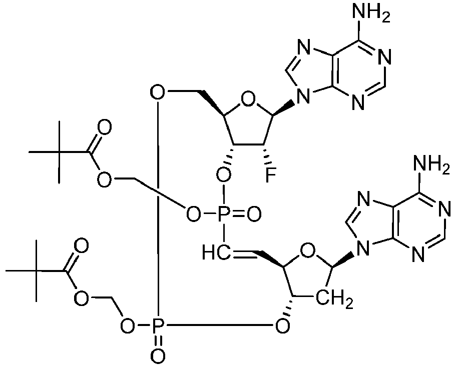 {[(1S,6R,8R,9R,10R,13E,15R,17R)-8,17-bis(6-amino-9H-purin-9-yl)-3-{[(2,2-dimethylpropanoyl)oxy]methoxy}-9-fluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylpropanoate. | 869.3 |
| **87a:** HPLC retention time (C18, min): 3.49. | | |
| **87b** | 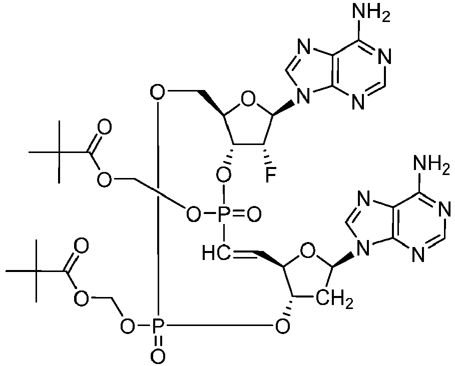 {[(1S,6R,8R,9R,10R,13E,15R,17R)-8,17-bis(6-amino-9H-purin-9-yl)-3-{[(2,2-dimethylpropanoyl)oxy]methoxy}-9-fluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylpropanoate. | 869.4 |
| **87b:** HPLC retention time (C18, min): 3.62. | | |
| **87c** | 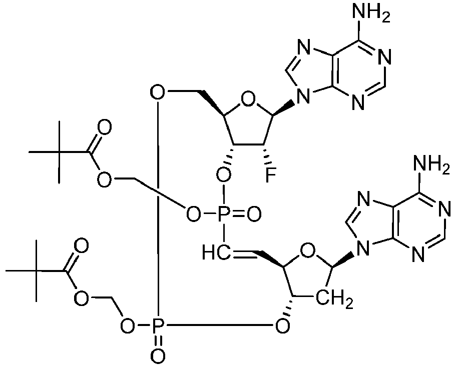 {[(1S,6R,8R,9R,10R,13E,15R,17R)-8,17-bis(6-amino-9H-purin-9-yl)-3-{[(2,2-dimethylpropanoyl)oxy]methoxy}-9-fluoro-3,12-dioxo-2,4,7,11,16-pentaoxa-3λ⁵,12λ⁵-diphosphatricyclo[13.3.0.0⁶,¹⁰]octadec-13-en-12-yl]oxy}methyl 2,2-dimethylpropanoate. | 869.4 |
| **87c:** HPLC retention time (C18, min): 3.82. ¹H NMR (501 MHz, CD₃CN) δ 8.29 (s, 1H), 8.14(s, 1H), 8.13 (s, 1H), 7.95 (s, 1H), 7.07 (ddd, *J* = 21.9, 17.1, 8.4 Hz, 1H), 6.68 (bs, 2H), 6.35 (dd, *J* = 8.9, 2.6 Hz, 1H), 6.28 (d, *J* = 18.1 Hz, 1H), 6.19 (dd, *J* = 19.7, 17.2 Hz, 1H), 6.14 (bs, 2H), 5.64 - 5,77 (m, 6H), 5.48 (m, 1H), 4.54 (m, 1H), 4.36 - 4.41 (m, 2H), 4.17 (m, 1H), 3.20 (m, 1H), 2.81 (dt, *J* = 13.9, 8.6 Hz, 1H), 1.21 (s, 9H), 1.19 (s, 9H). ³¹P NMR (202.4 MHz, CD₃CN) δ 19.88 (s), - 2.84 (s). ¹⁹F NMR (470.4 MHz, CD₃CN) δ -197.73. | | |

### 293T WT STING standard reporter assay

293T wtSTING-FL cells were seeded at density of 250,000 cells per cm² onto 96 well poly-D-lysine coated white micro plates in 100 µl DMEM medium with high glucose supplemented with 10% heat inactivated FBS. The medium was removed next day and three-fold serial dilutions of compounds in 100 µl medium were added to the cells. The plates with cells were incubated for 7 hours at 37 °C with 5% CO₂. After 50 µl of the medium was removed, 30 µl of ONE-Glo^{™} Luciferase Assay System reagent (cat. # E6120, Promega, Madison, USA) was added to each well and luminescence was read on Synergy H1 (Biotek, Winooski, USA). GraphPad Prism (La Jolla, USA) was used to calculate the 50% effective concentration (EC₅₀) from an 8-point dose-response curve. Control compounds 3'3'-cGAMP (cat. # tlrl-nacga), 2'3'-cGAMP (cat. # tlrl-nacga23), and 2'2'-cGAMP (cat. # tlrl-nacga22) were purchased from Invivogen (San Diego, USA).

**Table 3: 293T WT STING standard reporter assay data**

| **Compound** | **293T WT STING** EC₅₀ **(µM)** |
|---|---|
| **53** | 10.1 |
| **60** | 71.8 |
| **61** | >200 |
| **80a** | 0.084 |
| **80b** | 0.022 |
| **80c** | 0.012 |
| **81a** | 9.5 |
| **81b** | 1.2 |
| **81c** | 0.122 |
| **82a** | 0.2 |
| **82b** | 0.08 |
| **82c** | 0.015 |
| **83a** | 0.023 |
| **83b** | 0.003 |
| **83c** | 0.003 |
| **84a** | 0.15 |
| **84b** | 0.01 |
| **84c** | 0.007 |
| **85a** | 0.0004 |
| **85b** | 0.003 |
| **86** | 0.01 |
| **87a** | 12.1 |
| **87b** | 0.67 |
| **87c** | 0.260 |
| 3'3'-cGAMP | 68.4 |
| 2'2'-cGAMP | 10.2 |
| 2'3'-cGAMP | 36.9 |

### Peripheral Blood Mononuclear Cell Assay

Selected compounds were tested in an in vitro peripheral blood mononuclear cell (PBMC) assay. Freshly isolated PBMCs were seeded into U-shaped shaped 96-well plates at desity 500,000 cells per well in 50 µl of RPMI 1640 medium supplemented with 10% heat inactivated FBS. Serially dilluted tested compounds were added to wells in 50 µl of cultivation medium and cell were incubated with compounds for 1h at 37 °C with 5% CO₂. Plates with cells were then spun 500 g for 5 minutes and medium was removed. Cells were washed twice with the cell culture medium by the use of centrifugation and gently resuspended in 100 µl medium without compounds. After 15 hour incubation at 37 °C with 5% CO₂, the cell culture medium was collected and the levels of interferon-alpha (INF-α), interferon-gamma (INF-γ) and tumor necrosis factor-alpha (TNF-α) were determined with ProcartaPlex Assays (ThermoFisher, Waltham, USA) and MAGPIX System (Merck KGaA, Darmstadt, Germany). GraphPad Prism (San Diego, CA, USA) was used to calculate the 50% effective concentration (EC₅₀) from a 6-point dose-response curve.

**Table 4: Peripheral blood mononuclear cell data**

| **Compound** | **PBMC EC₅₀ (µM)** | | |
|---|---|---|---|
| | **IFN-γ** | **TNF-α** | **IFN-α** |
| **53** | 36 | 54 | 120 |
| **60** | 164 | 100 | >500 |
| **80c** | 0.18 | 0.1 | 0.1 |
| **81c** | 1.3 | 0.25 | 0.6 |
| **82c** | 0.07 | 0.06 | 0.1 |
| **84b** | 0.06 | 0.07 | 0.08 |
| **84c** | - | 0.01 | 0.03 |
| **85a** | 0.01 | 0.02 | 0.02 |
| **86** | 1.6 | 0.8 | 0.6 |

## Claims

1. A compound of formula (J): 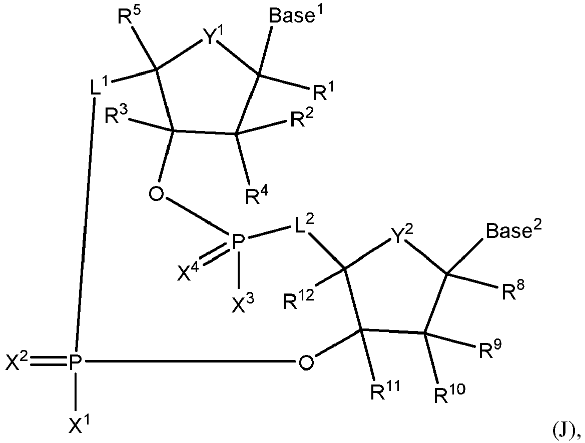 or an enantiomer, hydrate, solvate, or pharmaceutically acceptable salt thereof,
wherein
L¹ is -C(R⁶R⁷)-O- and L² is -C(R¹³)=C(R¹⁴)-,
L¹ is -O-C(R⁶R⁷)- and L² is -C(R¹³)=C(R¹⁴)-,
L¹ is -C(R¹³)=C(R¹⁴)- and L² is -C(R¹³)=C(R¹⁴)-,
L' is -C(R¹³)=C(R¹⁴)- and L² is -C(R⁶R⁷)-O-, or
L¹ is -C(R¹³)=C(R¹⁴)- and L² is -O-C(R⁶R⁷)-;
Y' and Y² are each independently -O-, -S-, or -CH₂-;
X¹ and X³ are each independently OH, SH, OR¹⁵, SR¹⁵, or N(R¹⁵)₂;
X² and X⁴ are each independently O or S;
R¹, R⁵, R⁸ and R¹² are each independently H, CN, N₃, F, Cl, Br, I, COOR¹⁵, CON(R¹⁵)₂, C₁-C₆ alkyl, C₂-C₆ alkenyl, C₂-C₆ alkynyl, OR¹⁵, SR¹⁵, or N(R¹⁵)₂;
R², R³, R⁴, R⁹, R¹⁰ and R¹¹ are each independently H, F, Cl, Br, I, CN, N₃, C₁-C₆ alkyl, C₂-C₆ alkenyl, C₂-C₆ alkynyl, OR¹⁵, SR¹⁵, or N(R¹⁵)₂;
R⁶, R⁷, R¹³ and R¹⁴ are each independently H, CN, N₃, F, Cl, Br, I, COOR¹⁵, CON(R¹⁵)₂, OR¹⁵, SR¹⁵, N(R¹⁵)₂, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each R¹⁵ is independently H, -C(=Z)R¹⁶, -C(=Z)OR¹⁶, -C(=Z)SR¹⁶, -C(=Z)N(R¹⁶)₂, -CH₂-Z-C(=Z)R¹⁶, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each R¹⁶ is independently H, C₁-C₁₂ alkyl, C₂-C₁₂ alkenyl, C₂-C₁₂ alkynyl, C₃-C₇ cycloalkyl, C₂-C₁₀ heterocycloalkyl, C₆-C₁₀ aryl, or C₂-C₁₀ heteroaryl; preferably each R¹⁶ is independently H, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each Z is independently O or S;
Base¹ and Base² are each independently: 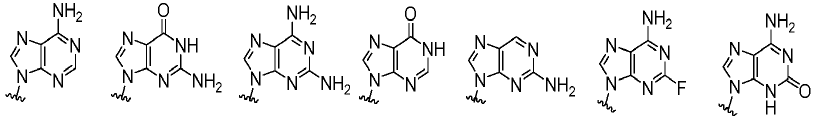 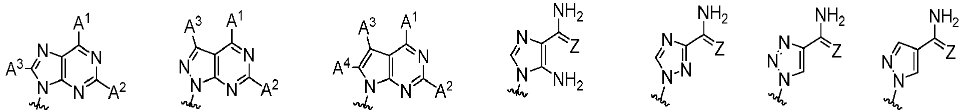 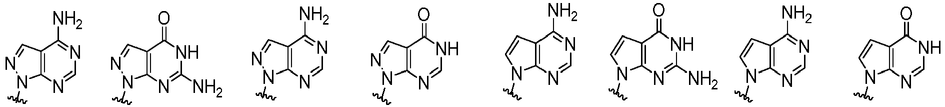 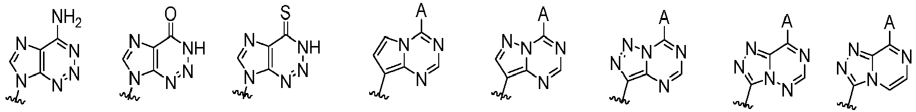 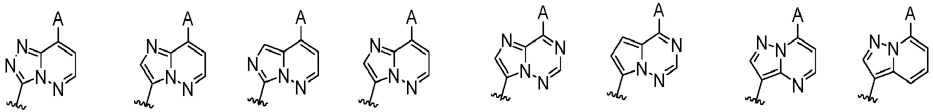 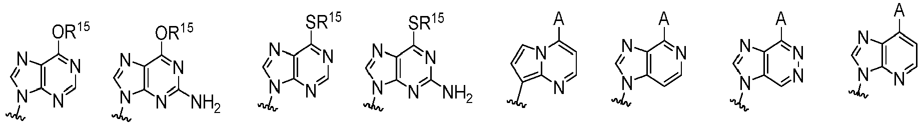 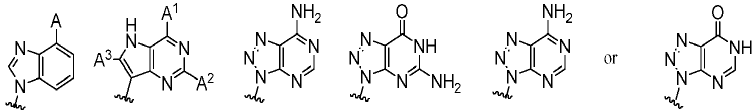
wherein
A, A¹, A², A³ and A⁴ are each independently H, OH, SH, F, Cl, Br, I, NH₂, OR¹⁵, SR¹⁵, NHR¹⁵, N(R¹⁵)₂, or R¹⁶; and
wherein the C₁-C₆ alkyl, C₂-C₆ alkenyl, C₂-C₆ alkynyl, C₂-C₁₀ heterocycloalkyl, C₆-C₁₀ aryl, or C₂-C₁₀ heteroaryl independently in each instance is optionally substituted with 1, 2, or 3 -OH; -SH; -NH₂; =O; =NH; =S; halogen; -N₃; C₆-C₁₀ aryl optionally substituted with 1, 2, or 3-OH, -CN, -O(C=O)OR^{B}, -O(C=O)R^{B}, or -COOR^{B}; unsubstituted C₁-C₆ alkyl; unsubstituted C₁-C₆ alkoxy; unsubstituted C₁-C₆ alkylthio; unsubstituted C₁-C₆ alkylamino; unsubstituted C₁-C₆ dialkylamino; -CN; -O(C=O)OR^{B}; -O(C=O)R^{B}; or -COOR^{B}; wherein R^{B} is H or unsubstituted C₁-C₆ alkyl.

2. A compound according to claim 1, wherein
Y¹ and Y² are each independently -O- or -S-;
X¹ and X³ are each independently OH, SH, OR¹⁵ or SR¹⁵;
X² and X⁴ are each independently O or S;
R¹, R⁵, R⁸ and R¹² are H;
R², R³, R⁹, and R¹¹ are H;
R⁴ and R¹⁰ are each independently H, F, Cl, CN, N₃, OH, SH, NH₂, OR¹⁵, SR¹⁵, or N(R¹⁵)₂; preferably these substituents are independently selected from H, F, Cl, OH, SH, NH₂, CN and N₃;
R⁶, R⁷, R¹³ and R¹⁴ are each independently H, C₁-C₆ alkyl, C₂-C₆ alkenyl, or C₆-C₁₀ aryl; preferably these substituents are H or C₁-C₄ alkyl;
R¹⁵ is independently H, -C(=Z)R¹⁶, -CH₂-Z-C(=Z)R¹⁶, C₁-C₆ alkyl, C₂-C₆ alkenyl, C₂-C₆ alkynyl, C₃-C₇ cycloalkyl, C₂-C₁₀ heterocycloalkyl, C₆-C₁₀ aryl, or C₂-C₁₀ heteroaryl;
each R¹⁶ is independently H, C₁-C₁₂ alkyl, C₂-C₁₂ alkenyl, C₂-C₁₂ alkynyl, C₃-C₇ cycloalkyl, C₂-C₁₀ heterocycloalkyl, C₆-C₁₀ aryl, or C₂-C₁₀ heteroaryl; preferably each R¹⁶ is independently H, C₁-C₆ alkyl, C₂-C₆ alkenyl, C₃-C₇ cycloalkyl, C₆-C₁₀ aryl, or C₂-C₁₀ heteroaryl;
each Z is independently O or S.

3. The compound of claim 1 or 2 having the structure selected from formula (I), formula (IIa), formula (Ia) and formula (IIIb), or an enantiomer, hydrate, solvate, or pharmaceutically acceptable salt thereof:

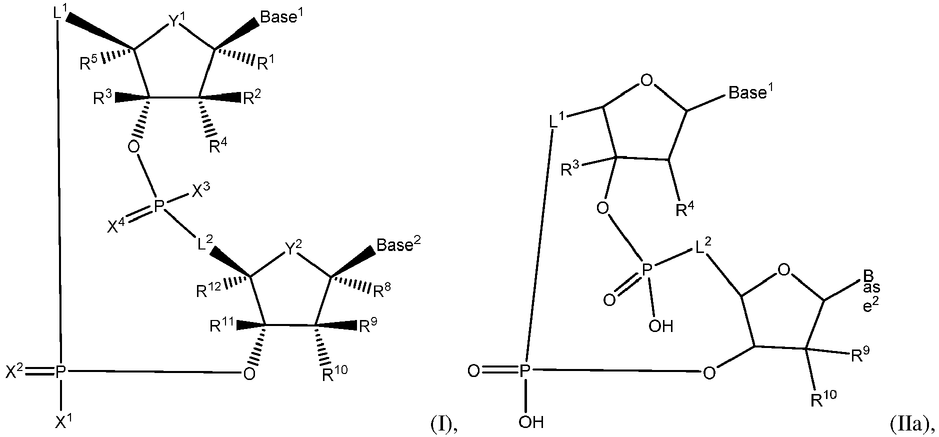

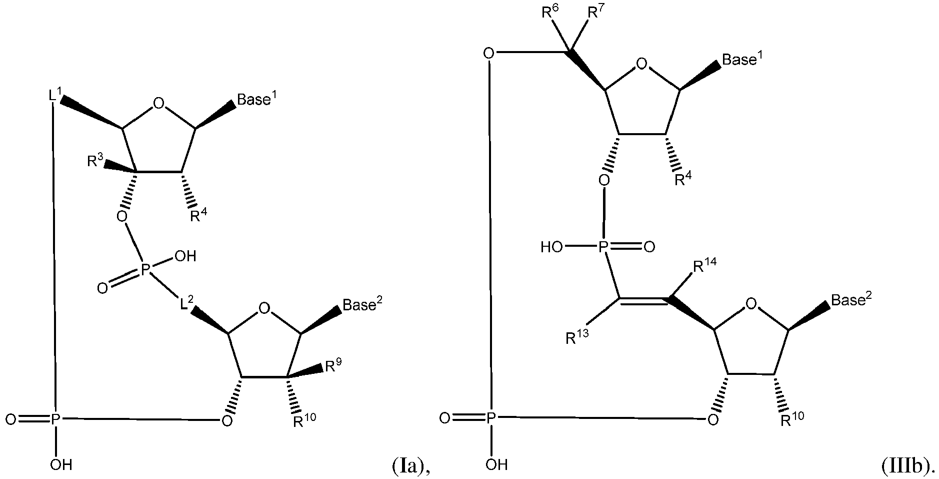

4. The compound of any one of claims 1 to 3, wherein
- X² and X⁴ are each O, and/or
- Y¹ and Y² are each independently -O- or -CH₂-, and/or
R¹, R², R⁵, R⁸, R¹¹ and R¹² are each independently H, OH, F, CN, or Ci-Ce alkyl, and/or
X¹ and X³ are each independently OH or SH.

5. The compound of any one of claims 1 to 4, wherein
L¹ is -C(R¹³)=C(R¹⁴)- and L² is -O-C(R⁶R⁷)-; or
L¹ is -O-C(R⁶R⁷)- and L² is -C(R¹³)=C(R¹⁴)-; or
L¹ is -C(R¹³)=C(R¹⁴)- and L² is -C(R¹³)=C(R¹⁴)-.

6. The compound of any one of claims 1 to 5, wherein
R³ and R⁴ are each independently H, OR¹⁵, F, Cl, CN, N₃, or C₁-C₆ alkyl, wherein
at least one of R³ and R⁴ is H;
or
R⁹ and R¹⁰ are each independently H, OR¹⁵, F, Cl, CN, N₃, or C₁-C₆ alkyl, wherein
at least one of R⁹ and R¹⁰ is H.

7. The compound of any one of claims 1 to 6, wherein R⁴ and R₁₀ are independently H, OH or F.

8. The compound of any one of claims 1 to 7, wherein R⁶, R⁷, R¹³ and R¹⁴ are each independently H, CN, F, Cl, COOR¹⁵, CON(R¹⁵)₂, OR¹⁵, SR¹⁵, N(R¹⁵)₂, or C₁-C₆ alkyl, wherein each R¹⁵ is independently H or C₁-C₆ alkyl or R⁶, R⁷, R¹³ and R¹⁴ are each H.

9. The compound of any one of claims 1 to 8, wherein Base¹ and Base² are each independently selected from:

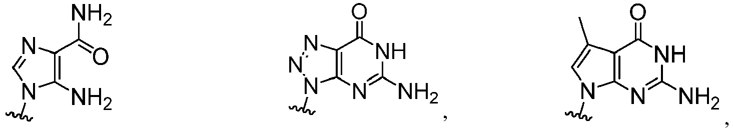

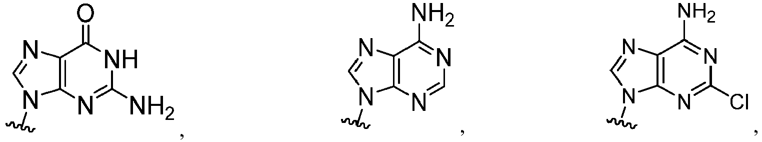

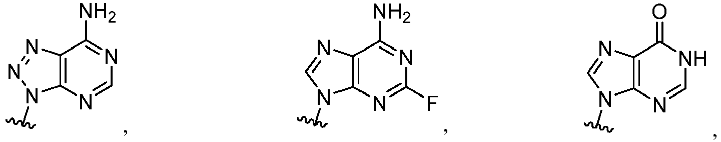

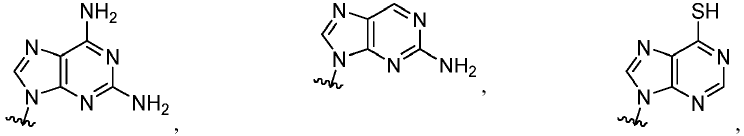

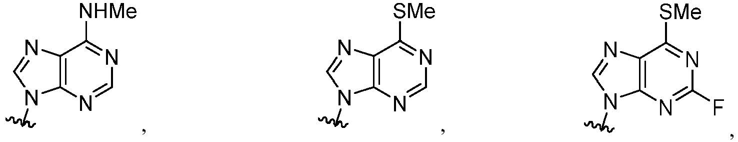

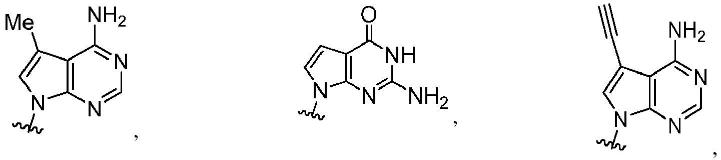

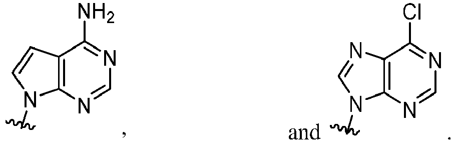

10. The compound of claim 1, wherein the compound is selected from: 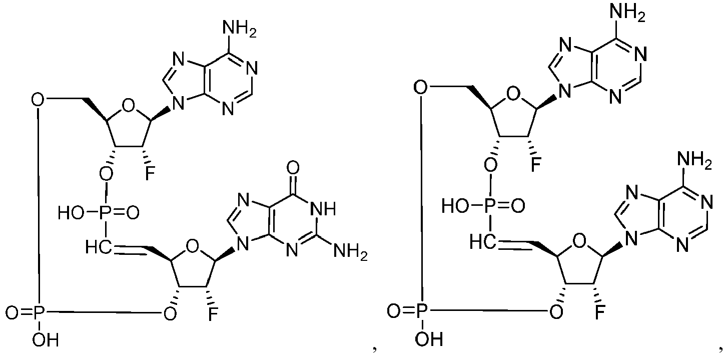 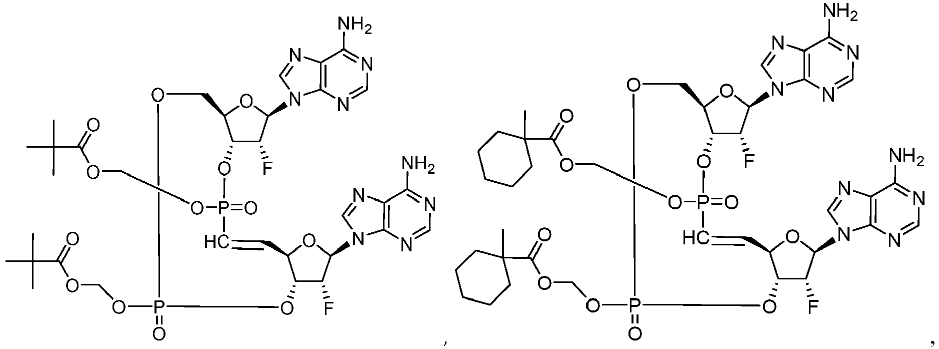 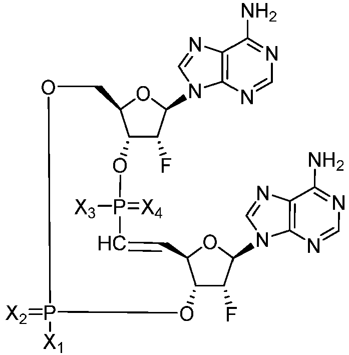 wherein X¹, X², X³, X⁴ are as defined in claim 1.

11. A pharmaceutical composition comprising the compound of any one of claims 1 to 10, or an enantiomer, solvate, hydrate, or a pharmaceutically acceptable salt thereof, and a pharmaceutically acceptable carrier, excipient, and/or diluent.

12. The compound according to any one of claims 1 to 10 for use in modulating the activity of STING adaptor protein to induce production of a type I interferon, cytokine and/or chemokine dependent on the STING adaptor protein, and/or for use in treating or preventing a viral infection, hepatitis B virus infection, HIV infection, allergic diseases, inflammatory diseases, hyperproliferative disease or cancer in a human or animal.

13. The compound according to any one of claims 1 to 10 for use as a component of an immunomodulating composition, and/or for use as a vaccine adjuvans.

## Patentansprüche

1. Eine Verbindung der Formel (J): 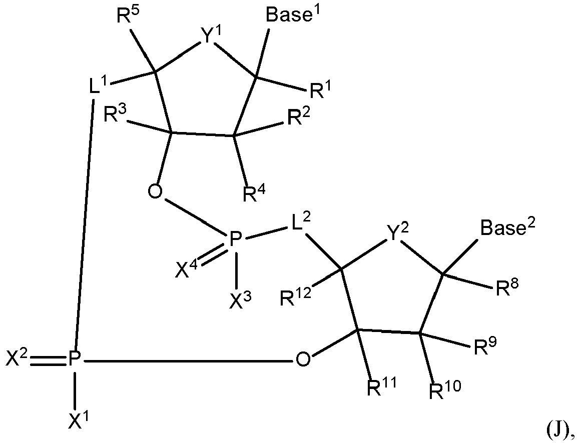 oder ein Enantiomer, Hydrat, Solvat oder pharmazeutisch akzeptables Salz davon,
wobei
L¹ ist -C(R⁶R⁷)-O- und L² ist -C(R¹³)=C(R¹⁴)-,
L¹ ist -O-C(R⁶R⁷)- und L² ist -C(R¹³)=C(R¹⁴)-,
L¹ ist -C(R¹³)=C(R¹⁴)- und L² ist -C(R¹³)=C(R¹⁴)-,
L¹ ist -C(R¹³)=C(R¹⁴)- und L² ist -C(R⁶R⁷)-O-, oder
L¹ ist -C(R¹³)=C(R¹⁴)- und L² ist -O-C(R⁶R⁷)-;
Y¹ und Y² sind jeweils unabhängig voneinander -O-, -S-, oder -CH₂-;
X¹ und X³ sind jeweils unabhängig voneinander OH, SH, OR¹⁵, SR¹⁵, oder N(R¹⁵)₂;
X² und X⁴ sind jeweils unabhängig voneinander O oder S;
R¹, R⁵, R⁸ und R¹² sind jeweils unabhängig voneinander H, CN, N₃, F, Cl, Br, I, COOR¹⁵, CON(R¹⁵)₂, C₁-C₆Alkyl, C₂-C₆ Alkenyl, C₂-C₆ Alkynyl, OR¹⁵, SR¹⁵, oder N(R¹⁵)₂,
R², R³, R4, R⁹, R¹⁰ und R¹¹ sind jeweils unabhängig voneinander H, F, Cl, Br, I, CN, N₃, C₁-C₆ alkyl, C₂-C₆ Alkenyl, C₂-C₆ Alkynyl, OR¹⁵, SR¹⁵, oder N(R¹⁵)₂,
R⁶, R⁷, R¹³ und R¹⁴ sind jeweils unabhängig voneinander H, CN, N₃, F, Cl, Br, I, COOR¹⁵, CON(R¹⁵)₂, OR¹⁵, SR¹⁵, N(R¹⁵)₂, C₁-C₆ Alkyl, C₂-C₆ Alkenyl, C₂-C₆ Alkynyl, C₃-C₇ Cycloalkyl, C₂-C₁₀ Heterocycloalkyl, C₆-C₁₀ Aryl, oder C₂-C₁₀ Heteroaryl;
jedes R¹⁵ ist unabhängig H, -C(=Z)R¹⁶, -C(=Z)OR¹⁶, -C(=Z)SR¹⁶, -C(=Z)N(R¹⁶)₂, -CH₂-Z-C(=Z)R¹⁶, C₁-C₆ Alkyl, C₂-C₆ Alkenyl, C₂-C₆ Alkynyl, C₃-C₇ Cycloalkyl, C₂-C₁₀ Heterocycloalkyl, C₆-C₁₀ Aryl, oder C₂-C₁₀ Heteroaryl;
jedes R¹⁶ ist unabhängig H, C₁-C₁₂ Alkyl, C₂-C₁₂ Alkenyl, C₂-C₁₂ Alkynyl, C₃-C₇ Cycloalkyl, C₂-C₁₀ Heterocycloalkyl, C₆-C₁₀ Aryl, oder C₂-C₁₀ Heteroaryl; vorzugsweise jedes R¹⁶ ist unabhängig H, C₁-C₆ Alkyl, C₂-C₆ Alkenyl, C₂-C₆ Alkynyl, C₃-C₇ Cycloalkyl, C₂-C₁₀ Heterocycloalkyl, C₆-C₁₀ Aryl, oder C₂-C₁₀ Heteroaryl;
jedes Z ist unabhängig O oder S;
Base¹ und Base² sind jeweils unabhängig voneinander: 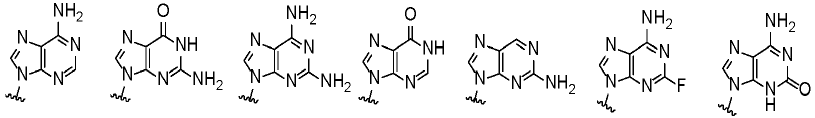 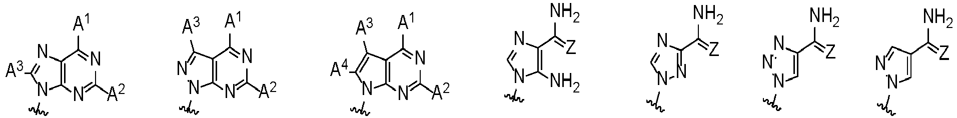 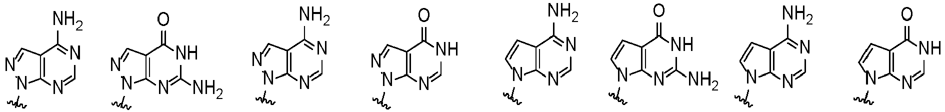 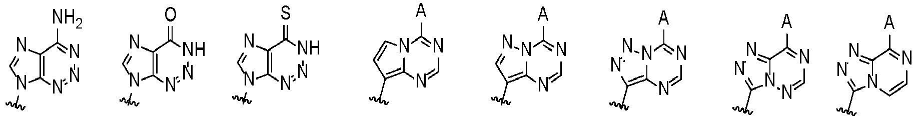 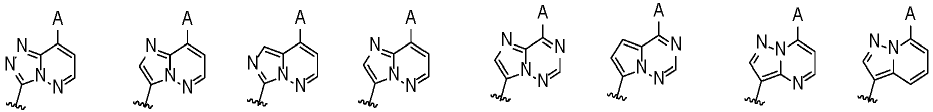 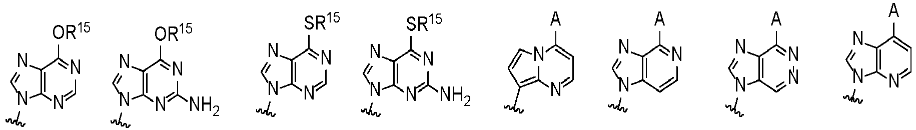 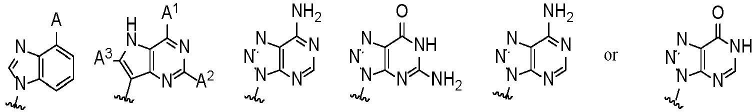
wobei
A, A¹, A², A³ und A⁴ sind jeweils unabhängig voneinander H, OH, SH, F, Cl, Br, I, NH₂, OR¹⁵, SR¹⁵, NHR¹⁵, N(R¹⁵)₂, oder R¹⁶; und
wobei die C₁-C₆ alkyl, C₂-C₆ Alkenyl, C₂-C₆ Alkynyl, C₂-C₁₀ Heterocycloalkyl, C₆-C₁₀ Aryl, oder C₂-C₁₀ Heteroaryl unabhängig voneinander jeweils gegebenenfalls substituiert sind mit 1, 2, oder 3 -OH; -SH; -NH₂; =O; =NH; =S; Halogen; -N₃; C₆-C₁₀ Aryl gegebenenfalls substituiert mit 1, 2, oder 3-OH, -CN, -O(C=O)OR^{B}, -O(C=O)R^{B}, oder -COOR^{B}; unsubstituierte C₁-C₆ Alkyl; unsubstituierte C₁-C₆ Alkoxy; unsubstituierte C₁-C₆ Alkylthio; unsubstituierte C₁-C₆ Alkylamino; unsubstituierte C₁-C₆ Dialkylamino; -CN; -O(C=O)OR^{B}; -O(C=O)R^{B}; oder -COOR^{B}; wobei R^{B} ist H oder unsubstituierte C₁-C₆ Alkyl.

2. Eine Verbindung nach Anspruch 1, wobei
Y¹ und Y² sind jeweils voneinander unabhängig -O- oder -S-;
X¹ und X³ sind jeweils voneinander unabhängig OH, SH, OR¹⁵ oder SR¹⁵;
X² und X⁴ sind jeweils voneinander unabhängig O oder S;
R¹, R⁵, R⁸ und R¹² sind H;
R², R³, R⁹, und R¹¹ sind H;
R⁴ und R¹⁰ sind jeweils unabhängig voneinander H, F, Cl, CN, N₃, OH, SH, NH₂, OR¹⁵, SR¹⁵, oder N(R¹⁵)₂; vorzugsweise sind diese Substituenten unabhängig voneinander ausgewählt aus H, F, Cl, OH, SH, NH₂, CN und N₃;
R⁶, R⁷, R¹³ und R¹⁴ sind jeweils unabhängig voneinander H, C₁-C₆ alkyl, C₂-C₆ Alkenyl, oder C₆-C₁₀ Aryl; vorzugsweise sind diese Substituenten H oder C₁-C₄ Alkyl;
R¹⁵ ist unabhängig H, -C(=Z)R¹⁶, -CH₂-Z-C(=Z)R¹⁶, C₁-C₆ Alkyl, C₂-C₆ Alkenyl, C₂-C₆ Alkynyl, C₃-C₇ Cycloalkyl, C₂-C₁₀ Heterocycloalkyl, C₆-C₁₀ Aryl, oder C₂-C₁₀ Heteroaryl;
jedes R¹⁶ ist unabhängig H, C₁-C₁₂ Alkyl, C₂-C₁₂ Alkenyl, C₂-C₁₂ Alkynyl, C₃-C₇ Cycloalkyl, C₂-C₁₀ Heterocycloalkyl, C₆-C₁₀ Aryl, oder C₂-C₁₀ Heteroaryl; vorzugsweise jedes R¹⁶ ist unabhängig H, C₁-C₆Alkyl, C₂-C₆ Alkenyl, C₃-C₇ Cycloalkyl, C₆-C₁₀ Aryl, oder C₂-C₁₀ Heteroaryl;
jedes Z ist unabhängig O oder S.

3. Eine Verbindung nach Anspruch 1 oder 2 mit der Struktur, ausgewählt aus Formel (I), Formel (IIa), Formel (Ia) und Formel (IIIb), oder ein Enantiomer, Hydrat, Solvat oder pharmazeutisch akzeptables Salz davon:

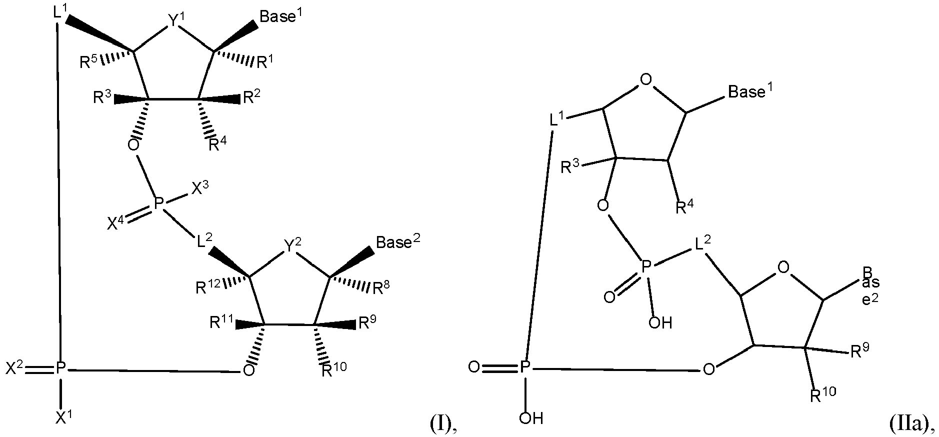

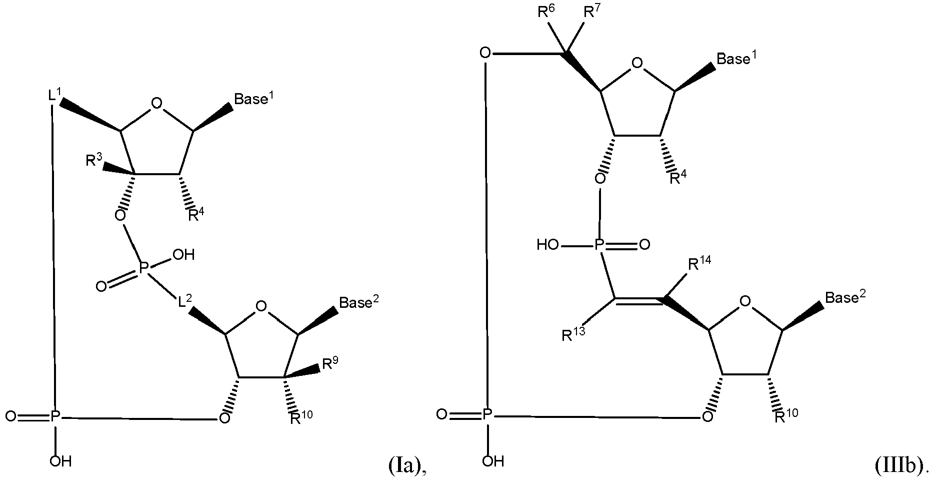

4. Eine Verbindung nach einem der Ansprüche 1 bis 3, wobei
- X² und X⁴ sind jeweils O, und/oder
- Y¹ und Y² sind jeweils unabhängig voneinander -O- oder -CH₂-, und/oder
R¹, R², R⁵, R⁸, R¹¹ und R¹² sind jeweils unabhängig voneinander H, OH, F, CN, oder C₁-C₆ Alkyl, und/oder
X¹ und X³ sind jeweils unabhängig voneinander OH und SH.

5. Eine Verbindung nach einem der Ansprüche 1 bis 4, wobei
L¹ ist -C(R¹³)=C(R¹⁴)- und L² ist -O-C(R⁶R⁷)-; oder
L¹ ist -O-C(R⁶R⁷)- und L² ist -C(R¹³)=C(R¹⁴)-; oder
L¹ ist -C(R¹³)=C(R¹⁴)- und L² ist -C(R¹³)=C(R¹⁴)-.

6. Eine Verbindung nach einem der Ansprüche 1 bis 5, wobei
R³ und R⁴ sind jeweils unabhängig voneinander H, OR¹⁵, F, Cl, CN, N₃, oder C₁-C₆Alkyl, wobei mindestens ein R³ und R⁴ H ist;
oder
R⁹ und R¹⁰ sind jeweils unabhängig voneinander H, OR¹⁵, F, Cl, CN, N₃, oder C₁-C₆ Alkyl, wobei mindestens eine R⁹ und R¹⁰ H ist.

7. Eine Verbindung nach einem der Ansprüche 1 bis 6, wobei R⁴ und R₁₀ unabhängig voneinander H, OH oder F sind.

8. Eine Verbindung nach einem der Ansprüche 1 bis 7, wobei R⁶, R⁷, R¹³ und R¹⁴ sind jeweils unabhängig voneinander H, CN, F, Cl, COOR¹⁵, CON(R¹⁵)₂, OR¹⁵, SR¹⁵, N(R¹⁵)₂, oder C₁-C₆ Alkyl, wobei jedes R¹⁵ ist unabhängig H oder C₁-C₆ Alkyl oder R⁶, R⁷, R¹³ und R¹⁴ sind jeweils H.

9. Eine Verbindung nach einem der Ansprüche 1 bis 8, wobei Base¹ und Base² jeweils unabhängig voneinander ausgewählt sind aus:

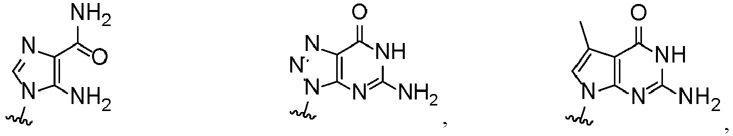

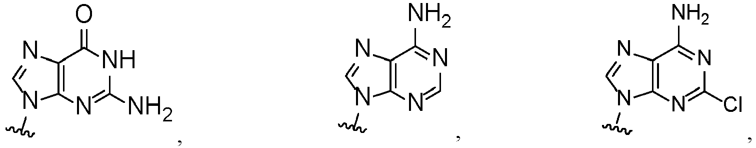

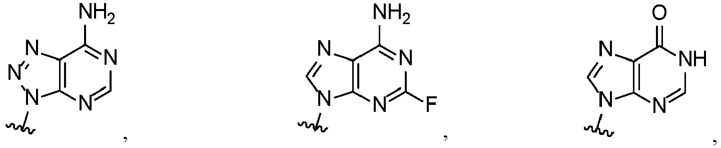

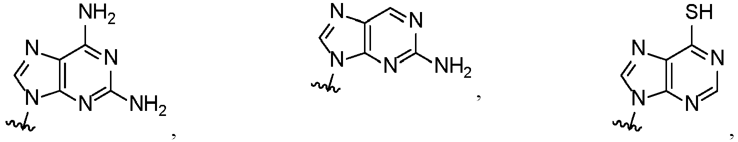

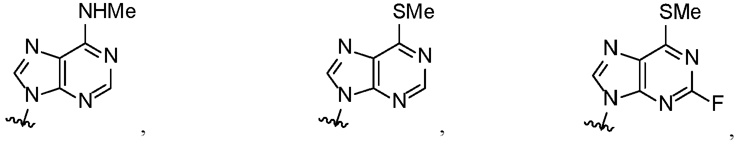

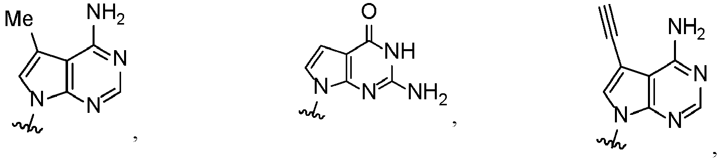

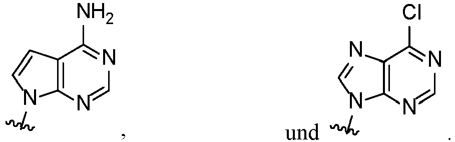

10. Eine Verbindung nach Anspruch 1, wobei die Verbindung ausgewählt ist aus: 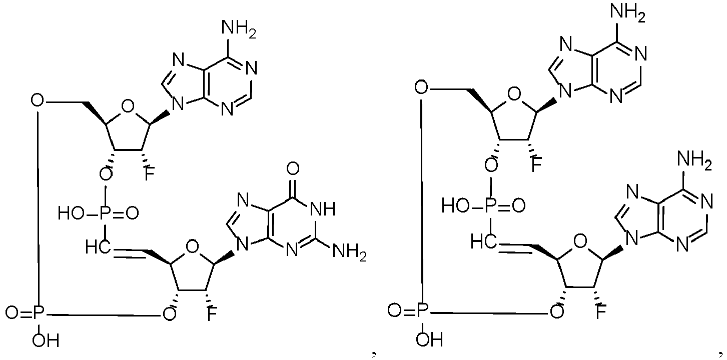 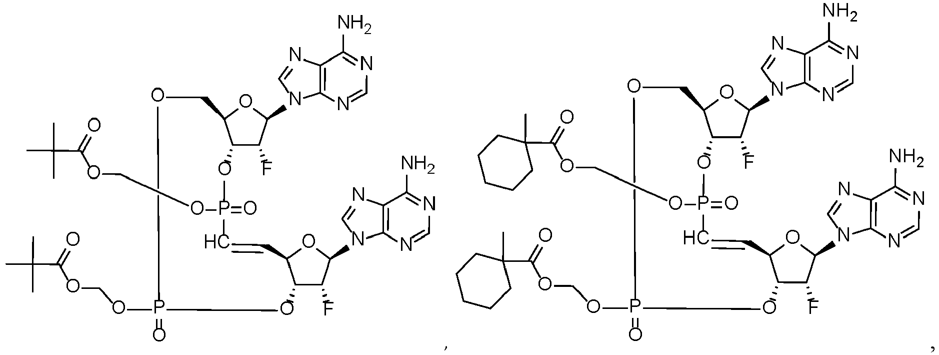 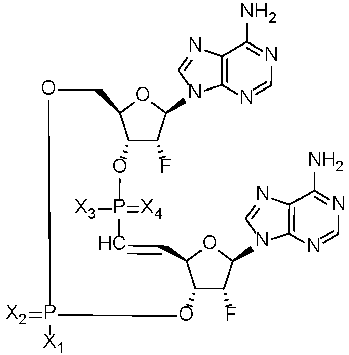 wobei X¹, X², X³, X⁴ sind wie in Anspruch 1 definiert.

11. Eine Pharmazeutische Zusammensetzung, umfassend die Verbindung nach einem der Ansprüche 1 bis 10 oder ein Enantiomer, Solvat, Hydrat oder ein pharmazeutisch annehmbares Salz davon und einen pharmazeutisch annehmbaren Träger, Exzipienten und/oder Verdünnungsmittel.

12. Eine Verbindung nach einem der Ansprüche 1 bis 10 zur Verwendung bei der Regulierung der Aktivität des STING-Adaptorproteins, um die Produktion eines Interferons vom Typ I, eines Zytokins und/oder Chemokins in Abhängigkeit von dem STING-Adaptorprotein zu induzieren, und/oder zur Verwendung bei der Behandlung oder Vorbeugung einer Virusinfektion, Hepatitis-B-Virusinfektion, HIV-Infektion, allergischen Erkrankungen, entzündlichen Erkrankungen, hyperproliferativen Erkrankung oder Krebs bei einem Menschen oder einem Tier.

13. Eine Verbindung nach einem der Ansprüche 1 bis 10 zur Verwendung als Bestandteil einer immunmodulierenden Zusammensetzung und/oder zur Verwendung als Impfstoff-Adjuvans.

## Revendications

1. Un composé de formule (J): 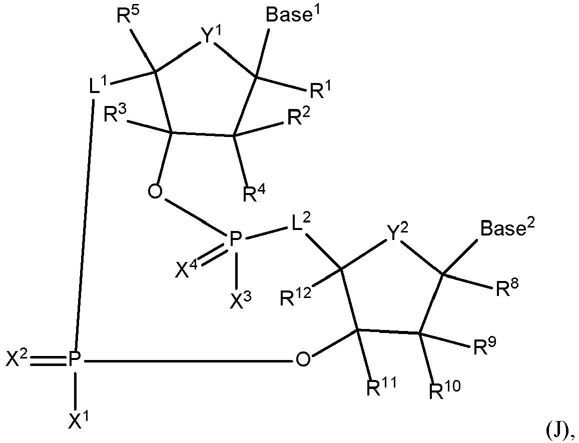 ou un énantiomère, un hydrate, un solvate ou un sel pharmaceutiquement acceptable de celui-ci,
dans lequel
L¹ est -C(R⁶R⁷)-O- et L² est -C(R¹³)=C(R¹⁴)-,
L¹ est -O-C(R⁶R⁷)- et L² est -C(R¹³)=C(R¹⁴)-,
L¹ est -C(R¹³)=C(R¹⁴)- et L² est -C(R¹³)=C(R¹⁴)-,
L¹ est -C(R¹³)=C(R¹⁴)- et L² est -C(R⁶R⁷)-O-, or
L¹ est -C(R¹³)=C(R¹⁴)- et L² est -O-C(R⁶R⁷)-;
Y¹ et Y² sont chacun indépendamment -O-, -S-, ou -CH₂-;
X¹ et X³ sont chacun indépendamment OH, SH, OR¹⁵, SR¹⁵, ou N(R¹⁵)₂;
X² et X⁴ sont chacun indépendamment O ou S;
R¹, R⁵, R⁸ et R¹² sont chacun indépendamment H, CN, N₃, F, Cl, Br, I, COOR¹⁵, CON(R¹⁵)₂, alkyle en C₁-C₆, alcényle en C₂-C₆, alcynyle en C₂-C₆, OR¹⁵, SR¹⁵, ou N(R¹⁵)₂;
R², R³, R⁴, R⁹, R¹⁰ et R¹¹ sont chacun indépendamment H, F, Cl, Br, I, CN, N₃, alkyle en C₁-C₆, alcényle en C₂-C₆, alcynyle en C₂-C₆, OR¹⁵, SR¹⁵, ou N(R¹⁵)₂;
R⁶, R⁷, R¹³ et R¹⁴ sont chacun indépendamment H, CN, N₃, F, Cl, Br, I, COOR¹⁵, CON(R¹⁵)₂, OR¹⁵, SR¹⁵, N(R¹⁵)₂, alkyle en Ci-Ce, alcényle en C₂-C₆, alcynyle en C₂-C₆, cycloalkyle en C₃-C₇, hétérocycloalkyle en C₂-C₁₀, aryle en C₆-C₁₀ ou hétéroaryle en C₂-C₁₀;
chacun R¹⁵ est indépendamment H, -C(=Z)R¹⁶, -C(=Z)OR¹⁶, -C(=Z)SR¹⁶, -C(=Z)N(R¹⁶)₂, -CH₂-Z-C(=Z)R¹⁶, alkyle en C₁-C₆, alcényle en C₂-C₆, alcynyle en C₂-C₆, cycloalkyle en C₃-C₇, hétérocycloalkyle en C₂-C₁₀, aryle en C₆-C₁₀ ou hétéroaryle en C₂-C₁₀;
chacun R¹⁶ est indépendamment H, alkyle en C₁-C₁₂, alcényle en C₂-C₁₂, alcynyle en C₂-C₁₂, cycloalkyle en C₃-C₇, hétérocycloalkyle en C₂-C₁₀, aryle en C₆-C₁₀ ou hétéroaryle en C₂-C₁₀;
de préférence, chaque R¹⁶ est indépendamment H, alkyle en C₁-C₆, alcényle en C₂-C₆, alcynyle en C₂-C₆, cycloalkyle en C₃-C₇, hétérocycloalkyle en C₂-C₁₀, aryle en C₆-C₁₀ ou hétéroaryle en C₂-C₁₀; chacun Z est indépendamment O ou S;
Base¹ et Base² sont chacun indépendamment: 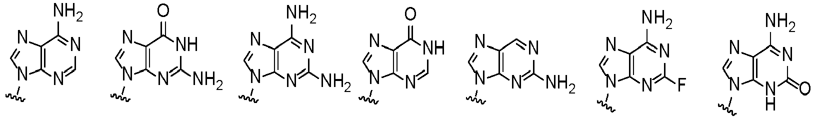 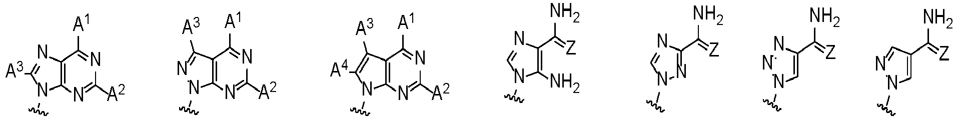 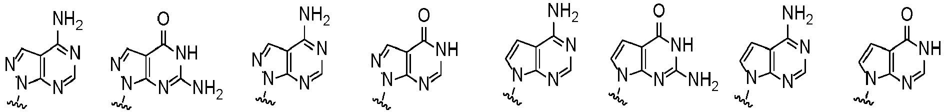 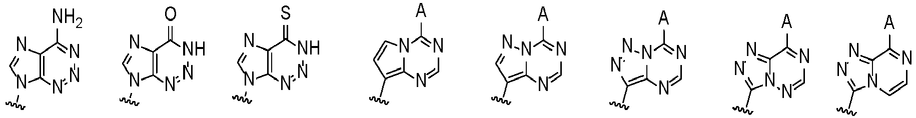 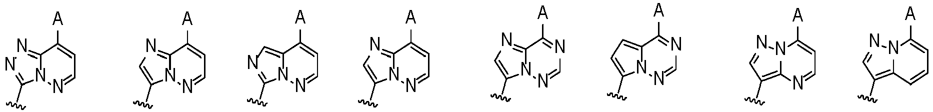 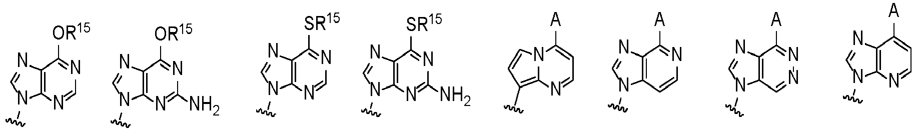 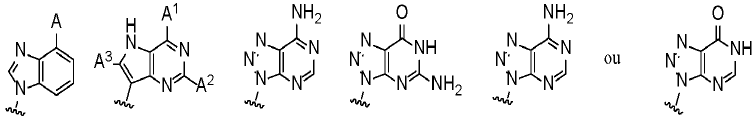
où
A, A¹, A², A³ et A⁴ sont chacun indépendamment H, OH, SH, F, Cl, Br, I, NH₂, OR¹⁵, SR¹⁵, NHR¹⁵, N(R¹⁵)₂, ou R¹⁶; et
où alkyle en Ci-Ce, alcényle en C₂-C₆, alcynyle en C₂-C₆, cycloalkyle en C₃-C₇, hétérocycloalkyle en C₂-C₁₀, aryle en C₆-C₁₀ ou hétéroaryle en C₂-C₁₀ est, indépendamment dans chaque cas, éventuellement substitué par 1, 2, ou 3 -OH; -SH; -NH₂; =O; =NH; =S; halogène; -N₃; aryle en C₆-C₁₀ éventuellement substitué par 1, 2, ou 3-OH, -CN, -O(C=O)OR^{B}, -O(C=O)R^{B}, ou -COOR^{B}; non substitué alkyle en Ci-C₆; non substitué alcoxy en C₁-C₆; non substitué alkylethio en C₁-C₆; non substitué alkylamino en Ci-Ce; non substitué dialkylamino en C₁-C₆; -CN; -O(C=O)OR^{B}; -O(C=O)R^{B}; ou -COOR^{B}; dans lequel R^{B} est H ou non substitué alkyle en C₁-C₆.

2. Composé selon la revendication 1, où
Y¹ et Y² sont chacun indépendamment -O- ou -S- ;
X¹ et X³ sont chacun indépendamment OH, SH, OR¹⁵ ou SR¹⁵;
X² et X⁴ sont chacun indépendamment O ou S;
R¹, R⁵, R⁸ et R¹² sont H;
R², R³, R⁹, et R¹¹ sont H;
R⁴ et R¹⁰ sont chacun indépendamment H, F, Cl, CN, N₃, OH, SH, NH₂, OR¹⁵, SR¹⁵, ou N(R¹⁵)₂; de préférence, ces substituants sont choisis indépendamment parmi H, F, Cl, OH, SH, NH₂, CN et N₃;
R⁶, R⁷, R¹³ et R¹⁴ sont chacun indépendamment H, alkyle en C₁-C₆, alcényle en C₂-C₆, ou aryle en C₆-C₁₀; de préférence, ces substituants sont H ou alkyle en C₁-C₆;
R¹⁵ est indépendamment H, -C(=Z)R¹⁶, -CH₂-Z-C(=Z)R¹⁶, alkyle en Ci-Ce, alcényle en C₂-C₆, alcynyle en C₂-C₆, cycloalkyle en C₃-C₇, hétérocycloalkyle en C₂-C₁₀, aryle en C₆-C₁₀ ou hétéroaryle en C₂-C₁₀;
chaque R¹⁶ est indépendamment H, alkyle en C₁-C₆, alcényle en C₂-C₆, alcynyle en C₂-C₆, cycloalkyle en C₃-C₇, hétérocycloalkyle en C₂-C₁₀, aryle en C₆-C₁₀ ou hétéroaryle en C₂-C₁₀; de préférence, chaque R¹⁶ est indépendamment H, alkyle en C₁-C₆, alcényle en C₂-C₆, cycloalkyle en C₃-C₇, aryle en C₆-C₁₀ ou hétéroaryle en C₂-C₁₀;
chaque Z est indépendamment O ou S.

3. Composé de la revendication 1 ou 2 ayant la structure choisie parmi la formule (I), la formule (IIa), la formule (Ia) et la formule (IIIb), ou un énantiomère, un hydrate, un solvate ou un sel pharmaceutiquement acceptable de celui-ci:

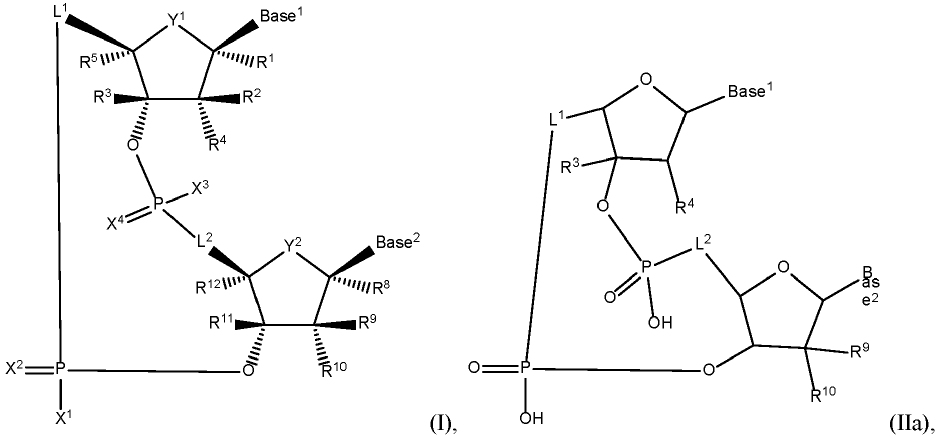

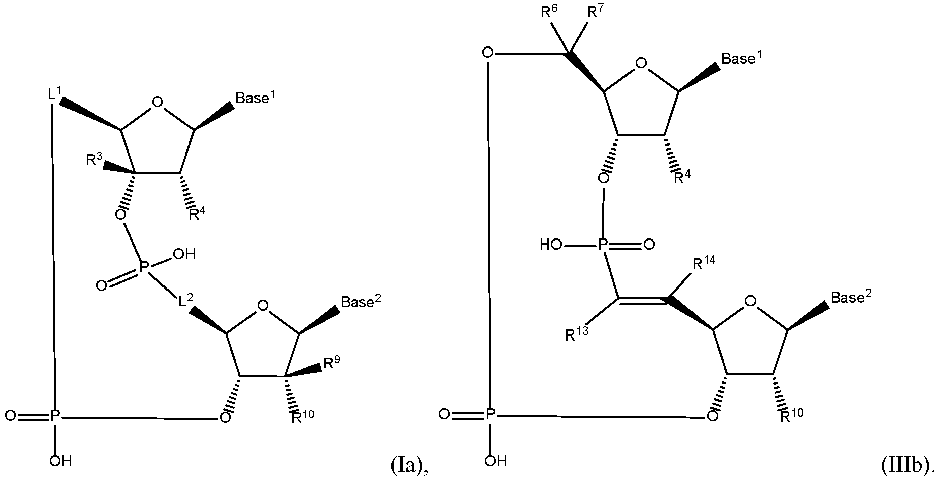

4. Composé de l'une quelconque des revendications 1 à 3, où
- X² et X⁴ sont chacun O, et/ou
- Y¹ et Y² sont chacun indépendamment -O- ou -CH₂-, et/ou
R¹, R², R⁵, R⁸, R¹¹ et R¹² sont chacun indépendamment H, OH, F, CN, ou alkyle en C₁-C₆, et/ou
X¹ et X³ sont chacun indépendamment OH ou SH.

5. Composé de l'une quelconque des revendications 1 à 4, où
L¹ est -C(R¹³)=C(R¹⁴)- et L² est -O-C(R⁶R⁷)-; ou
L¹ est -O-C(R⁶R⁷)- et L² est -C(R¹³)=C(R¹⁴)-; ou
L¹ est -C(R¹³)=C(R¹⁴)- et L² est -C(R¹³)=C(R¹⁴)-.

6. Composé de l'une quelconque des revendications 1 à 5, où
R³ et R⁴ sont chacun indépendamment H, OR¹⁵, F, Cl, CN, N₃, ou alkyle en C₁-C₆, dans lequel au moins un des R³ et R⁴ est H;
ou
R⁹ et R¹⁰ sont chacun indépendamment H, OR¹⁵, F, Cl, CN, N₃, ou alkyle en C₁-C₆, dans lequel au moins un des R⁹ et R¹⁰ est H.

7. Composé de l'une quelconque des revendications 1 à 6, où R₄ et R₁₀ sont indépendamment H, OH ou F.

8. Composé de l'une quelconque des revendications 1 à 7, où R⁶, R⁷, R¹³ et R¹⁴ sont chacun indépendamment H, CN, F, Cl, COOR¹⁵, CON(R¹⁵)₂, OR¹⁵, SR¹⁵, N(R¹⁵)₂, ou alkyle en C₁-C₆, où chacun R¹⁵ est i indépendamment H ou alkyle en C₁-C₆ ou R⁶, R⁷, R¹³ et R¹⁴ sont chacun H.

9. Composé de l'une quelconque des revendications 1 à 8, où Base¹ et Base² sont chacun indépendamment choisis parmi :

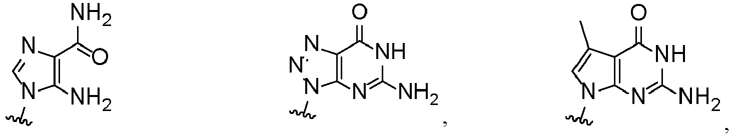

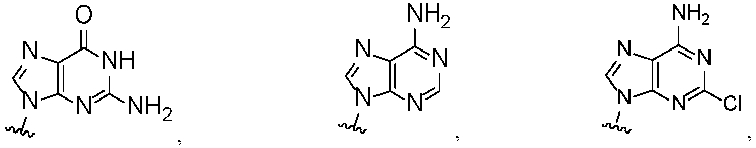

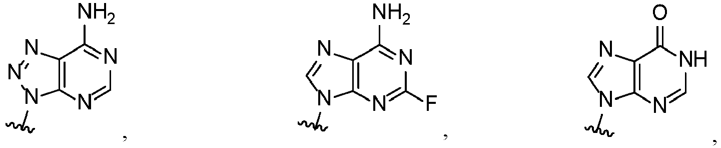

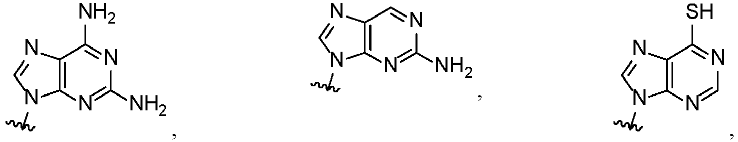

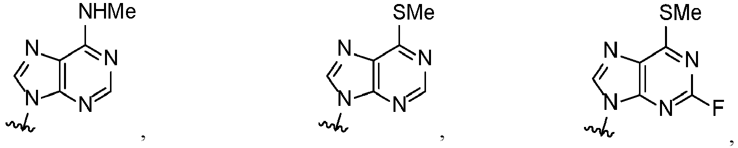

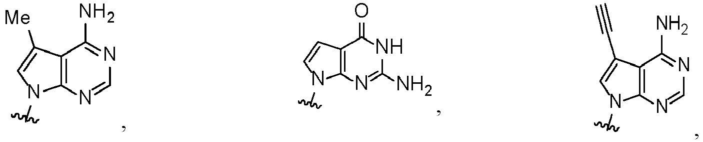

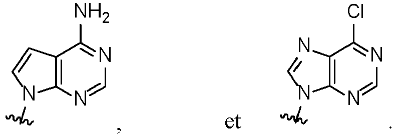

10. Composé de la revendication 1, où le composé est choisi parmi: 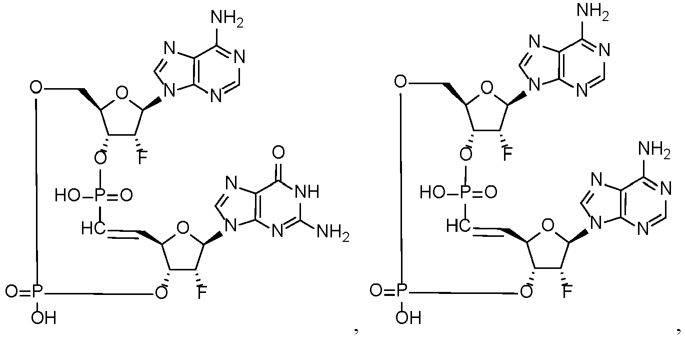 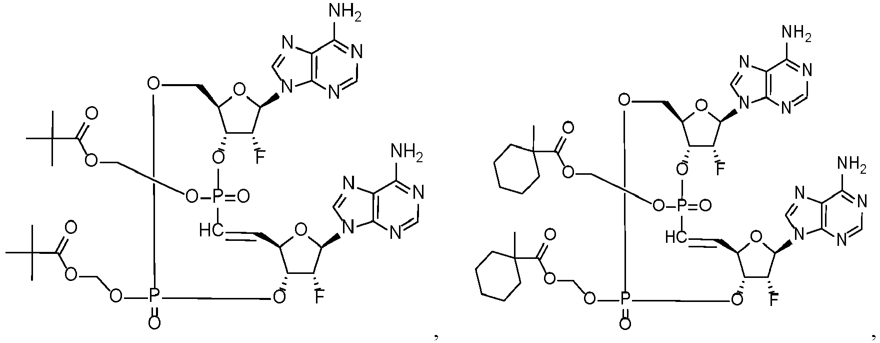 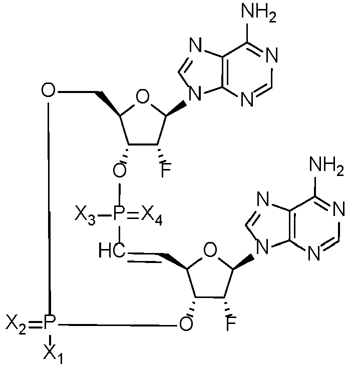 où
X¹, X², X³, X⁴ sont tels que définis dans la revendication 1.

11. Composition pharmaceutique comprenant le composé de l'une quelconque des revendications 1 à 10, ou un énantiomère, un solvate, un hydrate ou un sel pharmaceutiquement acceptable de celui-ci, et un support, excipient et/ou diluant pharmaceutiquement acceptable.

12. Composé de l'une quelconque des revendications 1 à 10, pour une utilisation dans la modulation de l'activité de la protéine adaptatrice STING afin d'induire la production d'un interféron de type I, d'une cytokine et/ou d'une chimiokine dépendant de la protéine adaptatrice STING, et/ou pour une utilisation dans le traitement ou la prévention de la maladie une infection virale, une infection par le virus de l'hépatite B, une infection par le VIH, des maladies allergiques, des maladies inflammatoires, une maladie hyperproliférative ou un cancer chez un humain ou un animal.

13. Composé selon l'une quelconque des revendications 1 à 10 pour une utilisation comme composant d'une composition immunomodulatrice, et/ou pour une utilisation comme adjuvant de vaccin.
